(19) Europäisches Patentamt

European Patent Office

Office européen des brevets

(11) **EP 2 734 633 B1**

(12) # EUROPEAN PATENT SPECIFICATION

(45) Date of publication and mention of the grant of the patent:
**01.05.2019 Bulletin 2019/18**

(51) Int Cl.:
***C12P 19/14*** *(2006.01)*

(21) Application number: **12746168.9**

(22) Date of filing: **19.07.2012**

(86) International application number:
**PCT/US2012/047326**

(87) International publication number:
**WO 2013/016115 (31.01.2013 Gazette 2013/05)**

(54) **PROCESSES FOR PRETREATING CELLULOSIC MATERIAL AND IMPROVING HYDROLYSIS THEREOF**

VERFAHREN ZUR VORBEHANDLUNG VON CELLULOSEMATERIAL UND VERBESSERUNG DEREN HYDROLYSE

PROCÉDÉS DE PRETRAITEMENT MATÉRIEL CELLULOSIQUE ET AMÉLIORATION DE SON HYDROLYSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.07.2011 US 201161510637 P**

(43) Date of publication of application:
**28.05.2014 Bulletin 2014/22**

(73) Proprietor: **Novozymes North America, Inc. Franklinton, NC 27525 (US)**

(72) Inventors:
• GASPAR, Armindo, Ribeiro
  Rolesville, NC 27571 (US)
• XU, Hui
  Wake Forest, NC 27587 (US)
• CROONENBERGHS, James
  Durham, NC 27707 (US)
• LUO, James
  Raleigh, NC 27616 (US)
• RANE, Kishore
  Raleigh, NC 27614 (US)

(74) Representative: **NZ EPO Representatives Krogshoejvej 36 2880 Bagsvaerd (DK)**

(56) References cited:
**EP-A1- 0 546 721          WO-A2-2009/042622 JP-A- 2009 240 167**

• **MONIRUZZAMAN M ET AL: "Enzymatic hydrolysis of high-moisture corn fiber pretreated by AFEX and recovery and recycling of the enzyme complex", APPLIED BIOCHEMISTRY AND BIOTECHNOLOGY, vol. 67, no. 1-2, 1997, pages 113-126, XP002686351, ISSN: 0273-2289**
• **COLLARES R M ET AL: "Optimization of enzymatic hydrolysis of cassava to obtain fermentable sugars", JOURNAL OF ZHEJIANG UNIVERSITY: SCIENCE B 2012 ZHEJIANG UNIVERSITY PRESS CHN LNKD-DOI:10.1631/JZUS.B1100297, vol. 13, no. 7, July 2012 (2012-07), pages 579-586, XP002686352, ISSN: 1673-1581**

EP 2 734 633 B1

## Description

### Reference to a Sequence Listing

This application contains a Sequence Listing in computer readable form. The computer readable form is incorporated herein by reference.

### Background of the Invention

### Field of the Invention

The present invention relates to processes for enzymatic pretreatment of a cellulosic material using one or more cutinase, peptidase and pectate lyase enzymes. The pretreated cellulosic material is suitable for saccharification. During hydrolysis, the addition of one or more amylase and glucoamylase and/or mannanase enzymes improves hydrolysis performance.

### Description of the Related Art

Cellulose is a polymer of the simple sugar glucose covalently linked by beta-1,4-bonds. Many microorganisms produce enzymes that hydrolyze beta-linked glucans. These enzymes include endoglucanases, cellobiohydrolases, and beta-glucosidases. Endoglucanases digest the cellulose polymer at random locations, opening it to attack by cellobiohydrolases. Cellobiohydrolases sequentially release molecules of cellobiose from the ends of the cellulose polymer. Cellobiose is a water-soluble beta-1,4-linked dimer of glucose. Beta-glucosidases hydrolyze cellobiose to glucose.

The conversion of lignocellulosic feedstocks into ethanol has the advantages of the ready availability of large amounts of feedstock, the desirability of avoiding burning or land filling the materials, and the cleanliness of the ethanol fuel. Wood, agricultural residues, herbaceous crops, and municipal solid wastes have been considered as feedstocks for ethanol production. These materials primarily consist of cellulose, hemicellulose, and lignin. Once the lignocellulose is converted to fermentable sugars, e.g., glucose, the fermentable sugars are easily fermented by yeast into ethanol. The sugars can also be catalytically converted or fermented to other chemicals besides ethanol.

The conversion of lignocellulosic feedstocks into sugars, typically, involves pretreatment of the cellulosic materials, followed by their enzymatic hydrolysis, prior to the conversion of the sugars into fermentation products or catalytically converted products. The pretreatments disrupt the lignocellulosic material, so enzymatic hydrolysis can take place efficiently.

However, pretreatment of cellulosic materials can produce impurities in the pretreated cellulosic materials having a deleterious effect on cellulase enzymes and/or decreases or inhibits enzymatic hydrolysis and/or saccharification.

It would be advantageous to the art to be able to improve the pretreated cellulosic material for saccharification. For example, it would be advantageous in the art to improve the enzymatic hydrolysis performance of pretreated cellulosic material by reducing, eliminating or removing impurities that have a deleterious effect on the cellulase enzymes.

WO 2009/042622 discloses a process for producing fermentation product from wood-containing material, wherein the process includes the steps of i) pre-treating wood-containing material; ii) hydrolyzing by subjecting the pre-treated wood-containing material to one or more cellulolytic enzymes; iii) fermenting using a fermenting organism, wherein the wood-containing material is subjected to one or more esterases before and/or during pre-treatment in step i) and/or hydrolysis in step ii) and/or fermentation in step iii).

There is a continuous need for processes for enzymatic pretreatment of a cellulosic material, and processes for the improvement of hydrolysis performance.

### Summary of the Invention

The present disclosure relates to a method for increasing cellulolytic enzyme activity during the hydrolysis of cellulosic material comprising or consisting of:

(a) contacting the cellulosic material with cutinase, peptidase and pectate lyase enzymes to form pretreated cellulosic material; and
(b) hydrolyzing the pretreated cellulosic material with one or more amylase and glucoamylase and/or mannanase enzymes.

The present disclosure further relates to a method for hydrolyzing a pretreated cellulosic material comprising or consisting of saccharifying a cellulosic material with an enzyme composition, comprising one or more amylases and glucoamylases and/or mannanase enzymes, wherein the cellulosic material is pretreated by contacting the cellulosic material with cutinase, peptidase and pectate lyase enzymes to form pretreated cellulosic material.

The present disclosure also relates to a method for producing a fermentation product, comprising or consisting of: (a) saccharifying a pretreated cellulosic material with an enzyme composition, comprising one or more cellulolytic enzymes, and at least one glucoamylase and/or mannanase enzymes mannanase, and mixtures thereof; (b) fermenting the saccharified pretreated cellulosic material with one or more (several) fermenting microorganisms to produce the fermentation product; and (c) recovering the fermentation product from the fermentation, wherein the pretreated cellulosic material was pretreated by contacting cellulosic material with one or more cutinase, peptidase and pectate lyase enzymes in accordance with the present disclosure.

In an embodiment, the pretreated cellulosic material is a woody biomass substrate.

## Definitions

**Cellulolytic enzyme or cellulase:** The term "cellulolytic enzyme" or "cellulase" means one or more (several) enzymes that hydrolyze a cellulosic material. Such enzymes include endoglucanase(s), cellobiohydrolase(s), beta-glucosidase(s), or combinations thereof. The two basic approaches for measuring cellulolytic activity include: (1) measuring the total cellulolytic activity, and (2) measuring the individual cellulolytic activities (endoglucanases, cellobiohydrolases, and beta-glucosidases) as reviewed in Zhang et al., Outlook for cellulase improvement: Screening and selection strategies, 2006, Biotechnology Advances 24: 452-481. Total cellulolytic activity is usually measured using insoluble substrates, including Whatman №1 filter paper, microcrystalline cellulose, bacterial cellulose, algal cellulose, cotton, pretreated lignocellulose, *etc.* The most common total cellulolytic activity assay is the filter paper assay using Whatman №1 filter paper as the substrate. The assay was established by the International Union of Pure and Applied Chemistry (IUPAC) (Ghose, 1987, Measurement of cellulase activities, Pure Appl. Chem. 59: 257-68).

For purposes of the present disclosure, cellulolytic enzyme activity is determined by measuring the increase in hydrolysis of a cellulosic material by cellulolytic enzyme(s) under the following conditions: 1-20 mg of cellulolytic enzyme protein/g of cellulose in PCS for 3-7 days at 50°C compared to a control hydrolysis without addition of cellulolytic enzyme protein. Typical conditions are 1 ml reactions, washed or unwashed PCS, 5% insoluble solids, 50 mM sodium acetate pH 5, 1 mM $MnSO_4$, 50°C, 72 hours, sugar analysis by AMINEX® HPX-87H column (Bio-Rad Laboratories, Inc., Hercules, CA, USA).

**Endoglucanase:** The term "endoglucanase" means an endo-1,4-(1,3;1,4)-beta-D-glucan 4-glucanohydrolase (E.C. 3.2.1.4), which catalyses endohydrolysis of 1,4-beta-D-glycosidic linkages in cellulose, cellulose derivatives (such as carboxymethyl cellulose and hydroxyethyl cellulose), lichenin, beta-1,4 bonds in mixed beta-1,3 glucans such as cereal beta-D-glucans or xyloglucans, and other plant material containing cellulosic components. Endoglucanase activity can be determined by measuring reduction in substrate viscosity or increase in reducing ends determined by a reducing sugar assay (Zhang et al., 2006, Biotechnology Advances 24: 452-481). For purposes of the present disclosure endoglucanase activity is determined using carboxymethyl cellulose (CMC) as substrate according to the procedure of Ghose, 1987, Pure and Appl. Chem. 59: 257-268, at pH 5, 40°C.

**Cellobiohydrolase:** The term "cellobiohydrolase" means a 1,4-beta-D-glucan cellobiohydrolase (E.C. 3.2.1.91), which catalyzes the hydrolysis of 1,4-beta-D-glucosidic linkages in cellulose, cellooligosaccharides, or any beta-1,4-linked glucose containing polymer, releasing cellobiose from the reducing or non-reducing ends of the chain (Teeri, 1997, Crystalline cellulose degradation: New insight into the function of cellobiohydrolases, Trends in Biotechnology 15: 160-167; Teeri et al., 1998, Trichoderma reesei cellobiohydrolases: why so efficient on crystalline cellulose?, Biochem. Soc. Trans. 26: 173-178). For purposes of the present disclosure cellobiohydrolase activity is determined according to the procedures described by Lever et al., 1972, Anal. Biochem. 47: 273-279; van Tilbeurgh et al., 1982, FEBS Letters, 149: 152-156; van Tilbeurgh and Claeyssens, 1985, FEBS Letters, 187: 283-288; and Tomme et al., 1988, Eur. J. Biochem. 170: 575-581. In the present invention, the Lever *et al.* method can be employed to assess hydrolysis of cellulose in corn stover, while the methods of van Tilbeurgh *et al.* and Tomme *et al.* can be used to determine the cellobiohydrolase activity on a fluorescent disaccharide derivative, 4-methylumbelliferyl-3-D-lactoside.

**Beta-glucosidase:** The term "beta-glucosidase" means a beta-D-glucoside glucohydrolase (E.C. 3.2.1.21), which catalyzes the hydrolysis of terminal non-reducing beta-D-glucose residues with the release of beta-D-glucose. For purposes of the present disclosure beta-glucosidase activity is determined according to the basic procedure described by Venturi et al., 2002, Extracellular beta-D-glucosidase from Chaetomium thermophilum var. coprophilum: production, purification and some biochemical properties, J. Basic Microbiol. 42: 55-66. One unit of beta-glucosidase is defined as 1.0 μmole of *p*-nitrophenolate anion produced per minute at 25°C, pH 4.8 from 1 mM p-nitrophenyl-beta-D-glucopyranoside as substrate in 50 mM sodium citrate containing 0.01% TWEEN® 20.

**Polypeptide having cellulolytic enhancing activity:** The term "polypeptide having cellulolytic enhancing activity" means a GH61 polypeptide that catalyzes the enhancement of the hydrolysis of a cellulosic material by enzyme having cellulolytic activity. For purposes of the present disclosure cellulolytic enhancing activity is determined by measuring the increase in reducing sugars or the increase of the total of cellobiose and glucose from the hydrolysis of a cellulosic material by cellulolytic enzyme under the following conditions: 1-50 mg of total protein/g of cellulose in PCS, wherein total protein is comprised of 50-99.5% w/w cellulolytic enzyme protein and 0.5-50% w/w protein of a GH61 polypeptide having cellulolytic enhancing activity for 1-7 days at 50°C compared to a control hydrolysis with equal total protein loading without cellulolytic enhancing activity (1-50 mg of cellulolytic protein/g of cellulose in PCS). In a preferred aspect, a mixture of CELLUCLAST® 1.5L (Novozymes A/S, Bagsværd, Denmark) in the presence of 2-3% of total protein weight *Aspergillus oryzae* beta-glucosidase (recombinantly produced in *Aspergillus oryzae* according to WO 02/095014) or 2-3% of total protein weight *Aspergillus fumigatus* beta-glucosidase (recombinantly produced in *Aspergillus oryzae* as described in WO 02/095014) of cellulase protein loading is used as the source of the cellulolytic activity.

The GH61 polypeptides having cellulolytic enhancing activity enhance the hydrolysis of a cellulosic material catalyzed by enzyme having cellulolytic activity by reducing the amount of cellulolytic enzyme required to reach the same degree of hydrolysis preferably at least 1.01-fold, more preferably at least 1.05-fold, more preferably at least 1.10-fold, more preferably at least 1.25-fold, more preferably at least 1.5-fold, more preferably at least 2-fold, more preferably at least 3-fold, more preferably at least 4-fold, more preferably at least 5-fold, even more preferably at least 10-fold, and most preferably at least 20-fold.

**Family 61 glycoside hydrolase:** The term "Family 61 glycoside hydrolase" or "Family GH61" or "GH61" means a polypeptide falling into the glycoside hydrolase Family 61 according to Henrissat, 1991, A classification of glycosyl hydrolases based on amino-acid sequence similarities, Biochem. J. 280: 309-316, and Henrissat B., and Bairoch A., 1996, Updating the sequence-based classification of glycosyl hydrolases, Biochem. J. 316: 695-696. The enzymes in this family were originally classified as a glycoside hydrolase family based on measurement of very weak endo-1,4-beta-D-glucanase activity in one family member. The structure and mode of action of these enzymes are non-canonical and they cannot be considered as bona fide glycosidases. However, they are kept in the CAZy classification on the basis of their capacity to enhance the breakdown of lignocellulose when used in conjunction with a cellulase or a mixture of cellulases.

**Hemicellulolytic enzyme or hemicellulase:** The term "hemicellulolytic enzyme" or "hemicellulase" means one or more (several) enzymes that hydrolyze a hemicellulosic material. See, for example, Shallom and Shoham, 2003, Microbial hemicellulases. Current Opinion In Microbiology 6(3): 219-228). Hemicellulases are key components in the degradation of plant biomass. Examples of hemicellulases include, but are not limited to, an acetylmannan esterase, an acetyxylan esterase, an arabinanase, an arabinofuranosidase, a coumaric acid esterase, a feruloyl esterase, a galactosidase, a glucuronidase, a glucuronoyl esterase, a mannanase, a mannosidase, a xylanase, and a xylosidase. The substrates of these enzymes, the hemicelluloses, are a heterogeneous group of branched and linear polysaccharides that are bound via hydrogen bonds to the cellulose microfibrils in the plant cell wall, crosslinking them into a robust network. Hemicelluloses are also covalently attached to lignin, forming together with cellulose a highly complex structure. The variable structure and organization of hemicelluloses require the concerted action of many enzymes for its complete degradation. The catalytic modules of hemicellulases are either glycoside hydrolases (GHs) that hydrolyze glycosidic bonds, or carbohydrate esterases (CEs), which hydrolyze ester linkages of acetate or ferulic acid side groups. These catalytic modules, based on homology of their primary sequence, can be assigned into GH and CE families marked by numbers. Some families, with overall similar fold, can be further grouped into clans, marked alphabetically (*e.g.*, GH-A). A most informative and updated classification of these and other carbohydrate active enzymes is available on the Carbohydrate-Active Enzymes (CAZy) database. Hemicellulolytic enzyme activities can be measured according to Ghose and Bisaria, 1987, Pure & Appl. Chem. 59: 1739-1752.

**Xylan degrading activity or xylanolytic activity:** The term "xylan degrading activity" or "xylanolytic activity" means a biological activity that hydrolyzes xylan-containing material. The two basic approaches for measuring xylanolytic activity include: (1) measuring the total xylanolytic activity, and (2) measuring the individual xylanolytic activities (*e.g.*, endoxylanases, beta-xylosidases, arabinofuranosidases, alpha-glucuronidases, acetylxylan esterases, feruloyl esterases, and alpha-glucuronyl esterases). Recent progress in assays of xylanolytic enzymes was summarized in several publications including Biely and Puchard, Recent progress in the assays of xylanolytic enzymes, 2006, Journal of the Science of Food and Agriculture 86(11): 1636-1647; Spanikova and Biely, 2006, Glucuronoyl esterase - Novel carbohydrate esterase produced by Schizophyllum commune, FEBS Letters 580(19): 4597-4601; Herrmann et al., 1997, The beta-D-xylosidase of Trichoderma reesei is a multifunctional beta-D-xylan xylohydrolase, Biochemical Journal 321: 375-381.

Total xylan degrading activity can be measured by determining the reducing sugars formed from various types of xylan, including, for example, oat spelt, beechwood, and larchwood xylans, or by photometric determination of dyed xylan fragments released from various covalently dyed xylans. The most common total xylanolytic activity assay is based on production of reducing sugars from polymeric 4-O-methyl glucuronoxylan as described in Bailey, Biely, Poutanen, 1992, Interlaboratory testing of methods for assay of xylanase activity, Journal of Biotechnology 23(3): 257-270. Xylanase activity can also be determined with 0.2% AZCL-arabinoxylan as substrate in 0.01% Triton X-100 and 200 mM sodium phosphate buffer pH 6 at 37°C. One unit of xylanase activity is defined as 1.0 μmole of azurine produced per minute at 37°C, pH 6 from 0.2% AZCL-arabinoxylan as substrate in 200 mM sodium phosphate pH 6 buffer.

For purposes of the present disclosure xylan degrading activity is determined by measuring the increase in hydrolysis of birchwood xylan (Sigma Chemical Co., Inc., St. Louis, MO, USA) by xylan-degrading enzyme(s) under the following typical conditions: 1 ml reactions, 5 mg/ml substrate (total solids), 5 mg of xylanolytic protein/g of substrate, 50 mM sodium acetate pH 5, 50°C, 24 hours, sugar analysis using p-hydroxybenzoic acid hydrazide (PHBAH) assay as described by Lever, 1972, A new reaction for colorimetric determination of carbohydrates, Anal. Biochem 47: 273-279.

**Xylanase:** The term "xylanase" means a 1,4-beta-D-xylan-xylohydrolase (E.C. 3.2.1.8) that catalyzes the endohydrolysis of 1,4-beta-D-xylosidic linkages in xylans. For purposes of the present invention, xylanase activity is determined with 0.2% AZCL-arabinoxylan as substrate in 0.01% Triton X-100 and 200 mM sodium phosphate buffer pH 6 at 37°C. One unit of xylanase activity is defined as 1.0 μmole of azurine produced per minute at 37°C, pH 6 from 0.2% AZCL-arabinoxylan as substrate in 200 mM sodium phosphate pH 6 buffer.

**Beta-xylosidase:** The term "beta-xylosidase" means a beta-D-xyloside xylohydrolase (E.C. 3.2.1.37) that catalyzes the exo-hydrolysis of short beta (1→4)-xylooligosaccharides, to remove successive D-xylose residues from the non-reducing termini. For purposes of the present disclosure one unit of beta-xylosidase is defined as 1.0 of p-nitrophenolate anion produced per minute at 40°C, pH 5 from 1 mM p-nitrophenyl-beta-D-xyloside as substrate in 100 mM sodium citrate containing 0.01% TWEEN® 20.

**Esterase:** The term "esterase" means a hydrolase enzyme that splits esters into an acid and an alcohol in a chemical reaction with water call hydrolysis. The term also refers to enzyme referred to as carboxylic ester hydrolyases, referring to enzymes acting on ester bonds, and includes enzymes classified in EC 3.1.1 carboxylic ester hydrolases according to Enzyme Nomenclature (available at http://www.chem.qmw.ac.uk/iubmb/enzyme or from Enzyme Nomenclature 1992, Academic Press, San Diego, California, with Supplement 1 (1993), Supplement 2 (1994), Supplement 3 (1995), Supplement 4 (1997) and Supplement 5, in Eur. J. Biochem. 1994, 223, 1-5; Eur. J. Biochem. 1995, 232, 1-6; Eur. J. Biochem. 1996, 237, 1-5; Eur. J. Biochem. 1997, 250; 1- 6, and Eur. J. Biochem. 1999, 264, 610-650; respectively). Non-limiting examples of esterases include carboxylesterase, arylesterase, triacylglycerol lipase, acetylesterase, acetylcholinesterase, cholinesterase, tropinesterase, pectinesterase, sterol esterase, chlorophyllase, L- arabinonolactonase, gluconolactonase, uronolactonase, tannase, retinyl-palmitate esterase, hydroxybutyrate-dimer hydrolase, acylglycerol lipase, 3-oxoadipate enol-lactonase, 1,4- lactonase, galactolipase, 4-pyridoxolactonase, acylcarnitine hydrolase, aminoacyl-tRNA hydrolase, D-arabinonolactonase, 6-phosphogluconolactonase, phospholipase A1 , 6- acetylglucose deacetylase, lipoprotein lipase, dihydrocoumarin lipase, limonin-D-ring-lactonase, steroid-lactonase, triacetate-lactonase, actinomycin lactonase, orsellinate- depside hydrolase, cephalosporin-C deacetylase, chlorogenate hydrolase, alpha-amino-acid esterase, 4-methyloxaloacetate esterase, carboxymethylenebutenolidase, deoxylimonate A- ring-lactonase, 2-acetyl-1-alkylglycerophosphocholine esterase, fusarinine-C ornithinesterase, sinapine esterase, wax-ester hydrolase, phorbol-diester hydrolase, phosphatidylinositol deacylase, sialate O-acetylesterase, acetoxybutynylbithiophene deacetylase, acetylsalicylate deacetylase, methylumbelliferyl-acetate deacetylase, 2-pyrone- 4,6-dicarboxylate lactonase, N-acetylgalactosaminoglycan deacetylase, juvenile-hormone esterase, bis(2-ethylhexyl)phthalate esterase, protein-glutamate methylesterase, 11-cis- retinyl-palmitate hydrolase, all-trans-retinyl-palmitate hydrolase, L-rhamnono-1,4-lactonase, 5-(3,4-diacetoxybut-1-ynyl)-2,2'-bithiophene deacetylase, fatty-acyl-ethyl-ester synthase, xylono-1 ,4-lactonase, N-acetylglucosaminylphosphatidylinositol deacetylase, cetraxate benzylesterase, acetylalkylglycerol acetylhydrolase, and acetylxylan esterase. Non-limiting examples of esterase include carboxylic ester hydrolases classified in EC 3.1.1.1 through and including EC3.1.1.85 according to the Enzyme Nomenclature (available at a website having the address www.chem.qmw.ac.uk/iubmb/enzyme). Esterases have wide specificity; and also may hydrolyze vitamin A esters. Esterases may also come from microsomes that also catalyze the reactions of EC 3.1.1.2, EC 3.1.1.5, EC 3.1.1.6, EC 3.1.1.23, EC 3.1.1.28, EC 3.1.2.2, EC 3.5.1.4, and EC 3.5.1.13.

**Acetylxylan esterase:** The term "acetylxylan esterase" means a carboxylesterase (EC 3.1.1.72) that catalyzes the hydrolysis of acetyl groups from polymeric xylan, acetylated xylose, acetylated glucose, alpha-napthyl acetate, and p-nitrophenyl acetate. For purposes of the present disclosure acetylxylan esterase activity is determined using 0.5 mM p-nitrophenylacetate as substrate in 50 mM sodium acetate pH 5.0 containing 0.01% TWEEN™ 20. One unit of acetylxylan esterase is defined as the amount of enzyme capable of releasing 1 of p-nitrophenolate anion per minute at pH 5, 25°C.

**Feruloyl esterase:** The term "feruloyl esterase" means a 4-hydroxy-3-methoxycinnamoyl-sugar hydrolase (EC 3.1.1.73) that catalyzes the hydrolysis of the 4-hydroxy-3-methoxycinnamoyl (feruloyl) group from an esterified sugar, which is usually arabinose in "natural" substrates, to produce ferulate (4-hydroxy-3-methoxycinnamate). Feruloyl esterase is also known as ferulic acid esterase, hydroxycinnamoyl esterase, FAE-III, cinnamoyl ester hydrolase, FAEA, cinnAE, FAE-I, or FAE-II. Non-limiting examples of feruloyl esterase for use in accordance with the present disclosure are set forth below. For purposes of the present disclosure feruloyl esterase activity is determined using 0.5 mM *p*-nitrophenylferulate as substrate in 50 mM sodium acetate pH 5.0. One unit of feruloyl esterase equals the amount of enzyme capable of releasing 1 of *p*-nitrophenolate anion per minute at pH 5, 25°C.

**Alpha-glucuronidase:** The term "alpha-glucuronidase" means an alpha-D-glucosiduronate glucuronohydrolase (EC 3.2.1.139) that catalyzes the hydrolysis of an alpha-D-glucuronoside to D-glucuronate and an alcohol. For purposes of the present disclosure alpha-glucuronidase activity is determined according to de Vries, 1998, J. Bacteriol. 180: 243-249. One unit of alpha-glucuronidase equals the amount of enzyme capable of releasing 1 of glucuronic or 4-O-methylglucuronic acid per minute at pH 5, 40°C.

**Alpha-L-arabinofuranosidase:** The term "alpha-L-arabinofuranosidase" means an alpha-L-arabinofuranoside arabinofuranohydrolase (EC 3.2.1.55) that catalyzes the hydrolysis of terminal non-reducing alpha-L-arabinofuranoside residues in alpha-L-arabinosides. The enzyme acts on alpha-L-arabinofuranosides, alpha-L-arabinans containing (1,3)- and/or (1,5)-linkages, arabinoxylans, and arabinogalactans. Alpha-L-arabinofuranosidase is also known as arabinosidase, alpha-arabinosidase, alpha-L-arabinosidase, alpha-arabinofuranosidase, polysaccharide alpha-L-arabinofuranosidase, alpha-L-arabinofuranoside hydrolase, L-arabinosidase, or alpha-L-arabinanase. For purposes of the present disclosure alpha-L-arabinofuranosidase activity is determined using 5 mg of medium viscosity wheat arabinoxylan (Megazyme International Ireland, Ltd., Bray, Co. Wicklow, Ireland) per ml of 100 mM sodium acetate pH 5 in a total volume of 200 $\mu$l for 30 minutes at 40°C followed by arabinose analysis by AMINEX® HPX-87H column chromatography (Bio-Rad Laboratories, Inc., Hercules, CA, USA).

**Cellulosic material:** The term "cellulosic material" means any material containing cellulose. The predominant polysaccharide in the primary cell wall of biomass is cellulose, the second most abundant is hemicellulose, and the third is pectin. The secondary cell wall, produced after the cell has stopped growing, also contains polysaccharides and is strengthened by polymeric lignin covalently cross-linked to hemicellulose. Cellulose is a homopolymer of anhydrocellobiose and thus a linear beta-(1-4)-D-glucan, while hemicelluloses include a variety of compounds, such as xylans, xyloglucans, arabinoxylans, and mannans in complex branched structures with a spectrum of substituents. Although generally polymorphous, cellulose is found in plant tissue primarily as an insoluble crystalline matrix of parallel glucan chains. Hemicelluloses usually hydrogen bond to cellulose, as well as to other hemicelluloses, which help stabilize the cell wall matrix.

Cellulose is generally found, for example, in the stems, leaves, hulls, husks, and cobs of plants or leaves, branches, and wood of trees. The cellulosic material can be, but is not limited to, agricultural residue, herbaceous material (including energy crops), municipal solid waste, pulp and paper mill residue, waste paper, and wood (including forestry residue) (see, for example, Wiselogel et al., 1995, in Handbook on Bioethanol (Charles E. Wyman, editor), pp.105-118, Taylor & Francis, Washington D.C.; Wyman, 1994, Bioresource Technology 50: 3-16; Lynd, 1990, Applied Biochemistry and Biotechnology 24/25: 695-719; Mosier et al., 1999, Recent Progress in Bioconversion of Lignocellulosics, in Advances in Biochemical Engineering/Biotechnology, T. Scheper, managing editor, Volume 65, pp.23-40, Springer-Verlag, New York). It is understood herein that the cellulose may be in the form of lignocellulose, a plant cell wall material containing lignin, cellulose, and hemicellulose in a mixed matrix. In a preferred aspect, the cellulosic material is any biomass material. In another preferred aspect, the cellulosic material is lignocellulose, which comprises cellulose, hemicelluloses, and lignin.

In one aspect, the cellulosic material is agricultural residue. In another aspect, the cellulosic material is herbaceous material (including energy crops). In another aspect, the cellulosic material is municipal solid waste. In another aspect, the cellulosic material is pulp and paper mill residue. In another aspect, the cellulosic material is waste paper. In another aspect, the cellulosic material is wood (including forestry residue).

In another aspect, the cellulosic material is arundo. In another aspect, the cellulosic material is bagasse. In another aspect, the cellulosic material is bamboo. In another aspect, the cellulosic material is corn cob. In another aspect, the cellulosic material is corn fiber. In another aspect, the cellulosic material is corn stover. In another aspect, the cellulosic material is miscanthus. In another aspect, the cellulosic material is orange peel. In another aspect, the cellulosic material is rice straw. In another aspect, the cellulosic material is switchgrass. In another aspect, the cellulosic material is wheat straw.

In another aspect, the cellulosic material is aspen. In another aspect, the cellulosic material is eucalyptus. In another aspect, the cellulosic material is fir. In another aspect, the cellulosic material is pine. In another aspect, the cellulosic material is poplar. In another aspect, the cellulosic material is spruce. In another aspect, the cellulosic material is willow.

In another aspect, the cellulosic material is algal cellulose. In another aspect, the cellulosic material is bacterial cellulose. In another aspect, the cellulosic material is cotton linter. In another aspect, the cellulosic material is filter paper. In another aspect, the cellulosic material is microcrystalline cellulose. In another aspect, the cellulosic material is phosphoric-acid treated cellulose.

In another aspect, the cellulosic material is an aquatic biomass. As used herein the term "aquatic biomass" means biomass produced in an aquatic environment by a photosynthesis process. The aquatic biomass can be algae, emergent plants, floating-leaf plants, or submerged plants.

The cellulosic material may be used as is or may be subjected to pretreatment, using conventional methods known in the art, as described herein. In a preferred aspect, the cellulosic material is pretreated.

**Pretreated Cellulosic material:** The term pretreated cellulosic material means any cellulosic material that has been treated in preparation for further processing. Non-limiting examples of pretreated cellulosic material includes cellulosic material treated by one or more chemical, enzymatic, mechanical, or physical pre-treatment steps in preparation for enzymatic hydrolysis. In another aspect, pretreatment includes re-pulping of woody biomass.

**Pretreated corn stover:** The term "PCS" or "Pretreated Corn Stover" means a cellulosic material derived from corn stover by treatment with heat. In some aspects cellulosic material derived from corn stover by treatment with heat is also treated with dilute acid.

**Isolated:** The term "isolated" means a substance in a form or environment which does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated (e.g., multiple copies of a gene encoding the substance; use of a stronger promoter than the promoter naturally associated with the gene encoding the substance).

**Mature polypeptide:** The term "mature polypeptide" means a polypeptide in its final form following translation and any post-translational modifications, such as N-terminal processing, C-terminal truncation, glycosylation, phosphorylation, etc. It is known in the art that a host cell may produce a mixture of two of more different mature polypeptides (*i.e.*, with a different C-terminal and/or N-terminal amino acid) expressed by the same polynucleotide. The mature polypeptide can be predicted using the SignalP program (Nielsen et al., 1997, Protein Engineering 10: 1-6).

**Mature polypeptide coding sequence:** The term "mature polypeptide coding sequence" is defined herein as a nucleotide sequence that encodes a mature polypeptide having biological activity. The mature polypeptide coding sequence can be predicted using the SignalP program (Nielsen *et al.*, 1997, *supra*).

**Sequence Identity:** The relatedness between two amino acid sequences or between two nucleotide sequences is described by the parameter "sequence identity".

For purposes of the present disclosure the degree of sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows: (Identical Residues x 100)/(Length of Alignment - Total Number of Gaps in Alignment).

For purposes of the present disclosure the degree of sequence identity between two deoxyribonucleotide sequences is determined using the Needleman-Wunsch algorithm (Needleman and Wunsch, 1970, *supra*) as implemented in the Needle program of the EMBOSS package (EMBOSS: The European Molecular Biology Open Software Suite, Rice et al., 2000, *supra*), preferably version 3.0.0 or later. The optional parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

(Identical Deoxyribonucleotides x 100)/(Length of Alignment – Total Number of Gaps in Alignment)

**Polypeptide fragment:** The term "fragment" means a polypeptide having one or more (several) amino acids deleted from the amino and/or carboxyl terminus of a mature polypeptide; wherein the fragment has biological activity.

**Subsequence:** The term "subsequence" means a polynucleotide having one or more (several) nucleotides deleted from the 5' and/or 3' end of a mature polypeptide coding sequence, wherein the subsequence encodes a fragment having biological activity.

**Allelic variant:** The term "allelic variant" means any of two or more alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene.

**Coding sequence:** The term "coding sequence" means a polynucleotide, which directly specifies the amino acid sequence of a polypeptide. The boundaries of the coding sequence are generally determined by an open reading frame, which usually begins with the ATG start codon or alternative start codons such as GTG and TTG and ends with a stop codon such as TAA, TAG, and TGA. The coding sequence may be a DNA, cDNA, synthetic, or recombinant polynucleotide.

**cDNA:** The term "cDNA" means a DNA molecule that can be prepared by reverse transcription from a mature, spliced, mRNA molecule obtained from a eukaryotic or prokaryotic cell. cDNA lacks intron sequences that may be present in the corresponding genomic DNA. The initial, primary RNA transcript is a precursor to mRNA that is processed through a series of steps, including splicing, before appearing as mature spliced mRNA.

**Nucleic acid construct:** The term "nucleic acid construct" means a nucleic acid molecule, either single- or double-stranded, which is isolated from a naturally occurring gene or is modified to contain segments of nucleic acids in a manner that would not otherwise exist in nature or which is synthetic. The term nucleic acid construct is synonymous with the term "expression cassette" when the nucleic acid construct contains the control sequences required for expression of a coding sequence.

**Control sequences:** The term "control sequences" means all components necessary for the expression of a polynucleotide encoding a polypeptide. Each control sequence may be native or foreign to the polynucleotide encoding the polypeptide or native or foreign to each other. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding a polypeptide.

**Operably linked:** The term "operably linked" means a configuration in which a control sequence is placed at an appropriate position relative to the coding sequence of a polynucleotide such that the control sequence directs the expression of the coding sequence.

**Expression:** The term "expression" includes any step involved in the production of a polypeptide including, but not limited to, transcription, post-transcriptional modification, translation, post-translational modification, and secretion.

**Expression vector:** The term "expression vector" means a linear or circular DNA molecule that comprises a polynucleotide encoding a polypeptide and is operably linked to additional nucleotides that provide for its expression.

**Host cell:** The term "host cell" means any cell type that is susceptible to transformation, transfection, transduction, and the like with a nucleic acid construct or expression vector comprising a polynucleotide. The term "host cell" encompasses any progeny of a parent cell that is not identical to the parent cell due to mutations that occur during replication.

**Variant:** The term "variant" means a polypeptide comprising an alteration, *i.e.*, a substitution, insertion, and/or deletion of one or more (several) amino acid residues at one or more (several) positions. A substitution means a replacement of an amino acid occupying a position with a different amino acid; a deletion means removal of an amino acid occupying a position; and an insertion means adding one or more (several) amino acids, e.g., 1-5 amino acids, adjacent to an amino acid occupying a position.

**Lipase:** The term lipase means a polypeptide having lipase activity. The term "lipase activity" as used herein means a carboxylic ester hydrolase activity which catalyses the hydrolysis of triacylglycerol under the formation of diacylglycerol and a carboxylate. On another aspect, the term "lipase activity" as used herein can also mean a carboxylic ester hydrolase activity which catalyses the hydrolysis of diacylglycerol under the formation of monoacylglycerol and a carboxylate. On another aspect, the term "lipase activity" as used herein can also means a carboxylic ester hydrolase activity which catalyses the hydrolysis of monoacylglycerol under the formation of glycerol and a carboxylate.

**Protease:** The term "protease" means a polypeptide having protease activity. The term "protease activity" is defined herein as a proteolytic activity which catalyzes the hydrolysis of the peptide bond connecting two amino acids in a peptide. Protease activity can be measured using any assay, in which a substrate is employed, that includes peptide bonds relevant for the specificity of the protease in question. The term protease further includes any enzyme belonging to the EC 3.4 enzyme group (including each of the thirteen subclasses thereof, these enzymes being in the following referred to as "belonging to the EC 3.4.-.-group"). The EC number refers to Enzyme Nomenclature 1992 from NC-IUBMB, Academic Press, San Diego, California, including supplements 1-5 published in Eur. J. Biochem. 1994, 223: 1-5; Eur. J. Biochem. 1995, 232: 1-6; Eur. J. Biochem. 1996, 237: 1-5; Eur. J. Biochem. 1997, 250: 1-6; and Eur. J. Biochem. 1999, 264: 610-650; respectively. The nomenclature is regularly supplemented and updated; see e.g. the World Wide Web at www.chem.qmw.ac.uk/iubmb/enzyme/index.html.

**Pectinase:** The term pectinase means a polypeptide having pectinase activity such that it can hydrolyze a pectic substance. The term can include any of the pectinolytic enzymes as described in Jayani et al., 2005, Microbial pectinolytic enzymes:A review, Process Biochemistry 40: 2931-2944. Various assay methods for determining pectinase activity are set out in Jayani et al., 2005, Microbial pectinolytic enzymes:A review, Process Biochemistry 40: 2931-2944.

**Mannanase:** The term "mannanase" or "galactomannanase" denotes a mannanase enzyme named mannan endo-1,4-beta-mannosidase and having the alternative names beta-mannanase and endo-1,4-mannanase and catalysing hydrolyses of 1,4-beta-D-mannosidic linkages in mannans, galactomannans, glucomannans, and galactoglucomannans which enzyme is classified according to the Enzyme Nomenclature as EC 3.2.1.78 (see, *e.g.*, the website address www. expasy. ch/enzyme). A polypeptide of the present disclosure having mannanase activity may be tested for mannanase activity according to standard test procedures known in the art, such as, for example, by applying a solution to be tested to 4 mm diameter holes punched out in agar plates containing 0.2% AZCL galactomannan (carob), *i.e.* substrate for the assay of endo-1,4-beta-D-mannanase available as Cat No. I-AZGMA from the company Megazyme (Megazyme's Internet address: www. megazyme. com).

## Detailed Description of the Invention

One aspect of the present disclosure relates to a method for increasing cellulolytic enzyme activity during the hydrolysis of pretreated cellulosic material including, comprising or consisting of: (a) contacting the cellulosic material with one or more cutinase, peptidase and pectate lyase enzymes to form pretreated cellulosic material; (b) hydrolyzing the pretreated cellulosic material with one or more enzyme compositions, wherein the step of hydrolyzing includes contacting the pretreated cellulosic material with one or more amylase and glucoamylase and/or mannanase enzymes,.

The present invention also relates to processes for hydrolysing pretreated cellulosic material, comprising: treating the cellulosic material with an enzyme composition in the presence of a polypeptide having amylase, and glucoamylase, and/or mannanase activity, wherein the cellulosic material was pretreated with cutinase, peptidase and pectate lyase enzyme.

The present invention also relates to a method for producing a fermentation product, comprising : (a) saccharifying the pretreated cellulosic material with an enzyme composition comprising one or more cellulolytic enzymes, and at least one or more amylases and glucoamylases and/or mannanase enzymes; (b) fermenting the saccharified pretreated cellulosic material with one or more (several) fermenting microorganisms to produce the fermentation product; and (c) recovering the degraded or converted cellulosic material, wherein the cellulosic material was pretreated with cutinase, peptidase and pectate lyase. Soluble products of degradation or conversion of the cellulosic material can be separated from insoluble cellulosic material using a method known in the art such as, for example, centrifugation, filtration, or gravity settling.

## Pretreatment of cellulosic material

In some aspects the cellulosic material and/or the lignocellulose-containing material may according to the present disclosure be pre-treated before being hydrolyzed and fermented. Cellulosic material suitable for use in accordance with the present disclosure is pretreated with any suitable method known in the art. The goal of pretreatment is to separate and/or release cellulose, hemicellulose and/or lignin and this way improve the rate of enzymatic hydrolysis.

Without wishing to be bound by the present disclosure it is believed that conventional pretreatment produces impurities in the pretreated cellulosic materials and may have a deleterious effect on cellulase enzymes and/or enzyme hydrolysis.

Pretreatment of cellulosic material includes any conventional pre-treatment step known in the art. Pre-treatment may take place in aqueous slurry or may be directly applied to the cellulosic material in raw form. In some aspects, the cellulose containing material may during pretreatment be present in an amount between 2-80 wt. %, for example between 20-50 wt. % of the total weight of the pretreatment reaction. In embodiments, conventional pretreatment is improved by including pretreatment in accordance with the present disclosure, e.g., contacting the cellulosic material with one or more cutinase, peptidase and pectate lyase enzymes to form pretreated cellulosic material.

Chemical, Mechanical and/or Biological Pre-treatment

Non-limiting examples of pretreatment of cellulosic material according to the present disclosure includes chemically, mechanically and/or biologically pre-treating cellulosic material before hydrolysis and/or fermentation. Mechanical treatment (often referred to as physical pre-treatment) may be used alone or in combination with subsequent or simultaneous hydrolysis, especially enzymatic hydrolysis, to promote the separation and/or release of cellulose, hemicellulose and/or lignin.

In some aspects the chemical, mechanical and/or biological pre-treatment is carried out prior to the hydrolysis and/or fermentation. Alternatively, the chemical, mechanical and/or biological pre-treatment is carried out simultaneously with hydrolysis, such as simultaneously with addition of one or more cellulolytic enzymes, or other enzyme activities mentioned below, to release fermentable sugars, such as glucose and/or maltose.

In an aspect of the present disclosure the pre-treated cellulosic material is washed and/or detoxified in accordance with the present disclosure before, during or after the hydrolysis step. This may improve the fermentability of, *e.g.*, dilute-acid hydrolyzed lignocellulose-containing material, such as corn stover.

In embodiments in accordance with the present disclosure detoxification is carried out by contacting the pretreated cellulosic material with an enzyme or enzyme composition including cutinase, peptidase and pectate lyase to form an enzymatically pretreated cellulosic material.

Chemical Pre-treatment

According to the present disclosure "chemical pre-treatment" refers to any chemical treatment that promotes the separation and/or release of cellulose, hemicellulose and/or lignin. Non-limiting examples of suitable chemical pre-treatment steps include treatment with, for example, dilute acid, lime, alkaline, organic solvent, ammonia, sulphur dioxide, carbon dioxide. Further, wet oxidation and pH-controlled hydrothermolysis are also contemplated chemical pre-treatments.

In some aspects, the chemical pre-treatment is acid treatment, for example, a continuous dilute and/or mild acid treatment, such as, treatment with sulfuric acid, or another organic acid, such as acetic acid, citric acid, tartaric acid, succinic acid, or mixtures thereof. Other acids may also be used. Mild acid treatment means in the context of the present disclosure that the treatment pH lies in the range from 1-5, for example from pH 1-3. In a one aspect the acid concentration is in the range from 0.1 to 2.0 wt % acid, for example sulphuric acid. In embodiments, the acid concentration is in the range from 0.1 to 70.0 wt % acid. Non-limiting examples include 10, 20, 30, 40, 50, 60, 70 wt % acid including but not limited to highly concentrated hydrochloric acid. The acid may be mixed or contacted with the material to be fermented according to the present disclosure and the mixture may be held at a temperature in the range of 160-220°C, for example 165-195°C, for periods ranging from minutes to seconds, e.g., 1-60 minutes, for example 2-30 minutes or 3-12 minutes. Addition of strong acids, such as sulphuric acid, may be applied to remove hemicellulose. This enhances the digestibility of cellulose.

Cellulose solvent treatment, also contemplated according to the present disclosure, has been shown to convert about 90% of cellulose to glucose. It has also been shown that enzymatic hydrolysis could be greatly enhanced when the lignocellulosic structure is disrupted. Alkaline $H_2O_2$, ozone, organosolv (uses Lewis acids, $FeCl_3$, $(Al)_2SO_4$ in aqueous alcohols), glycerol, dioxane, phenol, or ethylene glycol are among solvents known to disrupt cellulose structure and promote hydrolysis (Mosier et al., 2005, Bioresource Technology 96: 673-686).

Alkaline chemical pre-treatment with base, *e.g.*, NaOH, $Na_2CO_3$ and/or ammonia or the like, is also within the scope of the present disclosure. Pre-treatment methods using ammonia are described in, e.g., WO 2006/110891, WO 2006/110899, WO 2006/110900, WO 2006/110901.

Wet oxidation techniques involve use of oxidizing agents, such as: peroxide based oxidizing agents or the like. Wet oxidation techniques can also involve use of reducing agents, such as: sulphite based reducing agents or the like. Non-limiting examples of solvent pre-treatments include treatment with DMSO (Dimethyl Sulfoxide) or the like. Chemical pre-treatment is generally carried out for 1 to 60 minutes, such as from 5 to 30 minutes, but may be carried out for shorter or longer periods of time dependent on the material to be pre-treated.

Other non-limiting examples of suitable pre-treatment methods are described by Schell et al., 2003, Appl. Biochem and Biotechn. 105-108: 69-85, and Mosier et al., 2005, Bioresource Technology 96: 673-686, and US publication no. 2002/0164730,.

In accordance with some aspects of the present disclosure chemically pretreated cellulosic materials are detoxified by contacting the pretreated cellulosic material with an enzyme or enzyme composition including cutinase, peptidase and pectate lyase to form a pretreated cellulosic material.

Mechanical Pre-treatment

As used in context of the present disclosure the term "mechanical pre-treatment" refers to any mechanical or physical pre-treatment which promotes the separation and/or release of cellulose, hemicellulose and/or lignin from lignocellulose-containing material. Non-limiting examples of mechanical pre-treatment includes various types of milling, irradiation, steaming/steam explosion, pulping and hydrothermolysis.

Mechanical pre-treatment includes comminution (mechanical reduction of the particle size). Comminution includes dry milling, wet milling and vibratory ball milling. Mechanical pre-treatment may involve high pressure and/or high temperature (steam explosion). In an aspect of the present disclosure high pressure means pressure in the amount of 300 to 600 psi, for example 400 to 500 psi, or for example around 450 psi. In an aspect of the present disclosure high temperature means temperatures in the amount of from about 100 to 300°C, for example from about 140 to 235°C. In some aspects, mechanical pre-treatment is a batch-process, steam gun hydrolyzer system which uses high pressure and high temperature as defined above. A Sunds Hydrolyzer (available from Sunds Defibrator AB (Sweden) may be used for this.

In accordance with some aspects of the present disclosure mechanically pretreated cellulosic materials are detoxified by contacting the pretreated cellulosic material with an enzyme or enzyme composition including cutinase, peptidase and pectate lyase to form a pretreated cellulosic material.

Combined Chemical and Mechanical Pre-treatment

In some aspects of the present disclosure, both chemical and mechanical pre-treatments are carried out involving, for example, both dilute or mild acid pretreatment and high temperature and pressure treatment. The chemical and mechanical pretreatment may be carried out sequentially or simultaneously, as desired.

Accordingly, in some aspects the cellulose containing material is subjected to both chemical and mechanical pre-treatment to promote the separation and/or release of cellulose, hemicellulose and/or lignin.

In some aspects the pre-treatment is carried out as a dilute and/or mild acid steam explosion step. In some aspects, pre-treatment is carried out as an ammonia fiber explosion step (or AFEX pretreatment step).

In accordance with some aspects of the present disclosure combined chemical and mechanical pretreated cellulosic materials are detoxified by contacting the pretreated cellulosic material with an enzyme or enzyme composition including cutinase, peptidase and pectate lyase to form a pretreated cellulosic material.

Biological Pre-treatment

As used in the present disclosure the term "biological pre-treatment" refers to any biological pre-treatment which promotes the separation and/or release of cellulose, hemicellulose, and/or lignin from the lignocellulose-containing material. Biological pre-treatment techniques can involve applying lignin-solubilizing microorganisms (see, for example, Hsu, T.-A., 1996, Pretreatment of biomass, in Handbook on Bioethanol: Production and Utilization, Wyman, C. E., ed., Taylor & Francis, Washington, DC, 179-212; Ghosh and Singh, 1993, Physicochemical and biological treatments for enzymatic/microbial conversion of lignocellulosic biomass, Adv. Appl. Microbiol. 39: 295-333; McMillan, J. D., 1994, Pretreating lignocellulosic biomass: a review, in Enzymatic Conversion of Biomass for Fuels Production, Himmel, M. E., Baker, J. O., and Overend, R. P., eds., ACS Symposium Series 566, American Chemical Society, Washington, DC, chapter 15; Gong, C. S., Cao, N. J., Du, J., and Tsao, G. T., 1999, Ethanol production from renewable resources, in Advances in Biochemical Engineering/Biotechnology, Scheper, T., ed., Springer-Verlag Berlin Heidelberg, Germany, 65: 207-241; Olsson and Hahn-Hagerdal, 1996, Fermentation of lignocellulosic hydrolysates for ethanol production, Enz. Microb. Tech. 18: 312-331; and Vallander and Eriksson, 1990, Production of ethanol from lignocellulosic materials: State of the art, Adv. Biochem. Eng./Biotechnol. 42: 63-95).

In some aspects, biological pre-treatment involves applying lignin degrading enzymes to lignin or pretreated material. Non-limiting examples of suitable lignin degrading enzymes include one or more lignolytic enzymes, one or more oxidoreductases, and combinations thereof. Non-limiting examples of lignolytic enzymes include manganese peroxidase, lignin peroxidase and cellobiose dehydrogenase, and combinations thereof. Non-limiting examples of suitable pretreatment enzymes also include one ore more laccases, cellobiose dehydrogenases and combinations thereof.

In one aspect, lignin peroxidase such as "ligninase", EC number 1.14.99, is suitable for use in accordance with the present disclosure.

In one aspect, Ethazyme™ Pre available from Zymetis is suitable for use in pretreatment in accordance with the present disclosure.

In accordance with embodiments of the present disclosure biologically pretreated cellulosic material is detoxified by contacting the cellulosic material with an enzyme and/or enzyme composition including cutinase, peptidase and pectate lyase pretreated cellulosic material.

Pretreatment embodiments in accordance with the present disclosure

The present disclosure relates to pretreatment and to pretreated compositions. The pretreatment is suitable for use as a stand-alone pretreatment method, or to improve pretreatment methods known in the art. More specifically, the present disclosure relates to a method of pretreating cellulosic material such as woody biomass by contacting the cellulosic material with one or more cutinase, peptidase and pectate lyase enzymes thereof to form pretreated cellulosic material. Without wishing to be bound by the present disclosure it is believed that conventional pretreatment of cellulosic material increases impurities within the cellulosic material that can diminish enzyme hydrolysis of the material. For examples, fats, esters, proteins and pectin can be present in the cellulosic material or pretreated cellulosic material in an amount sufficient to have a negative affect on the enzymes such as cellulases used in hydrolysis. The present disclosure provides enzymes and enzyme compositions and methods for treating, removing or eliminating toxins in the cellulosic material. The method includes applying a predetermined amount of cutinase, peptidase and pectate lyase to cellulosic material in need of treatment, including cellulosic material with cellulase inhibiting amounts of toxins therein.

Accordingly, cutinase, peptidase and pectate lyase mixtures and/or compositions in accordance with the present disclosure provide a treatment of one or more cellulase inhibiting substance(s) in which the major active ingredient is cutinase, peptidase and pectate lyase enzyme. In embodiments, compositions in accordance with the present disclosure include cutinase, peptidase and pectate lyase in a commercially available form.

In embodiments, cutinase, peptidase and pectate lyase or compositions thereof in accordance with the present disclosure can be applied to cellulosic material in need of improvement e.g., such as the reduction or elimination of an undesirable cellulase inhibiting substance(s) such one or more esters, proteins, or pectins. As used herein the word "treat," "treating" or "treatment" refers to using the one or more cutinase, peptidase and pectate lyase or compositions thereof of the present disclosure prophylactically to prevent cellulase inhibiting substance(s) from accumulating in cellulosic material or pretreated cellulosic material, or to ameliorate an existing condition, and/or promote or extend the cellulase activity of cellulase enzyme or enzyme compositions used to hydrolyze the pretreated cellulosic material. A number of different treatments are now possible, which reduce and/or eliminate cellulase inhibiting substance(s) from the cellulose material such as woody biomass.

Treatments in accordance with the present disclosure contact cellulose material, such as woody biomass with one or more active cutinase, peptidase and pectate lyase enzymes in accordance with the present disclosure in an effective amount to improve the toxic conditions. The lipase, protease and/or pectinase ingredient or composition is applied until the treatment goals are obtained. However, the duration of the treatment can vary depending on the severity of the toxic condition or amount of toxins present in the sample. For example, treatments can last several minutes (non-limiting examples include 5, 10, 15, 20, 30, 60, 120 minutes) to days ( non-limiting examples include 1 day, 2 days, 3 days, 4 days, 5 days), depending on whether the goal of treatment is to reduce or eliminate the cellulase inhibiting condition.

In embodiments, the cutinase, peptidase and pectate lyase enzyme or compositions thereof comprises, or consists of one or more cutinase, peptidase and pectate lyase enzymes. The cutinase, peptidase and pectate lyase compositions can comprise any cutinase, peptidase and pectate lyase protein that is useful in detoxifying a cellulosic material such as woody biomass.

A lipase that is suitable for use in accordance with the present disclosure that refers generally to any enzyme or polypeptide having lipase activity. A lipase may be a carboxylic ester hydrolase EC 3.1.1.-, which includes activities such as EC 3.1.1.3 triacylglycerol lipase, EC 3.1.1.4 phospholipase A2, EC 3.1.1.5 lysophospholipase, EC 3.1.1.26 galactolipase, EC 3.1.1.32 phospholipase A1, EC 3.1.1.73 feruloyl esterase, and/or EC 3.1.1.74 cutinase.

As used herein, the EC number refers to Enzyme Nomenclature 1992 from NC-IUBMB, Academic Press, San Diego, Calif., including supplements 1-5 published in Eur. J. Biochem., 1994, 223: 1-5; Eur. J. Biochem., 1995, 232: 1-6; Eur. J. Biochem., 1996, 237: 1-5; Eur. J. Biochem., 1997, 250: 1-6; and Eur. J. Biochem., 1999, 264: 610-650; respectively. The nomenclature is regularly supplemented and updated; see, e.g., the World Wide Web at www.chem.qmw.ac.uk/iubmb/enzyme/index.html.

In embodiments according to the present invention, cutinases are used to form pretreated cellulosic material. Cutinases are lipolytic enzymes capable of hydrolyzing the substrate cutin. Cutinases are known from various fungi (P.E. Kolattukudy in "Lipases", Ed. B. Borgström and H.L. Brockman, Elsevier 1984, 471-504). The amino acid sequence and the crystal structure of a cutinase of *Fusarium solani pisi* have been described (Longhi et al., Journal of Molecular Biology, 268 (4), 779-799 (1997)). The amino acid sequence of a cutinase from *Humicola insolens* has also been published in U.S. Patent No. 5,827,719. A number of variants of the cutinase of *Fusarium solani pisi* suitable for use in accordance with the present disclosure have been published: WO 94/14963; WO 94/14964; WO 00/05389; Appl. Environm. Microbiol. 64: 2794-2799, 1998; Proteins: Structure, Function and Genetics 26: 442-458, 1996; J. of Computational Chemistry 17: 1783-1803, 1996; Protein Engineering 6: 157-165, 1993; Proteins: Structure, Function, and Genetics 33: 253-264, 1998; J. of Biotechnology 66: 11-26, 1998; Biochemistry 35: 398-410, 1996; Chemistry and Physics of Lipids 97: 181-191, 1999; Proteins: Structure, Function, and Genetics 31: 320-333, 1998; Biochimica et Biophysica Acta 1441: 185-196, 1999; Appl. Environm. Microbiol. 64: 316-324, 1998; BioTechniques 27: 1102-1108, 1999.

Suitable lipases for use in accordance with the present disclosure include STICKAWAY™ brand enzyme available from Novozymes A/S.

In various aspects suitable for use in accordance with the present disclosure, the parent enzyme is a cutinase classified as EC 3.1.1.74 according to Enzyme Nomenclature (see for example, the website available at www.chem.qmw.ac.uk/iubmb/enzyme. Such aspects include a fungal cutinase, such as a filamentous fungal cutinase, *e.g.* native to a strain of *Humicola* or *Fusarium,* specifically *H. insolens* or *F. solani pisi,* more specifically *H. insolens* strain DSM 1800. SEQ ID NO: 1 of U.S. Patent No. 6,960,459 shows the amino acid sequence of the cutinase of *H. insolens* strain DSM 1800 (the mature peptide) and the numbering system used herein for the *H. insolens* cutinase. The amino acid sequence and the DNA sequence encoding it were previously published as SEQ ID NO: 2 and SEQ ID NO: 1 of US Patent No. 5,827,719.

The amino acid sequence of the cutinase of *F. solani pisi* is shown as the mature peptide in Fig. 1D of WO 94/14964,. The numbering system used for the *F. solani pisi* cutinase is that used in WO 94/14964; it includes the pro-sequence shown in said Fig. 1D; thus, the mature cutinase is at positions 16-215.

In various aspects, suitable for use herein, the parent cutinase may have an amino acid sequence which is at least 50% (particularly at least 70%, at least 80%, at least 90%, at least 95% or at least 99%) homologous to the cutinase of *H. insolens* strain DSM 1800. The parent cutinase may particularly be one that can be aligned with the cutinase of *H.*

*insolens* strain DSM 1800.

In some aspects, variants of fungal cutinases are suitable for use in accordance with the present disclosure. For example variants suitable for use in accordance with the present disclosure include variants described in U.S. Patent No. 6,960,459. In particular, variant cutinases suitable for use in accordance with the present disclosure include variants identified in Col. 4 of U.S. Patent No. 6,960,459. In some aspects, cutinase include those which exhibit a high sequence identity to any variants identified in Col. 4 of U.S. Patent No. 6,960,459, e.g., at least 70%, at least 75%, at least 80%, at least 85%, at least 90%, at least 95%, at least 96%, at least 97%, at least 98%, at least 99% or even 100% sequence identity to the mature enzyme sequences.

In some aspects, cutinase activity is determined as in U.S. Patent No. 7,833,771.

In some aspects, cutinase activity is determined as lipolytic activity determined using tributyrin as substrate. This method was based on the hydrolysis of tributyrin by the enzyme, and the alkali consumption is registered as a function of time. One Lipase Unit (LU) is defined as the amount of enzyme which, under standard conditions (*e.g.* at 30°C.; pH 7; with Gum Arabic as emulsifier and tributyrine as substrate) liberates 1 micro mol titrable butyric acid per minute.

In some aspects lipases suitable for use according to the present disclosure include a microbial lipase. As such, the lipase may be selected from yeast, e.g., *Candida*; bacteria, e.g., *Pseudomonas* or *Bacillus*; or filamentous fungi, e.g., *Humicola* or *Rhizomucor.* More specifically, suitable lipases may be the *Rhizomucor miehei* lipase (e.g., prepared as described in EP 238 023), *Thermomyces lanuginosa* lipase e.g., prepared as described in EP 305 216, *Humicola insolens* lipase, *Pseudomonas stutzeri* lipase, *Pseudomonas cepacia* lipase, *Candida antarctica* lipase A or B, or lipases from rGPL, *Absidia blakesleena*, *Absidia corymbifera*, *Fusarium solani*, *Fusarium oxysporum, Penicillum cyclopium, Penicillum crustosum, Penicillum expansum, Rhodotorula glutinis, Thiarosporella phaseolina, Rhizopus microsporus, Sporobolomyces shibatanus, Aureobasidium pullulans, Hansenula anomala, Geotricum penicillatum, Lactobacillus curvatus, Brochothrix thermosohata, Coprinus cinerius, Trichoderma harzanium, Trichoderma reesei, Rhizopus japonicus*, or *Pseudomonas plantari.* Other non-limiting examples of suitable lipases may be variants of any one of the lipases mentioned above, e.g., as described in WO 92/05249 or WO 93/11254.

Non-limiting examples of commercially available lipases include Lipex™, Lipoprime™, Lipopan™, Lipopan F™, Lipopan Xtra™, Lipolase™, Lipolase™ Ultra, Lipozyme™, Palatase™, Resinase™, Novozym™ 435 and Lecitase™ (all available from Novozymes A/S). Other commercially available lipases include Lumafast™ (*Pseudomonas mendocina* lipase from Genencor International Inc.); Lipomax™ (*Ps. pseudoalcaligenes* lipase from Genencor Int. Inc.; and *Bacillus* sp. lipase from Solvay enzymes. Further lipases are available from other suppliers such as Lipase P "Amano" (Amano Pharmaceutical Co. Ltd.).

Suitable lipase for use in accordance with the present disclosure are lipase described in U.S. Patent Publication Nos. 2010/0034797, 2009/0029440, 2011/0053822 and/or 2010/0279915.

In some aspects, lipase activity is determined according to the following procedure: A substrate for lipase is prepared by emulsifying tributyrin (glycerin tributyrate) using gum Arabic as emulsifier. The hydrolysis of tributyrin at 30°C at pH 7 or 9 is followed in a pH-stat titration experiment. One unit of lipase activity (1 LU) is defined as the amount of enzyme capable of releasing 1 micro mol of butyric acid per minute at 30°C, pH 7.

In some aspects lipase include those which exhibit a high sequence identity to any of above mention lipases, e.g., at least 70%, at least 75%, at least 80%, at least 85%, at least 90%, at least 95%, at least 96%, at least 97%, at least 98%, at least 99% or even 100% sequence identity to the mature enzyme sequences.

In some aspects, cutinase for use in accordance with the present disclosure include the mature polypeptide of SEQ ID NO: 1, and fragments thereof having cutinase activity. In embodiments, cutinase include those which exhibit a high sequence identity to SEQ ID NO: 1, e.g., at least 70%, at least 75%, at least 80%, at least 85%, at least 90%, at least 95%, at least 96%, at least 97%, at least 98%, at least 99% or even 100% sequence identity to the mature enzyme sequence.

Protease enzymes are suitable for use in accordance with the present disclosure. For example, a protease may be added during pretreatment of cellulosic material such as woody biomass. The protease may be any protease. In some aspects the protease is an acid protease of microbial origin, for example of fungal or bacterial origin. In some aspects, an acid fungal protease is suitable for use in accordance with the present disclosure, but also other proteases can be used.

Non-limiting examples of suitable proteases include microbial proteases, for example fungal and bacterial proteases. In some aspects proteases are acidic proteases, *e.g.*, proteases characterized by the ability to hydrolyze proteins under acidic conditions below pH 7.

Non-limited examples of acid fungal proteases include fungal proteases derived from *Aspergillus, Mucor, Rhizopus, Candida, Coriolus, Endothia, Enthomophtra, Irpex, Penicillium, Sclerotiumand Torulopsis.* Additional non-limiting examples include proteases derived from *Aspergillus niger* (see, *e.g.*, Koaze et al., 1964, Agr. Biol. Chem. Japan 28: 216), *Aspergillus saitoi* (see, *e.g.*, Yoshida, 1954, J. Agr. Chem. Soc. Japan 28: 66), *Aspergillus awamori* (Hayashida et al., 1977, Agric. Biol. Chem. 42(5): 927-933, *Aspergillus aculeatus* (WO 95/02044), or *Aspergillus oryzae*, such as the pepA protease; and acidic proteases from *Mucor pusillus* or *Mucor miehei.*

Additional non-limiting examples of proteases include neutral or alkaline proteases, for example a protease derived from a strain of *Bacillus.* A particular protease contemplated for the present disclosure is protease derived from *Bacillus amyloliquefaciens* and has the sequence obtainable at Swissprot as Accession No. P06832. Also contemplated are the proteases having at least 90% sequence identity to amino acid sequence obtainable at Swissprot as Accession No. P06832 such as at least 92%, at least 95%, at least 96%, at least 97%, at least 98%, or particularly at least 99% sequence identity.

Non-limiting examples of proteases also include the proteases having at least 90% sequence identity to amino acid sequence disclosed as SEQ.ID.NO:1 in the WO 2003/048353 such as at 92%, at least 95%, at least 96%, at least 97%, at least 98%, or particularly at least 99% sequence identity.

Non-limiting examples of proteases also include papain-like proteases such as proteases within E.C. 3.4.22.* (cysteine protease), such as EC 3.4.22.2 (papain), EC 3.4.22.6 (chymopapain), EC 3.4.22.7 (asclepain), EC 3.4.22.14 (actinidain), EC 3.4.22.15 (cathepsin L), EC 3.4.22.25 (glycyl endopeptidase) and EC 3.4.22.30 (caricain).

In some aspects the protease is a protease preparation derived from a strain of *Aspergillus,* for example *Aspergillus oryzae.* In another embodiment the protease is derived from a strain of *Rhizomucor,* for example *Rhizomucor mehei.* In another embodiment the protease is a protease preparation, for example a mixture of a proteolytic preparation derived from a strain of *Aspergillus,* (*e.g.,Aspergillus oryzae*) and a protease derived from a strain of *Rhizomucor,* for example *Rhizomucor mehei.*

Other suitable proteases include aspartic acid proteases for example those described in, Hand-book of Proteolytic Enzymes, Edited by A.J. Barrett, N.D. Rawlings and J.F. Woessner, Aca-demic Press, San Diego, 1998, Chapter 270). Non-limiting examples of aspartic acid protease include, *e.g.*, those disclosed in Berka et al., 1990, Gene 96: 313; Berka et al., 1993, Gene 125: 195-198; and Gomi et al., 1993, Biosci. Biotech. Biochem. 57: 1095-1100.

Non-limiting examples of commercially available protease products include ALCALASE®, ESPERASE™, FLAVOURZYME™, PROMIX™, NEUTRASE®, RENNILASE®, NOVOZYM™ FM 2.0L, and NOVOZYM™ 50006 (available from Novozymes A/S, Denmark) and GC106™ and SPEZYME™ FAN from Genencor Int., Inc., USA. Additional enzymes include FERMGEN™ and GC 212 from Genencor.

In embodiments of the present disclosure, serine proteases are used to form pretreated cellulosic material. Suitable serine proteases include those of animal, vegetable or microbial origin. It may be a serine protease, preferably an alkaline microbial protease or a trypsin-like protease. Examples of serine proteases are subtilisins, especially those derived from *Bacillus*, *e.g.*, subtilisin Novo, subtilisin Carlsberg, subtilisin BPN subtilisin 309, subtilisin 147 and subtilisin 168 (described in WO 89/06279). Examples of trypsin-like proteases are trypsin (*e.g.*, of porcine or bovine origin) and the *Fusarium* protease described in WO 89/06270.

Non-limiting examples of commercially available serine proteases (peptidases) include Kannase™, Everlase™, Esperase™, Alcalase™, Neutrase™, Durazym™, Savinase™, Savinase™ Ultra, Ovozyme™, Liquanase™, Polarzyme™, Pyrase™, Pancreatic Trypsin NOVO (PTN), Bio-Feed™ Pro and Clear-Lens™ Pro (all available from Novozymes A/S, Bagsvaerd, Denmark). Preferred serine proteases include those described in WO 1998/020115, WO 01/44452, WO 01/58275, WO 01/58276, WO 2003/006602, and WO 2004/099401. Other commercially available serine proteases include Ronozyme™ Pro, Maxatase™, Maxacal™, Maxapem™, Opticlean™, Properase™, Purafect™, Purafect Ox™ and Purafact Prime™ (available from Genencor International Inc., BASF, or DSM Nutritional Products.

Non-limiting protease examples include those described in U.S. Patent Publication Nos. 2011/0028378, 2010/0322915, and/or 2010/0304433.

In some aspects protease include those which exhibit a high sequence identity to any of above mention proteases, *e.g.*, at least 70%, at least 75%, at least 80%, at least 85%, at least 90%, at least 95%, at least 96%, at least 97%, at least 98%, at least 99% or even 100% sequence identity to the mature enzyme sequences.

In some aspects, protease activity is determined according to the procedure described by Sawada et al., 1983, Experientia 39: 377. One unit of protease activity is defined as 1.0 micro-mole of 7-amino-4-methylcoumarin liberated from substrate Suc- Leu- Leu- Val- Tyr- MCA (available at Peptide Inc. (Osaka, Japan), with the product code: 3120-v) per minute at 25°C., pH 8. In embodiments, protease activity is determined using known methods in the art, including but not limited to those described in U.S. published patent application no. 2011/0158976.

In some aspects, proteases for use in accordance with the present disclosure include the mature polypeptide of SEQ ID NO: 2, and fragments thereof having protease activity. In embodiments, protease include those which exhibit a high sequence identity to SEQ ID NO: 2, e.g., at least 70%, at least 75%, at least 80%, at least 85%, at least 90%, at least 95%, at least 96%, at least 97%, at least 98%, at least 99% or even 100% sequence identity to the mature enzyme sequence.

In some aspects, protease may be provided in stabilized compositions, such as those described in U.S. Patent No. 5,972,873 and U.S. Patent Publication No. 20070060493.

In some aspects, pectinase is suitable for use in accordance with the present disclosure which refers generally to any pectinase, in particular of microbial origin, in particular of bacterial origin, such as a pectinase derived from a species within the genera *Bacillus, Clostridium, Pseudomonas, Xanthomonas* and *Erwinia*, or of fungal origin, such as a pectinase derived from a species within the genera *Aspergillus,* in particular from a strain within the species *A. niger* and *A. aculeatus.* Contemplated non-limiting commercially available pectinases include BIOPREP™, NOVOZYM™ 863, PEXTINEX™ 3XL, PECTINEX™ SMASH, and PECTINEX™ SMACH XXL, BIOCIP™ MEMBRANE and combinations of these (all available from Novozymes A/S, Denmark).

In some aspects the pectinase is PECTINEX™ ULTRA SP-L, including an *Aspergillus aculeatus* pectate lyase from Novozymes A/S, Denmark is suitable for use in accordance with the present disclosure.

In some aspects the pectinase is a polygalacturonase (EC 3.2.1.15), pectinesterase (EC 3.2.1.11), or pectin lyase (EC4.2.2.10). A suitable source organism for pectinases may be *Aspergillus niger.*

In embodiments, the pectin lyase of the present disclosure is used to form pretreated cellulosic material. Pectin lyases are pectinases that catalyze eliminative cleavage of (1.4)-alpha-D-galacturonan methyl ester to give oligosaccharides with 4-deoxy-6-O-methyl-alpha-D-galact-4-enuronosyl groups at their non-reducing ends. They are alternatively called pectolyase, polymethylgalacturonic transeliminase, pectin methyltranseliminase, pectin trans-eliminase etc.

The pectin lyase enzymatic reaction consists of splitting alpha 1-4 galacturonosidyl bond producing unsaturated delta 4,5 uronide. The double bond with carbonyl function in C6 has an absorption in U.V. Optical density at 235 nm assays the pectin lyase activity.

One Pectin lyase (PL) unit is the quantity of enzyme that catalyses the split of bound endo alpha 1-4 galacturonosidyl (C6 Methyl ester) forming one micromole of delta 4,5 unsaturated product in one minute, according to described conditions of 45°C and pH 5.5.

For the purposes of the disclosure, the source of the above enzymes including pectin lyase, pectate lyase and pectinesterase is not critical, e.g., the enzymes may be obtained from a plant, an animal, or a microorganism such as a bacterium or a fungus, *e.g.*, a filamentous fungus or a yeast. The enzymes may, *e.g.*, be obtained from these sources by use of recombinant DNA techniques as is known in the art. The enzymes may be natural or wild-type enzymes, or any mutant, variant, or fragment thereof exhibiting the relevant enzyme activity, as well as synthetic enzymes, such as shuffled enzymes, and consensus enzymes. Such genetically engineered enzymes can be prepared as is generally known in the art. *e.g.* by site-directed mutagenesis, by PCR (using a PCR fragment containing the desired mutation as one of the primers in the PCR reactions), or by Random Mutagenesis. The preparation of consensus proteins is described in, *e.g.*, EP 897985.

The pectinase may be a component occurring in an enzyme system produced by a given micro-organism, such an enzyme system mostly comprising several different pectinase components including those identified above.

Alternatively, the pectinase may be a single component, *i.e.*, a component essentially free of other pectinase enzymes which may occur in an enzyme system produced by a given micro-organism, the single component typically being a recombinant component, *i.e.*, produced by cloning of a DNA sequence encoding the single component and subsequent cell transformed with the DNA sequence and expressed in a host. Such useful recombinant enzymes., especially pectinase, pectin lyases and polygalacturonases are described in detail in, *e.g.*, WO 93/20193. WO 02/092741, WO 03/095638 and WO 2004/092479 (from Novozymes A/S). The host is preferably a heterologous host, but the host may under certain conditions also be the homologous host.

In a preferred aspect the pectinase used according to the invention is derived from the genus *Aspergillus.*

In a still preferred aspect, the pectinase is the protopectinase having an amino acid sequence of SEQ ID NO: 1 of JP 11682877 or the protopectinase having an amino acid sequence generated by deletion, substitution or insertion of one amino acid or several amino acids in the amino acid sequence and having an activity at the same level as or a higher level than the level of the activity of the protopectinase with the amino acid sequence of SEQ ID NO: 1 of JP 11682877.

In some aspects, pectinase include those which exhibit a high sequence identity to any of above mention pectinases, e.g., at least 70%, at least 75%, at least 80%, at least 85%, at least 90%, at least 95%, at least 96%, at least 97%, at least 98%, at least 99% or even 100% sequence identity to the mature enzyme sequences.

In some aspects, pectate lyases for use in accordance with the present disclosure include the mature polypeptide of SEQ ID NO: 3, and fragments thereof having pectate lyase activity. In embodiments, pectate lyase include those which exhibit a high sequence identity to SEQ ID NO: 3, *e.g.*, at least 70%, at least 75%, at least 80%, at least 85%, at least 90%, at least 95%, at least 96%, at least 97%, at least 98%, at least 99% or even 100% sequence identity to the mature enzyme sequence.

Cutinase, peptidase and pectate lyase in accordance with the present disclosure may include amino acid changes that are of a minor nature, that is conservative amino acid substitutions or insertions that do not significantly affect the folding and/or activity of the protein; small deletions, typically of one to about 30 amino acids; small amino- or carboxyl-terminal extensions, such as an amino-terminal methionine residue; a small linker peptide of up to about 20-25 residues; or a small extension that facilitates purification by changing net charge or another function, such as a polyhistidine tract, an antigenic epitope or a binding domain.

Examples of conservative substitutions are within the group of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. The most commonly occurring exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

Alternatively, the amino acid changes are of such a nature that the physico-chemical properties of the polypeptides are altered. For example, amino acid changes may improve the thermal stability of the polypeptide, alter the substrate specificity, change the pH optimum, and the like.

Essential amino acids in a parent polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for cellulolytic enhancing activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et a/., 1992, J. Mol. Biol. 224: 899-904; Wlodaver et al., 1992, FEBS Lett. 309: 59-64. The identities of essential amino acids can also be inferred from analysis of identities with polypeptides that are related to the parent polypeptide.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (*e.g.*, Lowman et al., 1991, Biochemistry 30: 10832-10837; U.S. Patent No. 5,223,409; WO 92/06204), and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

In some aspects, the total number of amino acid substitutions, deletions and/or insertions of the mature polypeptide of cutinase, peptidase and pectate lyase in accordance with the present disclosure is not more than 10, *e.g.,* 1, 2, 3, 4, 5, 6, 7, 8, 9 or 10.

In some aspects, suitable lipase enzyme compositions for use in accordance with the present disclosure comprise or consist of STICKAWAY® brand enzyme from Novozymes A/S Denmark, or fragments thereof have lipase activity. In embodiments, lipase include those which exhibit a high sequence identity to the above mentioned lipase, *e.g.*, at least 70%, at least 75%, at least 80%, at least 85%, at least 90%, at least 95%, at least 96%, at least 97%, at least 98%, at least 99% or even 100% sequence identity to the mature enzyme sequences.

In some aspects, a suitable protease enzyme composition for use in accordance with the present disclosure comprises, or consists of Savinase® Ultra 16XL brand enzyme from Novozymes A/S Denmark or fragments thereof have protease activity. In embodiments, protease include those which exhibit a high sequence identity to the above mentioned lipase, e.g., at least 70%, at least 75%, at least 80%, at least 85%, at least 90%, at least 95%, at least 96%, at least 97%, at least 98%, at least 99% or even 100% sequence identity to the mature enzyme sequences.

In some aspects, a suitable pectinase enzyme composition for use in accordance with the present disclosure comprises, or consists of Pectinex Ultra SP-L brand enzyme from Novozymes A/S Denmark or fragments thereof have pectinase activity. In embodiments, pectinase include those which exhibit a high sequence identity to the above mentioned lipase, e.g., at least 70%, at least 75%, at least 80%, at least 85%, at least 90%, at least 95%, at least 96%, at least 97%, at least 98%, at least 99% or even 100% sequence identity to the mature enzyme sequences.

In embodiments, combinations of one or more cutinase, peptidase and pectate lyase may be contacted with the cellulosic material, including pretreated cellulosic material. For example, in some aspects, suitable combinations of one or more mature polypeptides of including SEQ ID NOS: 1, 2 and 3 may be contacted with cellulosic material or pretreated cellulosic material. Suitable combinations also include mature polypeptides which exhibit a high sequence identity to SEQ ID NOS: 1, 2 and 3, *e.g.*, at least 70%, at least 75%, at least 80%, at least 85%, at least 90%, at least 95%, at least 96%, at least 97%, at least 98%, at least 99% or even 100% sequence identity to the mature lipase, protease, and pectinase enzyme sequences. The enzyme combinations are added in amounts sufficient to provide a benefit to the cellulosic material.

One or more (several) components of the cutinase, peptidase and pectate lyase enzyme or cutinase, peptidase and pectate lyase composition for use in accordance with the present disclosure may be wild-type proteins, recombinant proteins, or a combination of wild-type proteins and recombinant proteins. For example, one or more (several) components may be native proteins of a cell, which is used as a host cell to express recombinantly one or more (several) other components of the lipase, protease and/or pectinase composition. One or more (several) components of the lipase, protease and/or pectinase composition may be produced as monocomponents, which are then combined to form the enzyme composition. The enzyme composition may be a combination of multicomponent and monocomponent protein preparations.

The cutinase, peptidase and pectate lyase used in the processes of the present invention may be in any form suitable for use, such as, for example, a crude fermentation broth with or without cells removed, a cell lysate with or without cellular debris, a semi-purified or purified enzyme preparation, or a host cell as a source of the lipase, protease and/or pectinases. The lipase, protease and/or pectinase composition may be a dry powder or granulate, a non-dusting granulate, a liquid, a stabilized liquid, or a stabilized protected enzyme. Liquid lipase, protease and/or pectinase preparations may, for instance, be stabilized by adding stabilizers such as a sugar, a sugar alcohol or another polyol, and/or lactic acid or another organic acid according to established processes.

The cutinase, peptidase and pectate lyase can be derived or obtained from any suitable origin, including, bacterial, fungal, yeast, plant, or mammalian origin. The term "obtained" means herein that the cutinase, peptidase and pectate lyase may have been isolated from an organism that naturally produces the cutinase, peptidase and pectate lyase as a native enzyme. The term "obtained" also means herein that the enzyme may have been produced recombinantly in a host organism employing methods described herein, wherein the recombinantly produced cutinase, peptidase and pectate lyase is either native or foreign to the host organism or has a modified amino acid sequence, *e.g.*, having one or more (several) amino acids that are deleted, inserted and/or substituted, *i.e.,* a recombinantly produced enzyme that is a mutant and/or a fragment of a native amino acid sequence or an enzyme produced by nucleic acid shuffling processes known in the art. Encompassed within the meaning of a native enzyme are natural variants and within the meaning of a foreign enzyme are variants obtained recombinantly, such as by site-directed mutagenesis or shuffling.

Treatments in accordance with the present disclosure are not limited to direct treatment of the cellulosic material. It is envisioned that processes in accordance with the present disclosure include, comprise, or consist of treating and/or post-treating pretreated cellulosic material that was subject to other enzymatic pre-treatments, chemical pre-treatments, mechanical pre-treatments and/or a physical pretreatments or combinations of these.

In embodiments, the contacting or treating with enzyme or enzyme compositions such as cutinase, peptidase and pectate lyase is performed with a sufficient amount of enzyme per gram (g) of cellulosic material. In some aspects, compositions for use in accordance with the present invention contain cutinase, peptidase and pectate lyase enzyme in an effective amount to improve hydrolysis of the cellulosic material such as woody biomass. As used herein "effective amount" refers to an amount of cutinase, peptidase and pectate lyase or cutinase, peptidase and pectate lyase composition having cutinase, peptidase and pectate lyase constituents in accordance with the present disclosure sufficient to induce a particular positive benefit to the cellulosic material or portion thereof. The positive benefit can relate to cellulase inhibition, or it can relate more to the nature of enzyme hydrolysis, or it may be a combination of the two. In embodiments, the positive benefit is achieved by contacting the cellulosic material or a portion thereof with one or more cutinase, peptidase and pectate lyase or compositions thereof to improve the cellulosic materials condition in order to improve its hydrolysis performance. For example, the amount cutinase, peptidase and pectate lyase enzyme added in accordance with the present disclosure includes an amount sufficient to detoxify the pretreated cellulosic material such that hydrolysis thereof can be improved. Non-limiting examples of improvements include a reduction of toxins in the cellulosic material such as woody biomass and/or an increase in the amount of sugar formed during the hydrolysis of the material. In embodiments, the amount of toxins in the pretreated cellulosic material is reduced by an amount of 1-10%, 20%, 30%, 40%, 50%, 60%, 70%, 80%, 90% or 100%. The reduction of toxins can be performed by any suitable analytical method known in the art such as HPLC. In embodiments, the amount of toxins in the cellulosic material is reduced by an amount of: 10-30% of the total amount of toxins present, 20-40% of the total amount of toxins present, 40-50% of the total amount of toxins present, 50-60% of the total amount of toxins present, 60-70% of the total amount of toxins present, 70-80% of the total amount of toxins present, 80-90% of the total amount of toxins present, or 90-100% of the total amount of toxins present. In embodiments, the improvement could refer to an increased amount of hydrolysis product, such as sugar, greater than the amount of hydrolysis product produced compared to the use of cellulosic material hydrolyzed but not treated in accordance with the present disclosure. In embodiments, the amount of hydrolysis product or sugar is increased 1.1, 1.2, 1.3, 1.4, 1.5, 1.6, 1.7, 1.8, 1.9 or 2 times more than the amount of hydrolysis product produced compared to use of cellulosic material hydrolyzed, but not treated in accordance with the present disclosure. In embodiments, the amount of hydrolysis product or sugar is increased 3, 4, 5, 6, 7, 8, 8, 9 or 10 times more than the amount of hydrolysis product produced compared to use of cellulosic material hydrolyzed but not treated in accordance with the present disclosure. Non-limiting suitable amounts of enzyme such as cutinase for use in accordance with the present disclosure include contacting the cutinase with about 0.0005 to about 5 mg, *e.g.*, about 0.001 to about 5 mg, about 0.0025 to about 5 mg, about 0.005 to about 5 mg, about 0.005 to about 4.5 mg, about 0.005 to about 4 mg, about 0.005 to about 3.5 mg, about 0.005 to about 3 mg, about 0.005 to about 2 mg, about 0.005 to about 1 mg, about 0.075 to about 1 mg, or about 0.1 to about 1 mg of lipase per g of cellulosic material. Non-limiting suitable amounts of enzyme such as peptidase for use in accordance with the present disclosure include contacting the peptidase with about 0.0005 to about 5 mg, *e.g.*, about 0.001 to about 5 mg, about 0.0025 to about 5 mg, about 0.005 to about 5 mg, about 0.005 to about 4.5 mg, about 0.005 to about 4 mg, about 0.005 to about 3.5 mg, about 0.005 to about 3 mg, about 0.005 to about 2 mg, about 0.005 to about 1 mg, about 0.075 to about 1 mg, or about 0.1 to about 1 mg of protease per g of cellulosic material. Non-limiting suitable amounts of enzyme such as pectate lyase for use in accordance with the present disclosure include contacting the pectate lyase with about 0.0005 to about 5 mg, *e.g.*, about 0.001 to about 5 mg, about 0.0025 to about 5 mg, about 0.005 to about 5 mg, about 0.005 to about 4.5 mg, about 0.005 to about 4 mg, about 0.005 to about 3.5 mg, about 0.005 to about 3 mg, about 0.005 to about 2 mg, about 0.005 to about 1 mg, about 0.075 to about 1 mg, or about 0.1 to about 1 mg of pectinase per g of cellulosic material. Enzyme mixtures including combinations of these amounts are also suitable for use in accordance with the present disclosure.

In some aspects, treatments in accordance with the present invention comprise, consist of, or include an amount of cellulosic material sufficient to be useful. For example, treatments include contacting of cellulosic material with an amount of cellulosic material such that treatments are performed with a total solids (TS) of about 1% to about 50% e.g., about 2% to about 40%, about 2% to about 35%, about 3% to about 30%, about 3% to about 25%, about 4% to about 20%, or about 5% to about 10%. In embodiments, treatments are performed with a total solids (TS) of about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49% or 50%. The total solids (TS) can relate to biomass or filtrate in the reaction.

In embodiments, the contacting or treating the cellulosic material with cutinase, peptidase and pectate lyase or with a cutinase, peptidase and pectate lyase composition is performed at a pH suitable for the lipase, protease, and/or pectinase enzyme. Non-limiting examples of suitable pH's include contacting or the treating with the cutinase, peptidase and pectate lyase at a pH of about 2 to about 9, *e.g.*, about 3 to about 8, about 3 to about 7.5, about 3.5 to about 7, about 4 to about 6.5, about 4.5 to about 6.5, about 4.5 to about 6.0, about 5 to about 6.0, or about 5 to about 5.5. In embodiments the pH is 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9 or 9.0. In some aspects, examples of suitable pH's include contacting or the treating with the cutinase, peptidase and pectate lyase or with a cutinase, peptidase and pectate lyase enzyme composition at a pH of 2 to 9, e.g., 3 to 8, 3 to 7.5, 3.5 to 7, 4 to 6.5, 4.5 to 6.5, 4.5 to 6.0, 5 to 6.0, or 5 to 5.5.

In some aspects, the contacting or treating the cellulosic material with cutinase, peptidase and pectate lyase or with a cutinase, peptidase and pectate lyase composition is performed at a temperature suitable for the cutinase, peptidase and pectate lyase enzyme. Non-limiting examples of suitable temperatures include a temperature in the range of about 20°C to about 70°C, *e.g.*, about 25°C to about 65°C, about 30°C to about 65°C, about 35°C to about 65°C, about 40°C to about 60°C, about 45°C to about 55°C, or about 45°C to about 50°C. In embodiments, a suitable temperature is 46°C, 47°C, 48°C, 49°C, 50°C, 51°C, 52°C, 53°C, 54°C, 55°C, 56°C, 57°C, 58°C, 59°C, 60°C, 61°C, 62°C, 63°C, 64°C, 65°C, 66°C, 67°C, 68°C, 69°C, 70°C.

In some aspects, the contacting or treating the pretreated cellulosic material with one or more cutinase, peptidase and pectate lyase enzyme composition is performed for a duration suitable for the one or more cutinase, peptidase and pectate lyase enzyme to react on toxins in the cellulosic material such as woody biomass. As used herein toxins refer generally to substances that inhibit cellulase and/or hydrolysis. Non-limiting examples of suitable durations include a period of time of 5 minutes to 35 hours, *e.g.*, 10 minutes to 15 hours, 10 hours to 15 hours, 10 hours to 20 hours, 10 hours to 20 hours, 20 hours to 24 hours, 24 hours to 30 hours, 1 hour to 72 hours, 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours or 20 hours.

In some aspects, the contacting with one or more cutinase, peptidase and pectate lyase is performed with a total solids (TS) of 6%, a pH of 7, at a temperature of 50°C for about 16 hours. TS refers to either biomass or filtrate used in the reaction.

The lipase, protease, pectinase treatment is generally performed in tanks under controlled pH, temperature, and conditions as described herein. In embodiments, the contacting or treating the cellulosic material with cutinase, peptidase and pectate lyase enzyme or enzyme composition is performed with an amount of cellulosic material described herein, with an amount of enzyme such as cutinase, peptidase and pectate lyase described herein, at a pH, temperature and duration in accordance with the present disclosure. Various modification of the amounts used herein can be used to optimize the performance of the lipase, protease, pectinase in removing the toxins and/or increasing enzyme hydrolysis yields. In some aspects, cutinase, peptidase and pectate lyase treatment is preferably carried out in a suitable aqueous environment under conditions that can be readily determined by one skilled in the art. In a preferred aspect, cutinase, peptidase and pectate lyase treatment is performed under conditions suitable for the activity of the cutinase, peptidase and pectate lyase *i.e.,* optimal for the enzymes and mixtures thereof. The treatment(s) can be carried out as a fed batch or continuous process where the cellulosic material (substrate) is fed gradually to, for example, cutinase, peptidase and pectate lyase containing solution.

In embodiments, the present disclosure relates to a process for hydrolyzing a pretreated cellulosic material comprising or consisting of saccharifying a cellulosic material with an enzyme composition, wherein the cellulosic material was pretreated by contacting the cellulosic material with one or more cutinase, peptidase and pectate lyase enzymes to form pretreated cellulosic material. In embodiments the hydrolysis is carried out using enzyme composition including one or more (several) enzymes selected from the group consisting of a cellulase, a GH61 polypeptide having cellulolytic enhancing activity, a hemicellulase, an expansin, an esterase, a laccase, a ligninolytic enzyme, a pectinase, a peroxidase, a protease, and a swollenin. In embodiments, the cellulase is one or more (several) enzymes selected from the group consisting of an endoglucanase, a cellobiohydrolase, and a beta-glucosidase. In embodiments, the hemicellulase is one or more (several) enzymes selected from the group consisting of a xylanase, an acetyxylan esterase, a feruloyl esterase, an arabinofuranosidase, a xylosidase, a beta-xylosidase and a glucuronidase. In embodiments in accordance with the present disclosure the enzyme composition comprises or consists of one or more amylase or mannanase enzymes, which may be referred to as secondary enzymes herein.

In embodiments, the method of the present disclosure further includes the steps comprising or consisting of recovering the saccharified pretreated cellulosic material from the saccharification. In embodiments, the saccharified cellulosic material is a sugar. Non-limiting examples of sugars include glucose, xylose, mannose, galactose, and arabinose.

In embodiments, the present disclosure relates to a method for producing a fermentation product, comprising or consisting of: (a) saccharifying a pretreated cellulosic material, treated with one or more cutinase, peptidase and pectate lyase enzymes or compositions thereof in accordance with the present disclosure. Here saccharification is performed using an enzyme composition suitable for saccharification. In embodiments, enzymes suitable for saccharification include one or more (several) enzymes selected from the group consisting of amylase, glucoamylase, cellulase, a GH61 polypeptide having cellulolytic enhancing activity, a hemicellulase, an expansin, an esterase, a laccase, a ligninolytic enzyme, a mannanase, a pectinase, a peroxidase, a protease, and a swollenin. In embodiments, cellulase is one or more (several) enzymes selected from the group consisting of an endoglucanase, a cellobiohydrolase, and a beta-glucosidase. In embodiments, hemicellulase is one or more (several) enzymes selected from the group consisting of a xylanase, an acetyxylan esterase, a feruloyl esterase, an arabinofuranosidase, a xylosidase, and a glucuronidase. The next step includes (b) fermenting the saccharified pretreated cellulosic material with one or more (several) fermenting microorganisms to produce the fermentation product; and (c) recovering the fermentation product from the fermentation. In embodiments, the steps (a) saccharifying a pretreated cellulosic material (wherein the pretreated cellulosic material is contacted, treated or refined in accordance with the present disclosure using one or more cutinase, peptidase and pectate lyase enzymes or compositions thereof) and (b) (fermenting the saccharified pretreated cellulosic material with one or more (several) fermenting microorganisms to produce the fermentation product) are performed simultaneously in a simultaneous saccharification and fermentation. In embodiments, the saccharifying is performed by including beneficial amounts of one or more amylase, glucoamylase and/or mannanase enzymes or compositions thereof in the reaction. In embodiments, the fermentation product is an alcohol, an alkane, a cycloalkane, an alkene, an amino acid, a gas, isoprene, a ketone, an organic acid, or polyketide.

The present disclosure further relates to a method for fermenting a pretreated cellulosic material, comprising or consisting of: fermenting a pretreated cellulosic material with one or more (several) fermenting microorganisms, wherein the pretreated cellulosic material is treated, and/or saccharified according to the present disclosure. For example, the cellulosic material is pretreated by contacting the material with cutinase, peptidase and pectate lyase enzyme or compositions thereof in accordance with the present disclosure, and the saccharification step includes mannanase and amylase enzymes or compositions thereof. In embodiments, the fermenting of the pretreated cellulosic material produces a fermentation product. In embodiments, the method comprises or consists of a step of recovering the fermentation product from the fermentation. In embodiments, the fermentation product is an alcohol, an alkane, a cycloalkane, an alkene, an amino acid, a gas, isoprene, a ketone, an organic acid, or polyketide.

In some aspects, methods of the present disclosure are preferably used on woody biomass. Non-limiting examples of woody cellulosic materials include trees and woody plants, including limbs, needles, leaves and other woody parts. Woody biomass may include things grown in a forest, woodland or range environment that are the by-products of the environment and forest management. In one aspect, the cellulosic material is a wood material. In another aspect, the cellulosic material is a woody forest by-product. In one aspect, the woody biomass is derived from wood waste stream or wood output of a community, region or state. This wood waste can include whole trees, pruned branches, stumps, used lumber, housing trim, construction debris and shipping pallets, or woody office waste such as wood desks or chairs. In one aspect, the cellulosic material is a woody energy crop. In one aspect, woody biomass is derived from wood fiber products, such as papers, cardboards and derived materials. In one aspect, woody biomass is derived from wood fibers, such as recycled papers and cardboards. In one aspect, woody biomass is derived from once dried wood fiber products, such as virgin or recycled papers. In one aspect, the cellulosic material contains at least a fraction of woody biomass. In one aspect of the invention, fully 100% non-woody feedstock is excluded from use as a suitable feedstock or cellulosic material in accordance with the present disclosure.

**Saccharification**

In the hydrolysis step, also known as saccharification, the cellulosic material, *i.e.*, pretreated, is hydrolyzed to break down cellulose and alternatively also hemicellulose to fermentable sugars, such as glucose, cellobiose, xylose, xylulose, arabinose, mannose, galactose, and/or soluble oligosaccharides. The hydrolysis is performed enzymatically by an enzyme composition in the presence of one or more (several) polypeptides having amylase and/or mannanase activity of the present invention. The enzyme and protein components of the compositions can be added sequentially.

Enzymatic hydrolysis is preferably carried out in a suitable aqueous environment under conditions that can be readily determined by one skilled in the art. In one aspect, hydrolysis is performed under conditions suitable for the activity of the enzyme(s), *i.e.,* optimal for the enzyme(s). The hydrolysis can be carried out as a fed batch or continuous process where the cellulosic material is fed gradually to, for example, an enzyme containing hydrolysis solution.

The saccharification is generally performed in stirred-tank reactors or fermentors under controlled pH, temperature, and mixing conditions. Suitable process time, temperature and pH conditions can readily be determined by one skilled in the art. For example, the saccharification can last up to 200 hours, but is typically performed for preferably about 12 to about 96 hours, more preferably about 16 to about 72 hours, and most preferably about 24 to about 48 hours. The temperature is in the range of preferably about 25°C to about 70°C, more preferably about 30°C to about 65°C, and more preferably about 40°C to 60°C, in particular about 50°C. The pH is in the range of preferably about 3 to about 8, more preferably about 3.5 to about 7, and most preferably about 4 to about 6, in particular about pH 5. The dry solids content is in the range of preferably about 5 to about 50 wt. %, more preferably about 10 to about 40 wt. %, and most preferably about 20 to about 30 wt. %.

The optimum amounts of the enzymes depend on several factors including, but not limited to, the mixture of component cellulolytic enzymes, the cellulosic material, the concentration of the cellulosic material, the pretreatment(s) of the cellulosic material, temperature, time, pH, and inclusion of fermenting organism (*e.g.*, yeast for Simultaneous Saccharification and Fermentation).

The enzyme compositions can comprise any protein useful in degrading the cellulosic material.

In one aspect, an effective amount of cellulolytic or hemicellulolytic enzyme protein to cellulosic material is about 0.5 to about 50 mg, preferably at about 0.5 to about 40 mg, more preferably at about 0.5 to about 25 mg, more preferably at about 0.75 to about 20 mg, more preferably at about 0.75 to about 15 mg, even more preferably at about 0.5 to about 10 mg, and most preferably at about 2.5 to about 10 mg per g of cellulosic material.

In another aspect, an effective amount of a GH61 polypeptide having cellulolytic enhancing activity to cellulosic material is about 0.01 to about 50.0 mg, preferably about 0.01 to about 40 mg, more preferably about 0.01 to about 30 mg, more preferably about 0.01 to about 20 mg, more preferably about 0.01 to about 10 mg, more preferably about 0.01 to about 5 mg, more preferably at about 0.025 to about 1.5 mg, more preferably at about 0.05 to about 1.25 mg, more preferably at about 0.075 to about 1.25 mg, more preferably at about 0.1 to about 1.25 mg, even more preferably at about 0.15 to about 1.25 mg, and most preferably at about 0.25 to about 1.0 mg per g of cellulosic material.

In another aspect, an effective amount of a GH61 polypeptide having cellulolytic enhancing activity to cellulolytic enzyme protein is about 0.005 to about 1.0 g, preferably at about 0.01 to about 1.0 g, more preferably at about 0.15 to about 0.75 g, more preferably at about 0.15 to about 0.5 g, more preferably at about 0.1 to about 0.5 g, even more preferably at about 0.1 to about 0.5 g, and most preferably at about 0.05 to about 0.2 g per g of cellulolytic enzyme protein.

In another aspect of the present disclosure, the step of hydrolyzing comprises or consists of contacting the pretreated cellulosic material with one or more amylase and/or mannanase enzymes. In embodiments, these enzymes alone or in combination may be added to the hydrolysis enzyme compositions as set forth in detail below.

Non-limiting suitable amylase for use in accordance with the present disclosure includes, alpha-amylase, bacterial alpha-amylase, bacterial hybrid alpha-amylase, fungal alpha-amylase, fungal hybrid alpha-amylase, commercial alpha-amylases known in the art, carbohydrate-source generating enzyme such as glucoamylase, beta-amylase, maltogenic amylase, and combinations thereof.

In accordance with the present disclosure any alpha-amylase may be used, such as of fungal, bacterial or plant origin. For example the alpha-amylase may be an acid alpha-amylase, e.g., acid fungal alpha-amylase or acid bacterial alpha-amylase. The term "acid alpha-amylase" means an alpha-amylase (E.C. 3.2.1.1) which added in an effective amount has activity optimum at a pH in the range of 3 to 7, or in embodiments from 3.5 to 6, or in embodiments from 4-5.

In some aspects, suitable bacterial alpha-amylase for use in accordance with the present disclosure, include those derived from the genus *Bacillus.*

In some aspects, the *Bacillus* alpha-amylase is derived from a strain of *Bacillus licheniformis, Bacillus amyloliquefaciens, Bacillus subtilis* or *Bacillus stearothermophilus,* but may also be derived from other *Bacillus* sp. Non-limiting examples of contemplated alpha-amylases include the *Bacillus licheniformis* alpha-amylase shown in SEQ ID NO: 4 in WO 99/19467, the *Bacillus amyloliquefaciens* alpha-amylase SEQ ID NO: 5 in WO 99/19467 and the *Bacillus stearothermophilus* alpha-amylase shown in SEQ ID NO: 3 in WO 99/19467. In some aspects, the alpha-amylase may be an enzyme having a sequence identity of at least 60%, or at least 70%, or at least 80%, or at least 90%, or at least 95%, or at least 96%, or at least 97%, or at least 98% or at least 99% to any of the sequences shown in SEQ ID NOS: 1, 2 or 3, respectively, in WO 99/19467.

The *Bacillus* alpha-amylase may also be a variant and/or hybrid, especially one described in any of WO 96/23873, WO 96/23874, WO 97/41213, WO 99/19467, WO 00/60059, and WO 02/10355. Specifically contemplated alpha-amylase variants are disclosed in US patent Nos. 6,093,562, 6,297,038 or US patent no. 6,187,576 and include *Bacillus stearothermophilus* alpha-amylase (BSG alpha-amylase) variants having a deletion of one or two amino acid in positions R179 to G182, or a double deletion disclosed in WO 96/23873 - see *e.g.*, page 20, lines 1-10, for example corresponding to delta(181-182) compared to the wild-type BSG alpha-amylase amino acid sequence set forth in SEQ ID NO:3 disclosed in WO 99/19467 or deletion of amino acids R179 and G180 using SEQ ID NO:3 in WO 99/19467 for numbering. Other non-limiting examples include *Bacillus* alpha-amylases, for example *Bacillus stearothermophilus* alpha-amylase, which have a double deletion corresponding to delta(181-182) and further includes a N193F substitution (also denoted I181* + G182* + N193F) compared to the wild-type BSG alpha-amylase amino acid sequence set forth in SEQ ID NO:3 disclosed in WO 99/19467.

Bacterial hybrid alpha-amylases are suitable for use in accordance with the present disclosure. For example, a hybrid alpha-amylase specifically contemplated comprises 445 C-terminal amino acid residues of the *Bacillus licheniformis* alpha-amylase (shown in SEQ ID NO: 4 of WO 99/19467) and the 37 N-terminal amino acid residues of the alpha-amylase derived from *Bacillus amyloliquefaciens* (shown in SEQ ID NO: 5 of WO 99/19467), with one or more, especially all, of the following substitution: G48A+T49I+G107A+H156Y+A181T+N190F+I201F+A209V+Q264S (using the *Bacillus licheniformis* numbering in SEQ ID NO: 4 of WO 99/19467). Other non-limiting examples include variants having one or more of the following mutations (or corresponding mutations in other Bacillus alpha-amylase backbones): H154Y, A181T, N190F, A209V and Q264S and/or deletion of two residues between positions 176 and 179, preferably deletion of E178 and G179 (using the SEQ ID NO: 5 numbering of WO 99/19467).

In an aspect the bacterial alpha-amylase is dosed in an amount of 0.0005-5 KNU per g DS, or 0.001-1 KNU per g DS, or around 0.050 KNU per g DS.

Fungal alpha-amylases are suitable for use as enzymes in accordance with the present disclosure. Non-limiting examples include alpha-amylases derived from a strain of the genus *Aspergillus,* such as, *Aspergillus oryzae, Aspergillus niger* and *Aspergillis kawachii* alpha-amylases.

In some aspects, acidic fungal alpha-amylase includes a Fungamyl-like alpha-amylase which is derived from a strain of *Aspergillus oryzae.* According to the present disclosure, the term "Fungamyl-like alpha-amylase" indicates an alpha-amylase which exhibits a high sequence identity, e.g. at least 70%, at least 75%, at least 80%, at least 85%, at least 90%, at least 95%, at least 96%, at least 97%, at least 98%, at least 99% or even 100% sequence identity to the mature part of the amino acid sequence shown in SEQ ID NO: 10 in WO 96/23874.

Another non-limiting example of an acid alpha-amylase derived from a strain *Aspergillus niger.* In embodiments the acid fungal alpha-amylase is the one from *Aspergillus niger* disclosed as "AMYA_ASPNG" in the Swiss-prot/TeEMBL database under the primary accession no. P56271 and described in WO 89/01969 (Example 3). In some aspects, a commercially available acid fungal alpha-amylase derived from *Aspergillus niger* is SP288 (available from Novozymes A/S, Denmark) is suitable for use in accordance with the present disclosure.

Other non-limiting examples include contemplated wild-type alpha-amylases include those derived from a strain of the genera *Rhizomucor* and *Meripilus,* or a strain of *Rhizomucor pusillus* (See WO 2004/055178) or *Meripilus giganteus.*

In some aspects, the alpha-amylase is derived from *Aspergillus kawachii* and disclosed by Kaneko et al., 1996, J. Ferment. Bioeng. 81: 292-298 "Molecular-cloning and determination of the nucleotide-sequence of a gene encoding an acid-stable alpha-amylase from *Aspergillus kawachii*"; and further as EMBL: #AB008370.

In some aspects, the fungal alpha-amylase may also be a wild-type enzyme including a starch-binding domain (SBD) and an alpha-amylase catalytic domain (*e.g.*, non-hybrid), or a variant thereof. In embodiments the wild-type alpha-amylase is derived from a strain of *Aspergillus kawachii.*

Fungal hybrid alpha-amylase enzymes are suitable for use in accordance with the present disclosure. In some aspects, the fungal acid alpha-amylase is a hybrid alpha-amylase. Non-limiting examples of fungal hybrid alpha-amylases for use in accordance with the present disclosure include the hybrid alpha-amylases disclosed in WO 2005/003311 or U.S. Patent Publication no. 2005/0054071 (Novozymes) or US patent application No. 60/638,614. A hybrid alpha-amylase may include an alpha-amylase catalytic domain (CD) and a carbohydrate-binding domain/module (CBM), such as a starch binding domain, and optional a linker.

Non-limiting examples of contemplated hybrid alpha-amylases include those disclosed in Table 1 to 5 of the examples in US patent application no. 60/638,614, including Fungamyl variant with catalytic domain JA118 and *Athelia rolfsii* SBD (SEQ ID NO:100 in US 60/638,614), *Rhizomucor pusillus* alpha-amylase with *Athelia rolfsii* AMG linker and SBD (SEQ ID NO:101 in US 60/638,614), *Rhizomucor pusillus* alpha-amylase with *Aspergillus niger* glucoamylase linker and SBD (which is disclosed in Table 5 as a combination of amino acid sequences SEQ ID NO:20, SEQ ID NO:72 and SEQ ID NO:96 in US application no. 11/316,535) or as V039 in Table 5 in WO 2006/069290, and *Meripilus giganteus* alpha-amylase with *Athelia rolfsii* glucoamylase linker and SBD (SEQ ID NO:102 in US 60/638,614). Other non-limiting examples of hybrid alpha-amylases are any of those listed in Tables 3, 4, 5, and 6 in Example 4 in US application No. 11/316,535 and WO 2006/069290.

Other non-limiting examples of contemplated hybrid alpha-amylases include those disclosed in U.S. Patent Publication no. 2005/0054071, including those disclosed in Table 3 on page 15, such as *Aspergillus niger* alpha-amylase with *Aspergillus kawachii* linker and starch binding domain.

In some aspects, alpha-amylases include those which exhibit a high sequence identity to any of above mention alpha-amylases, *e.g.*, at least 70%, at least 75%, at least 80%, at least 85%, at least 90%, at least 95%, at least 96%, at least 97%, at least 98%, at least 99% or even 100% sequence identity to the mature enzyme sequences.

An acid alpha-amylases may according to the present disclosure be added in an amount of 0.001 to 10 AFAU/g DS, or in embodiments from 0.01 to 5 AFAU/g DS, or 0.3 to 2 AFAU/g DS or 0.001 to 1 FAU-F/g DS, or in embodiments 0.01 to 1 FAU-F/g DS.

Commercial alpha-amylase enzymes are suitable for use in accordance with the present disclosure. Non-limiting examples of commercial compositions comprising alpha-amylase include MYCOLASE™ from DSM (Gist Brocades), BAN™, TERMAMYL™ SC, FUNGAMYL™, LIQUOZYME™ X, LIQUOZYME™ SC and SAN™ SUPER, SAN™ EXTRA L (Novozymes A/S) and CLARASE™ L-40,000, DEX-LO™, SPEZYME™ FRED, SPEZYME™ AA, and SPEZYME™ DELTA AA (Genencor Int.), FUELZYME™ (from Verenium Corp, USA), and the acid fungal alpha-amylase sold under the trade name SP288 (available from Novozymes A/S, Denmark).

In some aspects, amylases for use in accordance with the present disclosure include the mature polypeptide of SEQ ID NO: 4 and fragments thereof having amylase activity. In embodiments, amylase include those which exhibit a high sequence identity to SEQ ID NO: 4, *e.g.*, at least 70%, at least 75%, at least 80%, at least 85%, at least 90%, at least 95%, at least 96%, at least 97%, at least 98%, at least 99% or even 100% sequence identity to the mature enzyme sequence.

In some aspects, amylase activity is determined using known methods in the art, including but not limited to those described in U.S. published patent application no. 2011/0158976.

As used herein the term "carbohydrate-source generating enzyme" includes glucoamylase (being glucose generators), beta-amylase and maltogenic amylase (being maltose generators) and also pullulanase and alpha-glucosidase which are all suitable for use in accordance with the present disclosure. A carbohydrate-source generating enzyme is capable of producing a carbohydrate that can be used as an energy-source by the fermenting organism(s) in question, for instance, when used in a method of the present disclosure for producing a fermentation product, for example ethanol. The generated carbohydrate may be converted directly or indirectly to the desired fermentation product, for example ethanol. According to the present disclosure a mixture of carbohydrate-source generating enzymes may be used. Especially contemplated blends are mixtures comprising at least a glucoamylase and an alpha-amylase, for example an acid amylase, or an acid fungal alpha-amylase.

The ratio between glucoamylase activity (AGU) and acid fungal alpha-amylase activity (FAU-F) (*e.g.*, AGU per FAU-F) may in some aspects of the present disclosure be in an amount of 0.1 and 100 AGU/FAU-F, or in some aspects, 2 and 50 AGU/FAU-F, such as in an amount of 10-40 AGU/FAU-F, especially when doing one-step fermentation (Raw Starch Hydrolysis - RSH), *e.g.*, when saccharification in step (a) and fermentation in step (b) are carried out simultaneously (*e.g.* without a liquefaction step).

In a conventional starch-to-ethanol process (*e.g.*, including a liquefaction step (a)) the ratio may be as defined in EP 140,410-B1, especially when saccharification in step ii) and fermentation in step iii) are carried out simultaneously.

Glucoamylase enzymes are suitable for use in accordance with the present disclosure. Non-limiting examples include a glucoamylase derived from any suitable source, *e.g.*, derived from a microorganism or a plant. Non-limiting examples of glucoamylases are of fungal or bacterial origin, selected from the group consisting of *Aspergillus* glucoamylases, in particular *Aspergillus niger* G1 or G2 glucoamylase (Boel et a/., 1984, EMBO J. 3(5): 1097-1102), or variants thereof, such as those disclosed in WO 92/00381, WO 00/04136 and WO 01/04273 (from Novozymes, Denmark); the *A. awamori* glucoamylase disclosed in WO 84/02921, *Aspergillus oryzae* glucoamylase (Agric. Biol. Chem. 55(4): 941-949 (1991)), or variants or fragments thereof. Other *Aspergillus* glucoamylase variants include variants with enhanced thermal stability: G137A and G139A (Chen et a/., 1996, Prot. Eng. 9: 499-505); D257E and D293E/Q (Chen et al., 1995, Prot. Eng. 8: 575-582); N182 (Chen et al., 1994, Biochem. J. 301: 275-281); disulphide bonds, A246C (Fierobe et al., 1996, Biochemistry 35: 8698-8704; and introduction of Pro residues in position A435 and S436 (Li et al., 1997, Protein Eng. 10: 1199-1204.

Other non-limiting examples of glucoamylases include *Athelia rolfsii* (previously denoted *Corticium rolfsii*) glucoamylase (see US patent no. 4,727,026 and (Nagasaka,Y. et al. (1998) "Purification and properties of the raw-starch-degrading glucoamylases from Corticium rolfsii, Appl. Microbiol. Biotechnol. 50:323-330), *Talaromyces* glucoamylases, in particular derived from *Talaromyces emersonii* (WO 99/28448), *Talaromyces leycettanus* (US patent no. Re. 32,153), *Talaromyces duponti, Talaromyces thermophilus* (US patent no. 4,587,215).

Non-limiting examples of bacterial glucoamylases include glucoamylases from the genus *Clostridium,* in particular *C. thermoamylolyticum* (EP 135,138), and *C. thermohydrosulfuricum* (WO 86/01831) and *Trametes cingulata, Pachykytospora papyracea;* and *Leucopaxillus giganteus* all disclosed in WO 2006/069289; or *Peniophora rufomarginata* disclosed in PCT/US2007/066618; or a mixture thereof. Also hybrid glucoamylase may be suitable for use in accordance with the present disclosure. Non-limiting examples include the hybrid glucoamylases disclosed in WO 2005/045018 and the hybrid glucoamylase disclosed in Table 1 and 4 of Example 1.

In some aspects, glucoamylases suitable for use in accordance with the present disclosure include those which exhibit a high sequence identity to any of above mention glucoamylases, *e.g.*, at least 70%, at least 75%, at least 80%, at least 85%, at least 90%, at least 95%, at least 96%, at least 97%, at least 98%, at least 99% or even 100% sequence identity to the mature enzymes sequences mentioned above.

In some aspects, glucoamylase for use in accordance with the present disclosure include the mature polypeptides of SEQ ID NOS: 6 and 7, and fragments thereof having glucoamylase activity. In embodiments, glucoamylase include those which exhibit a high sequence identity to SEQ ID NOS: 6 and 7, *e.g.*, at least 70%, at least 75%, at least 80%, at least 85%, at least 90%, at least 95%, at least 96%, at least 97%, at least 98%, at least 99% or even 100% sequence identity to the mature enzyme sequence.

Non-limiting examples of commercially available compositions comprising glucoamylase include AMG 200L; AMG 300 L; SAN™ SUPER, SAN™ EXTRA L, SPIRIZYME™ PLUS, SPIRIZYME™ FUEL, SPIRIZYME™ B4U, SPIRIZYME ULTRA™, and AMG™ E (from Novozymes A/S); OPTIDEX™ 300, GC480™ and GC147™ (from Genencor Int., USA); AMIGASE™ and AMIGASE™ PLUS (from DSM); G-ZYME™ G900, G-ZYME™ and G990 ZR (from Genencor Int.). In some aspects, glucoamylases include those which exhibit a high sequence identity to any of above mention glucoamylases, *e.g.*, at least 70%, at least 75%, at least 80%, at least 85%, at least 90%, at least 95%, at least 96%, at least 97%, at least 98%, at least 99% or even 100% sequence identity to the mature enzyme sequences.

In one aspect, an effective amount of glucoamylase enzyme protein to cellulosic material is about 0.05 to about 50 mg, preferably at about 0.05 to about 40 mg, more preferably at about 0.05 to about 25 mg, more preferably at about 0.75 to about 20 mg, more preferably at about 0.75 to about 15 mg, even more preferably at about 0.05 to about 10 mg, and most preferably at about 2.5 to about 10 mg per g of cellulosic material.

Beta-amylase enzymes are suitable for use in accordance with the present disclosure. A beta-amylase (E.C 3.2.1.2) is the name traditionally given to exo-acting maltogenic amylases, which catalyze the hydrolysis of 1,4-alpha-glucosidic linkages in amylose, amylopectin and related glucose polymers. Maltose units are successively removed from the non-reducing chain ends in a step-wise manner until the molecule is degraded or, in the case of amylopectin, until a branch point is reached. The maltose released has the beta anomeric configuration, hence the name beta-amylase.

Beta-amylases have been isolated from various plants and microorganisms (Fogarty and Kelly, 1979, Progress in Industrial Microbiology 15: 112-115). These beta-amylases are characterized by having optimum temperatures in the range from 40°C to 65°C and optimum pH in the range from 4.5 to 7. Non-limiting examples of beta-amylase suitable for use in accordance with the present disclosure include the commercially available beta-amylase from barley is NOVOZYM™ WBA from Novozymes A/S, Denmark and SPEZYME™ BBA 1500 from Genencor Int., USA.

Maltogenic amylase is an enzyme suitable for use in accordance with the present disclosure. A "maltogenic alpha-amylase" (glucan 1,4-alpha-maltohydrolase, E.C. 3.2.1.133) is able to hydrolyze amylose and amylopectin to maltose in the alpha-configuration. Non-limiting examples of maltogenic amylase includes those from *Bacillus stearothermophilus* strain NCIB 11837 which is commercially available from Novozymes A/S. Additional examples of maltogenic alpha-amylases include those described in US Patent nos. 4,598,048, 4,604,355 and 6,162,628.

In some aspects, maltogenic amylase may be added in an amount of 0.05 to about 50 mg enzyme protein per g of cellulosic material. In some aspects, maltogenic amylase may be added in an amount of 0.5 to about 40 mg, at about 0.5 to about 25 mg, at about 0.75 to about 20 mg, at about 0.75 to about 15 mg, at about 0.5 to about 10 mg, and at about 2.5 to about 10 mg per g of cellulosic material.

In some aspects, amylase such as those described in U.S. Patent Publication 2011/0076741 are suitable for use in accordance with the present disclosure.

Mannanase is used in the the hydrolysis step of the present disclosure as described herein. Mannanases are hemicellulases classified as EC 3.2.1.78, and called endo-1,4-beta-mannosidase. Mannanase includes beta-mannanase, endo-1,4-mannanase, and galactomannanase. Mannanase is preferably capable of catalyzing the hydrolysis of 1,4-beta-D-mannosidic linkages in mannans, including glucomannans, galactomannans and galactoglucomannans. Mannans are polysaccharides primarily or entirely composed of D-mannose units. The mannanase may be of any origin such as a bacterium or a fungal organism.

In one aspect, the mannanase is derived from a strain of the filamentous fungus genus Aspergillus, preferably Aspergillus niger or Aspergillus aculeatus (WO 94/25576). WO 93/24622 discloses a mannanase isolated from Trichoderma reseei useful for bleaching lignocellulosic pulps.

Mannanases have been identified in several *Bacillus* organisms. For example, Talbot et al., 1990, Appl. Environ.

Microbiol. 56(11): 3505-3510 describes a beta-mannanase derived from *Bacillus stearothermophilus.* Mendoza et al., 1994, World J. Microbiol. Biotech. 10(5); 551-555 describes a beta-mannanase derived from *Bacillus subtilis.* JP-A-03047076 discloses a beta-mannanase derived from *Bacillus sp.* JP-A-63056289 describes the production of an alkaline, thermostable beta-mannanase. JP-A-63036775 relates to the *Bacillus* microorganism FERM P-8856 which produces beta-mannanase and beta-mannosidase. JP-A-08051975 discloses alkaline beta-mannanases from alkalophilic *Bacillus sp.* AM-001. A purified mannanase from *Bacillus amyloliquefaciens* is disclosed in WO 97/11164. WO 91/18974 describes a hemicellulase such as a glucanase, xylanase or mannanase active.

Examples of commercially available mannanases include GAMANASE™ available from Novozymes A/S Denmark.

Non-limiting examples of mannanase suitable for use in accordance with the present disclosure also include Mannaway™ (product of Novozymes) and MannaStar (product of Genencor).

Non-limiting examples of mannanse also include those described in WO 99/064573, U.S. Patent Nos. 7,183,093, 6,060,299, 6,376,445.

In some aspects, mannanase include those which exhibit a high sequence identity to any of above mention mannanases, *e.g.*, at least 70%, at least 75%, at least 80%, at least 85%, at least 90%, at least 95%, at least 96%, at least 97%, at least 98%, at least 99% or even 100% sequence identity to the mature enzyme sequences.

In some aspects, mannanase for use in accordance with the present disclosure include the mature polypeptide of SEQ ID NO: 5, and fragments thereof having mannanase activity. In embodiments, mannanase include those which exhibit a high sequence identity to SEQ ID NO: 5, *e.g.*, at least 70%, at least 75%, at least 80%, at least 85%, at least 90%, at least 95%, at least 96%, at least 97%, at least 98%, at least 99% or even 100% sequence identity to the mature enzyme sequence.

Mannanase activity can be determined using the method set out in U.S. Patent No. 5,795,764. For example, 0.4% suspensions of AZCL-mannan (Megazyme, Australia) can be mixed 1:1 with 0.1M buffer (sodium citrate/trisodium phosphate), enzyme is added, and the incubations can be carried out at 30°C. for 15 min, followed by inactivation of the enzyme at 95°C. for 20 min. After centrifugation the release of blue color into the supernatant was measured in microtiter plates at 620 nm. For determination of pH optima, the enzymatic reaction is carried out in citrate/phosphate buffers from pH 2.5 to 9. For determination of temperature optimum, the enzyme is incubated at pH 5.0 with substrate at temperatures from 30 to 80°C. $K_m$ and specific activity is measured by carrying out incubations in 0.1 M citrate buffer pH 5.0 at substrate concentrations ranging from 0.025 to 1% carob galactomannan (Megazyme, Austrailia). The results may be plotted in a "Hanes plot" where the slope is $1/V_{max}$ and the intercept is $K_m/V_{max}$.

In one aspect, an effective amount of mannanse enzyme protein to cellulosic material is about 0.05 to about 50 mg, preferably at about 0.5 to about 40 mg, more preferably at about 0.5 to about 25 mg, more preferably at about 0.75 to about 20 mg, more preferably at about 0.75 to about 15 mg, even more preferably at about 0.5 to about 10 mg, and most preferably at about 2.5 to about 10 mg per g of cellulosic material.

Enzyme compositions suitable for use in accordance with the present disclosure comprises one or more (several) enzymes selected from the group consisting of an amylase, a cellulase, a GH61 polypeptide having cellulolytic enhancing activity, a hemicellulase, an expansin, an esterase, a laccase, a ligninolytic enzyme, a mannanase, a pectinase, a peroxidase, a protease, a swollenin, and mixtures thereof.

In some aspects of the present disclosure the step of hydrolyzing is performed with a total solids (TS) sufficient for the reaction. Non-limiting amounts of total solids for use in accordance with the present disclosure include amounts of about 1% to about 50%, *e.g.*, about 2% to about 40%, about 2% to about 35%, about 3% to about 30%, about 3% to about 25%, about 4% to about 20%, about 5% to about 10%, about 1%, about 2%, about 3%, about 4%, about 5%, about 6%, about 7%, about 8%, about 9%, or about 10%. TS can refer to the total biomass and/or filtrate used in the reaction.

In some aspects of the present disclosure the step of hydrolyzing is performed at a pH suitable for the hydrolyzing step. Non-limiting examples of suitable pH include of about 2 to about 9, *e.g.*, about 3 to about 7.5, about 3.5 to about 7, about 4 to about 6.5, about 4.5 to about 6.5, about 4.5 to about 6.0, about 5 and about 6.0, about 5 to about 5.5, 5, about 5, 6, about 6, 7, about 7.

In some aspects of the present disclosure the step of hydrolyzing is performed at a suitable temperature for the hydrolyzing step. Non-limiting suitable temperatures include temperatures in the amount of about 20°C to about 70°C, *e.g.*, about 25°C to about 65°, about 30°C to about 65°C, about 35°C to about 65°C, about 40°C to about 60°C, about 45°C to about 55°C, or about 45°C to about 50°C.

In some aspects, of the present disclosure the step of hydrolyzing is performed for a sufficient period of time. Non-limiting periods of time include a period of time of 5 minutes to 35 hours, *e.g.*, 10 minutes to 15 hours, 10 hours to 15 hours, 10 hours to 20 hours, 10 hours to 20 hours, 20 hours to 24 hours, 24 hours to 30 hours, 1 hour to 72 hours, 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 24 hours, 48 hours, 72 hours, or 96 hours.

In some aspects, the hydrolyzing is performed using a combination of TS, pH, termperature and duration as set out herein. One non-limiting examples is where the hydrolyzing step is performed with a total solids (TS) of 5.3%, a pH of 5, at a temperature of 50°C for 72 hours.

In embodiments, the present disclosure relates to a method for hydrolyzing a pretreated cellulosic material comprising saccharifying a cellulosic material with an enzyme composition, wherein the cellulosic material was pretreated by contacting the cellulosic material with one or more cutinase, peptidase and pectate lyase enzymes to form pretreated cellulosic material. In embodiments, the enzyme composition comprises or consists of one or more (several) enzymes selected from the group consisting of a cellulase, a GH61 polypeptide having cellulolytic enhancing activity, a hemicellulase, an expansin, an esterase, a laccase, a ligninolytic enzyme, a pectinase, a peroxidase, a protease, a swollenin, an amylase and a mannanase. In embodiments, the cellulase is one or more (several) enzymes selected from the group consisting of an endoglucanase, a cellobiohydrolase, and a beta-glucosidase. In embodiments, the hemicellulase is one or more (several) enzymes selected from the group consisting of a xylanase, an acetyxylan esterase, a feruloyl esterase, an arabinofuranosidase, a xylosidase, and a glucuronidase. In embodiments, the method of the present disclosure further includes recovering a saccharified material from the saccharification. The saccharified material may be a sugar. Non-limiting examples of sugar include glucose, xylose, mannose, galactose, and arabinose.

## Fermentation

The fermentable sugars obtained from the hydrolyzed cellulosic material can be fermented by one or more (several) fermenting microorganisms capable of fermenting the sugars directly or indirectly into a desired fermentation product. "Fermentation" or "fermentation process" refers to any fermentation process or any process comprising a fermentation step. Fermentation processes also include fermentation processes used in the consumable alcohol industry (*e.g.*, beer and wine), dairy industry (*e.g.*, fermented dairy products), leather industry, and tobacco industry. The fermentation conditions depend on the desired fermentation product and fermenting organism and can easily be determined by one skilled in the art.

In the fermentation step, sugars, released from cellulosic material as a result of the pretreatment and enzymatic hydrolysis steps, are fermented to a product, *e.g.*, ethanol, by a fermenting organism, such as yeast. Hydrolysis (saccharification) and fermentation can be separate or simultaneous, as described herein.

The term "fermentation medium" is understood herein to refer to a medium before the fermenting microorganism(s) is(are) added, such as, a medium resulting from a saccharification process, as well as a medium used in a simultaneous saccharification and fermentation process (SSF).

"Fermenting microorganism" refers to any microorganism, including bacterial and fungal organisms, suitable for use in a desired fermentation process to produce a fermentation product. The fermenting organism can be hexose and/or pentose fermenting organisms, or a combination thereof. Both hexose and pentose fermenting organisms are well known in the art. Suitable fermenting microorganisms are able to ferment, *i.e.*, convert, sugars, such as glucose, xylose, xylulose, arabinose, maltose, mannose, galactose, and/or oligosaccharides, directly or indirectly into the desired fermentation product. Examples of bacterial and fungal fermenting organisms producing ethanol are described by Lin et al., 2006, Appl. Microbiol. Biotechnol. 69: 627-642.

Examples of fermenting microorganisms that can ferment hexose sugars include bacterial and fungal organisms, such as yeast. Preferred yeast includes strains of *Candida, Kluyveromyces,* and *Saccharomyces, e.g., Candida sonorensis, Kluyveromyces marxianus,* and *Saccharomyces cerevisiae.*

Examples of fermenting organisms that can ferment pentose sugars in their native state include bacterial and fungal organisms, such as some yeast. Preferred xylose fermenting yeast include strains of *Candida,* preferably *C. sheatae* or *C. sonorensis;* and strains of *Pichia,* preferably *P. stipitis,* such as *P. stipitis* CBS 5773. Preferred pentose fermenting yeast include strains of *Pachysolen,* preferably *P. tannophilus.* Organisms not capable of fermenting pentose sugars, such as xylose and arabinose, may be genetically modified to do so by methods known in the art.

Examples of bacteria that can efficiently ferment hexose and pentose to ethanol include, for example, *Bacillus coagulans, Clostridium acetobutylicum, Clostridium thermocellum, Clostridium phytofermentans, Geobacillus* sp., *Thermoanaerobacter saccharolyticum,* and *Zymomonas mobilis* (Philippidis, 1996, *supra*).

Other fermenting organisms include strains of *Bacillus,* such as *Bacillus coagulans; Candida,* such as *C. sonorensis, C. methanosorbosa, C. diddensiae, C. parapsilosis, C. naedodendra, C. blankii, C. entomophilia, C. brassicae, C. pseudotropicalis, C. boidinii, C. utilis,* and *C. scehatae; Clostridium,* such as *C. acetobutylicum, C. thermocellum,* and *C. phytofermentans; E. coli,* especially *E. coli* strains that have been genetically modified to improve the yield of ethanol; *Geobacillus* sp.; *Hansenula,* such as *Hansenula anomala*; *Klebsiella,* such as *K. oxytoca*; *Kluyveromyces,* such as *K. marxianus, K. lactis, K. thermotolerans,* and *K. fragilis*; *Schizosaccharomyces,* such as *S. pombe*; *Thermoanaerobacter,* such as *Thermoanaerobacter saccharolyticum*; and *Zymomonas,* such as *Zymomonas mobilis.*

In a preferred aspect, the yeast is a *Bretannomyces.* In a more preferred aspect, the yeast is *Bretannomyces clausenii.* In another preferred aspect, the yeast is a *Candida.* In another more preferred aspect, the yeast is *Candida sonorensis.* In another more preferred aspect, the yeast is *Candida boidinii.* In another more preferred aspect, the yeast is *Candida blankii.* In another more preferred aspect, the yeast is *Candida brassicae.* In another more preferred aspect, the yeast is *Candida diddensii.* In another more preferred aspect, the yeast is *Candida entomophiliia.* In another more preferred aspect, the yeast is *Candida pseudotropicalis.* In another more preferred aspect, the yeast is *Candida scehatae.* In another more preferred aspect, the yeast is *Candida utilis.* In another preferred aspect, the yeast is a *Clavispora.* In another more preferred aspect, the yeast is *Clavispora lusitaniae.* In another more preferred aspect, the yeast is *Clavispora opuntiae.* In another preferred aspect, the yeast is a *Kluyveromyces.* In another more preferred aspect, the yeast is *Kluyveromyces fragilis.* In another more preferred aspect, the yeast is *Kluyveromyces marxianus.* In another more preferred aspect, the yeast is *Kluyveromyces thermotolerans.* In another preferred aspect, the yeast is a *Pachysolen.* In another more preferred aspect, the yeast is *Pachysolen tannophilus.* In another preferred aspect, the yeast is a *Pichia.* In another more preferred aspect, the yeast is a *Pichia stipitis.* In another preferred aspect, the yeast is a *Saccharomyces* spp. In a more preferred aspect, the yeast is *Saccharomyces cerevisiae.* In another more preferred aspect, the yeast is *Saccharomyces distaticus.* In another more preferred aspect, the yeast is *Saccharomyces uvarum.*

In a preferred aspect, the bacterium is a *Bacillus.* In a more preferred aspect, the bacterium is *Bacillus coagulans.* In another preferred aspect, the bacterium is a *Clostridium.* In another more preferred aspect, the bacterium is *Clostridium acetobutylicum.* In another more preferred aspect, the bacterium is *Clostridium phytofermentans.* In another more preferred aspect, the bacterium is *Clostridium thermocellum.* In another more preferred aspect, the bacterium is *Geobacilus* sp. In another more preferred aspect, the bacterium is a *Thermoanaerobacter.* In another more preferred aspect, the bacterium is *Thermoanaerobacter saccharolyticum.* In another preferred aspect, the bacterium is a *Zymomonas.* In another more preferred aspect, the bacterium is *Zymomonas mobilis.*

Commercially available yeast suitable for ethanol production include, *e.g.*, BIOFERM™ AFT and XR (NABC - North American Bioproducts Corporation, GA, USA), ETHANOL RED™ yeast (Fermentis/Lesaffre, USA), FALI™ (Fleischmann's Yeast, USA), FERMIOL™ (DSM Specialties), GERT STRAND™ (Gert Strand AB, Sweden), and SUPERSTART™ and THERMOSACC™ fresh yeast (Ethanol Technology, WI, USA).

In a preferred aspect, the fermenting microorganism has been genetically modified to provide the ability to ferment pentose sugars, such as xylose utilizing, arabinose utilizing, and xylose and arabinose co-utilizing microorganisms.

The cloning of heterologous genes into various fermenting microorganisms has led to the construction of organisms capable of converting hexoses and pentoses to ethanol (co-fermentation) (Chen and Ho, 1993, Cloning and improving the expression of Pichia stipitis xylose reductase gene in Saccharomyces cerevisiae, Appl. Biochem. Biotechnol. 39-40: 135-147; Ho et al., 1998, Genetically engineered Saccharomyces yeast capable of effectively cofermenting glucose and xylose, Appl. Environ. Microbiol. 64: 1852-1859; Kotter and Ciriacy, 1993, Xylose fermentation by Saccharomyces cerevisiae, Appl. Microbiol. Biotechnol. 38: 776-783; Walfridsson et al., 1995, Xylose-metabolizing Saccharomyces cerevisiae strains overexpressing the TKL1 and TAL1 genes encoding the pentose phosphate pathway enzymes transketolase and transaldolase, Appl. Environ. Microbiol. 61: 4184-4190; Kuyper et al., 2004, Minimal metabolic engineering of Saccharomyces cerevisiae for efficient anaerobic xylose fermentation: a proof of principle, FEMS Yeast Research 4: 655-664; Beall et al., 1991, Parametric studies of ethanol production from xylose and other sugars by recombinant Escherichia coli, Biotech. Bioeng. 38: 296-303; Ingram et al., 1998, Metabolic engineering of bacteria for ethanol production, Biotechnol. Bioeng. 58: 204-214; Zhang et al., 1995, Metabolic engineering of a pentose metabolism pathway in ethanologenic Zymomonas mobilis, Science 267: 240-243; Deanda et al., 1996, Development of an arabinose-fermenting Zymomonas mobilis strain by metabolic pathway engineering, Appl. Environ. Microbiol. 62: 4465-4470; WO 2003/062430, xylose isomerase).

In a preferred aspect, the genetically modified fermenting microorganism is *Candida sonorensis.* In another preferred aspect, the genetically modified fermenting microorganism is *Escherichia coli.* In another preferred aspect, the genetically modified fermenting microorganism is *Klebsiella oxytoca.* In another preferred aspect, the genetically modified fermenting microorganism is *Kluyveromyces marxianus.* In another preferred aspect, the genetically modified fermenting microorganism is *Saccharomyces cerevisiae.* In another preferred aspect, the genetically modified fermenting microorganism is *Zymomonas mobilis.*

It is well known in the art that the organisms described above can also be used to produce other substances, as described herein.

The fermenting microorganism is typically added to the degraded cellulosic material or hydrolysate and the fermentation is performed for about 8 to about 96 hours, *e.g.*, about 24 to about 60 hours. The temperature is typically between about 26°C to about 60°C, *e.g.*, about 32°C or 50°C, and about pH 3 to about pH 8, *e.g.*, pH 4-5, 6, or 7.

In one aspect, the yeast and/or another microorganism are applied to the degraded cellulosic material and the fermentation is performed for about 12 to about 96 hours, such as typically 24-60 hours. In another aspect, the temperature is preferably between about 20°C to about 60°C, *e.g.*, about 25°C to about 50°C, about 32°C to about 50°C, or about 32°C to about 50°C, and the pH is generally from about pH 3 to about pH 7, *e.g.*, about pH 4 to about pH 7. However, some fermenting organisms, *e.g.*, bacteria, have higher fermentation temperature optima. Yeast or another microorganism is preferably applied in amounts of approximately $10^5$ to $10^{12}$, preferably from approximately $10^7$ to $10^{10}$, especially

approximately $2 \times 10^8$ viable cell count per ml of fermentation broth. Further guidance in respect of using yeast for fermentation can be found in, *e.g.*, "The Alcohol Textbook" (Editors K. Jacques, T.P. Lyons and D.R. Kelsall, Nottingham University Press, United Kingdom 1999).

For ethanol production, following the fermentation the fermented slurry is distilled to extract the ethanol. The ethanol obtained according to the processes of the invention can be used as, *e.g.*, fuel ethanol, drinking ethanol, *i.e.*, potable neutral spirits, or industrial ethanol.

A fermentation stimulator can be used in combination with any of the processes described herein to further improve the fermentation process, and in particular, the performance of the fermenting microorganism, such as, rate enhancement and ethanol yield. A "fermentation stimulator" refers to stimulators for growth of the fermenting microorganisms, in particular, yeast. Preferred fermentation stimulators for growth include vitamins and minerals. Examples of vitamins include multivitamins, biotin, pantothenate, nicotinic acid, meso-inositol, thiamine, pyridoxine, para-aminobenzoic acid, folic acid, riboflavin, and Vitamins A, B, C, D, and E. See, for example, Alfenore et al., Improving ethanol production and viability of Saccharomyces cerevisiae by a vitamin feeding strategy during fed-batch process, Springer-Verlag (2002). Examples of minerals include minerals and mineral salts that can supply nutrients comprising P, K, Mg, S, Ca, Fe, Zn, Mn, and Cu.

**Fermentation Products**

The fermentation product can be any substance derived from the fermentation.

The fermentation product can be, without limitation, an alcohol (*e.g.*, arabinitol, n-butanol, isobutanol, ethanol, glycerol, methanol, ethylene glycol, 1,3-propanediol [propylene glycol], butanediol, glycerin, sorbitol, and xylitol); an alkane (*e.g.*, pentane, hexane, heptane, octane, nonane, decane, undecane, and dodecane), a cycloalkane (*e.g.*, cyclopentane, cyclohexane, cycloheptane, and cyclooctane), an alkene (*e.g.* pentene, hexene, heptene, and octene); an amino acid (*e.g.*, aspartic acid, glutamic acid, glycine, lysine, serine, and threonine); a gas (*e.g.*, methane, hydrogen ($H_2$), carbon dioxide ($CO_2$), and carbon monoxide (CO)); isoprene; a ketone (*e.g.*, acetone); an organic acid (*e.g.*, acetic acid, acetonic acid, adipic acid, ascorbic acid, citric acid, 2,5-diketo-D-gluconic acid, formic acid, fumaric acid, glucaric acid, gluconic acid, glucuronic acid, glutaric acid, 3-hydroxypropionic acid, itaconic acid, lactic acid, malic acid, malonic acid, oxalic acid, oxaloacetic acid, propionic acid, succinic acid, and xylonic acid); and polyketide. The fermentation product can also be protein as a high value product.

In a preferred aspect, the fermentation product is an alcohol. It will be understood that the term "alcohol" encompasses a substance that contains one or more hydroxyl moieties. In a more preferred aspect, the alcohol is n-butanol. In another more preferred aspect, the alcohol is isobutanol. In another more preferred aspect, the alcohol is ethanol. In another more preferred aspect, the alcohol is methanol. In another more preferred aspect, the alcohol is arabinitol. In another more preferred aspect, the alcohol is butanediol. In another more preferred aspect, the alcohol is ethylene glycol. In another more preferred aspect, the alcohol is glycerin. In another more preferred aspect, the alcohol is glycerol. In another more preferred aspect, the alcohol is 1,3-propanediol. In another more preferred aspect, the alcohol is sorbitol. In another more preferred aspect, the alcohol is xylitol. See, for example, Gong, C. S., Cao, N. J., Du, J., and Tsao, G. T., 1999, Ethanol production from renewable resources, in Advances in Biochemical Engineering/Biotechnology, Scheper, T., ed., Springer-Verlag Berlin Heidelberg, Germany, 65: 207-241; Silveira and Jonas, 2002, The biotechnological production of sorbitol, Appl. Microbiol. Biotechnol. 59: 400-408; Nigam, P., and Singh, D., 1995, Processes for fermentative production of xylitol - a sugar substitute, Process Biochemistry 30(2): 117-124; Ezeji et al., 2003, Production of acetone, butanol and ethanol by Clostridium beijerinckii BA101 and in situ recovery by gas stripping, World Journal of Microbiology and Biotechnology 19(6): 595-603.

In another preferred aspect, the fermentation product is an alkane. The alkane can be an unbranched or a branched alkane. In another more preferred aspect, the alkane is pentane. In another more preferred aspect, the alkane is hexane. In another more preferred aspect, the alkane is heptane. In another more preferred aspect, the alkane is octane. In another more preferred aspect, the alkane is nonane. In another more preferred aspect, the alkane is decane. In another more preferred aspect, the alkane is undecane. In another more preferred aspect, the alkane is dodecane.

In another preferred aspect, the fermentation product is a cycloalkane. In another more preferred aspect, the cycloalkane is cyclopentane. In another more preferred aspect, the cycloalkane is cyclohexane. In another more preferred aspect, the cycloalkane is cycloheptane. In another more preferred aspect, the cycloalkane is cyclooctane.

In another preferred aspect, the fermentation product is an alkene. The alkene can be an unbranched or a branched alkene. In another more preferred aspect, the alkene is pentene. In another more preferred aspect, the alkene is hexene. In another more preferred aspect, the alkene is heptene. In another more preferred aspect, the alkene is octene.

In another preferred aspect, the fermentation product is an amino acid. In another more preferred aspect, the organic acid is aspartic acid. In another more preferred aspect, the amino acid is glutamic acid. In another more preferred aspect, the amino acid is glycine. In another more preferred aspect, the amino acid is lysine. In another more preferred aspect, the amino acid is serine. In another more preferred aspect, the amino acid is threonine. See, for example, Richard and Margaritis, 2004, Empirical modeling of batch fermentation kinetics for poly(glutamic acid) production and other microbial biopolymers, Biotechnology and Bioengineering 87(4): 501-515.

In another preferred aspect, the fermentation product is a gas. In another more preferred aspect, the gas is methane. In another more preferred aspect, the gas is $H_2$. In another more preferred aspect, the gas is $CO_2$. In another more preferred aspect, the gas is CO. See, for example, Kataoka et al., 1997, Studies on hydrogen production by continuous culture system of hydrogen-producing anaerobic bacteria, Water Science and Technology 36(6-7): 41-47; and Gunaseelan, 1997, Biomass and Bioenergy, 3(1-2): 83-114, Anaerobic digestion of biomass for methane production: A review.

In another preferred aspect, the fermentation product is isoprene.

In another preferred aspect, the fermentation product is a ketone. It will be understood that the term "ketone" encompasses a substance that contains one or more ketone moieties. In another more preferred aspect, the ketone is acetone. See, for example, Qureshi and Blaschek, 2003, *supra.*

In another preferred aspect, the fermentation product is an organic acid. In another more preferred aspect, the organic acid is acetic acid. In another more preferred aspect, the organic acid is acetonic acid. In another more preferred aspect, the organic acid is adipic acid. In another more preferred aspect, the organic acid is ascorbic acid. In another more preferred aspect, the organic acid is citric acid. In another more preferred aspect, the organic acid is 2,5-diketo-D-gluconic acid. In another more preferred aspect, the organic acid is formic acid. In another more preferred aspect, the organic acid is fumaric acid. In another more preferred aspect, the organic acid is glucaric acid. In another more preferred aspect, the organic acid is gluconic acid. In another more preferred aspect, the organic acid is glucuronic acid. In another more preferred aspect, the organic acid is glutaric acid. In another preferred aspect, the organic acid is 3-hydroxypropionic acid. In another more preferred aspect, the organic acid is itaconic acid. In another more preferred aspect, the organic acid is lactic acid. In another more preferred aspect, the organic acid is malic acid. In another more preferred aspect, the organic acid is malonic acid. In another more preferred aspect, the organic acid is oxalic acid. In another more preferred aspect, the organic acid is propionic acid. In another more preferred aspect, the organic acid is succinic acid. In another more preferred aspect, the organic acid is xylonic acid. See, for example, Chen, R., and Lee, Y. Y., 1997, Membrane-mediated extractive fermentation for lactic acid production from cellulosic biomass, Appl. Biochem. Biotechnol. 63-65: 435-448.

In another preferred aspect, the fermentation product is polyketide.

The fermentation product(s) can be optionally recovered from the fermentation medium using any method known in the art including, but not limited to, chromatography, electrophoretic procedures, differential solubility, distillation, or extraction. For example, alcohol is separated from the fermented cellulosic material and purified by conventional methods of distillation. Ethanol with a purity of up to about 96 vol. % can be obtained, which can be used as, for example, fuel ethanol, drinking ethanol, *i.e.,* potable neutral spirits, or industrial ethanol.

**Hydrolysis Enzyme Compositions**

The enzyme compositions can comprise any protein that is useful in saccharifying a cellulosic material.

In one aspect, the enzyme composition comprises or further comprises one or more (several) proteins selected from the group consisting of a cellulase, a GH61 polypeptide having cellulolytic enhancing activity, a hemicellulase, an expansin, an esterase, a laccase, a ligninolytic enzyme, a pectinase, a peroxidase, a protease, and a swollenin. In another aspect, the cellulase is preferably one or more (several) enzymes selected from the group consisting of an endoglucanase, a cellobiohydrolase, and a beta-glucosidase. In another aspect, the hemicellulase is preferably one or more (several) enzymes selected from the group consisting of an acetylmannan esterase, an acetyxylan esterase, an arabinanase, an arabinofuranosidase, a coumaric acid esterase, a feruloyl esterase, a galactosidase, a glucuronidase, a glucuronoyl esterase, a mannanase, a mannosidase, a xylanase, and a xylosidase.

In another aspect, the enzyme composition comprises one or more (several) cellulolytic enzymes. In another aspect, the enzyme composition comprises or further comprises one or more (several) hemicellulolytic enzymes. In another aspect, the enzyme composition comprises one or more (several) cellulolytic enzymes and one or more (several) hemicellulolytic enzymes. In another aspect, the enzyme composition comprises one or more (several) enzymes selected from the group of cellulolytic enzymes and hemicellulolytic enzymes. In another aspect, the enzyme composition comprises an endoglucanase. In another aspect, the enzyme composition comprises a cellobiohydrolase. In another aspect, the enzyme composition comprises a beta-glucosidase. In another aspect, the enzyme composition comprises a polypeptide having cellulolytic enhancing activity. In another aspect, the enzyme composition comprises an endoglucanase and a polypeptide having cellulolytic enhancing activity. In another aspect, the enzyme composition comprises a cellobiohydrolase and a polypeptide having cellulolytic enhancing activity. In another aspect, the enzyme composition comprises a beta-glucosidase and a polypeptide having cellulolytic enhancing activity. In another aspect, the enzyme composition comprises an endoglucanase and a cellobiohydrolase. In another aspect, the enzyme composition comprises an endoglucanase and a beta-glucosidase. In another aspect, the enzyme composition comprises a cellobiohydrolase and a beta-glucosidase. In another aspect, the enzyme composition comprises an endoglucanase, a cellobiohydrolase, and a polypeptide having cellulolytic enhancing activity. In another aspect, the enzyme composition comprises an endoglucanase, a beta-glucosidase, and a polypeptide having cellulolytic enhancing activity. In another aspect, the enzyme composition comprises a cellobiohydrolase, a beta-glucosidase, and a polypeptide having cellulolytic enhancing activity. In another aspect, the enzyme composition comprises an endoglucanase, a cellobiohydrolase, and a beta-glucosidase, and a polypeptide having cellulolytic enhancing activity.

In another aspect, the enzyme composition comprises an acetylmannan esterase. In another aspect, the enzyme composition comprises an acetyxylan esterase. In another aspect, the enzyme composition comprises an arabinanase (*e.g.*, alpha-L-arabinanase). In another aspect, the enzyme composition comprises an arabinofuranosidase (*e.g.*, alpha-L-arabinofuranosidase). In another aspect, the enzyme composition comprises a coumaric acid esterase. In another aspect, the enzyme composition comprises a feruloyl esterase. In another aspect, the enzyme composition comprises a galactosidase (*e.g.*, alpha-galactosidase and/or beta-galactosidase). In another aspect, the enzyme composition comprises a glucuronidase (*e.g.*, alpha-D-glucuronidase). In another aspect, the enzyme composition comprises a glucuronoyl esterase. In another aspect, the enzyme composition comprises a mannanase. In another aspect, the enzyme composition comprises a mannosidase (*e.g.*, beta-mannosidase). In another aspect, the enzyme composition comprises a xylanase. In a preferred aspect, the xylanase is a Family 10 xylanase. In another aspect, the enzyme composition comprises a xylosidase (*e.g.*, beta-xylosidase). In another aspect, the enzyme composition comprises an expansin. In another aspect, the enzyme composition comprises an esterase. In another aspect, the enzyme composition comprises a laccase. In another aspect, the enzyme composition comprises a ligninolytic enzyme. In a preferred aspect, the ligninolytic enzyme is a manganese peroxidase. In another preferred aspect, the ligninolytic enzyme is a lignin peroxidase. In another preferred aspect, the ligninolytic enzyme is a $H_2O_2$-producing enzyme. In another aspect, the enzyme composition comprises a pectinase. In another aspect, the enzyme composition comprises a peroxidase. In another aspect, the enzyme composition comprises a protease. In another aspect, the enzyme composition comprises a swollenin.

In another aspect, the enzyme composition comprises amylase and mannanase alone or in combination as described above. Non-limiting examples include a combination of *Rhizomucer pusillus* amylase of SEQ ID NO:4, *Talaromices emersoni* glucoamylase (SEQ ID NO:6), and *Trametes cingulata* glucoamylase (SEQ ID NO:7). Aspergillus niger mannanase such as SEQ ID NO:5) is also suitable for use in accordance with the present disclosure. In embodiments, amylase and mannanase include those which exhibit a high sequence identity to any of above mention amylases and mannanases, *e.g.*, at least 70%, at least 75%, at least 80%, at least 85%, at least 90%, at least 95%, at least 96%, at least 97%, at least 98%, at least 99% or even 100% sequence identity to the mature enzyme sequences.

In the processes of the present invention, the enzyme(s) can be added prior to or during fermentation, *e.g.*, during saccharification or during or after propagation of the fermenting microorganism(s).

One or more (several) components of the enzyme composition may be wild-type proteins, recombinant proteins, or a combination of wild-type proteins and recombinant proteins. For example, one or more (several) components may be native proteins of a cell, which is used as a host cell to express recombinantly one or more (several) other components of the enzyme composition. One or more (several) components of the enzyme composition may be produced as monocomponents, which are then combined to form the enzyme composition. The enzyme composition may be a combination of multicomponent and monocomponent protein preparations.

The enzymes used in the processes of the present invention may be in any form suitable for use, such as, for example, a crude fermentation broth with or without cells removed, a cell lysate with or without cellular debris, a semi-purified or purified enzyme preparation, or a host cell as a source of the enzymes. The enzyme composition may be a dry powder or granulate, a non-dusting granulate, a liquid, a stabilized liquid, or a stabilized protected enzyme. Liquid enzyme preparations may, for instance, be stabilized by adding stabilizers such as a sugar, a sugar alcohol or another polyol, and/or lactic acid or another organic acid according to established processes.

The enzymes can be derived or obtained from any suitable origin, including, bacterial, fungal, yeast, plant, or mammalian origin. The term "obtained" means herein that the enzyme may have been isolated from an organism that naturally produces the enzyme as a native enzyme. The term "obtained" also means herein that the enzyme may have been produced recombinantly in a host organism employing methods described herein, wherein the recombinantly produced enzyme is either native or foreign to the host organism or has a modified amino acid sequence, *e.g.*, having one or more (several) amino acids that are deleted, inserted and/or substituted, *i.e.*, a recombinantly produced enzyme that is a mutant and/or a fragment of a native amino acid sequence or an enzyme produced by nucleic acid shuffling processes known in the art. Encompassed within the meaning of a native enzyme are natural variants and within the meaning of a foreign enzyme are variants obtained recombinantly, such as by site-directed mutagenesis or shuffling.

The polypeptide having enzyme activity may be a bacterial polypeptide. For example, the polypeptide may be a gram positive bacterial polypeptide such as a *Bacillus, Streptococcus, Streptomyces, Staphylococcus, Enterococcus, Lactobacillus, Lactococcus, Clostridium, Geobacillus,* or *Oceanobacillus* polypeptide having enzyme activity, or a Gram negative bacterial polypeptide such as an *E. coli, Pseudomonas, Salmonella, Campylobacter, Helicobacter, Flavobacterium, Fusobacterium, Ilyobacter, Neisseria,* or *Ureaplasma* polypeptide having enzyme activity.

In a preferred aspect, the polypeptide is a *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* or *Bacillus thuringiensis* polypeptide having enzyme activity.

In another preferred aspect, the polypeptide is a *Streptococcus equisimilis, Streptococcus pyogenes, Streptococcus uberis,* or *Streptococcus equi* subsp. *Zooepidemicus* polypeptide having enzyme activity.

In another preferred aspect, the polypeptide is a *Streptomyces achromogenes, Streptomyces avermitilis, Streptomyces coelicolor, Streptomyces griseus,* or *Streptomyces lividans* polypeptide having enzyme activity.

The polypeptide having enzyme activity may also be a fungal polypeptide, and more preferably a yeast polypeptide such as a *Candida, Kluyveromyces, Pichia, Saccharomyces, Schizosaccharomyces,* or *Yarrowia* polypeptide having enzyme activity; or more preferably a filamentous fungal polypeptide such as an *Acremonium, Agaricus, Alternaria, Aspergillus, Aureobasidium, Botryospaeria, Ceriporiopsis, Chaetomidium, Chrysosporium, Claviceps, Cochliobolus, Coprinopsis, Coptotermes, Corynascus, Cryphonectria, Cryptococcus, Diplodia, Exidia, Filibasidium, Fusarium, Gibberella, Holomastigotoides, Humicola, Irpex, Lentinula, Leptospaeria, Magnaporthe, Melanocarpus, Meripilus, Mucor, Myceliophthora, Neocallimastix, Neurospora, Paecilomyces, Penicillium, Phanerochaete, Piromyces, Poitrasia, Pseudoplectania, Pseudotrichonympha, Rhizomucor, Schizophyllum, Scytalidium, Talaromyces, Thermoascus, Thielavia, Tolypocladium, Trichoderma, Trichophaea, Verticillium, Volvariella,* or *Xylaria* polypeptide having enzyme activity.

In a preferred aspect, the polypeptide is a *Saccharomyces carlsbergensis, Saccharomyces cerevisiae, Saccharomyces diastaticus, Saccharomyces douglasii, Saccharomyces kluyveri, Saccharomyces norbensis,* or *Saccharomyces oviformis* polypeptide having enzyme activity.

In another preferred aspect, the polypeptide is an *Acremonium cellulolyticus, Aspergillus aculeatus, Aspergillus awamori, Aspergillus fumigatus, Aspergillus foetidus, Aspergillus japonicus, Aspergillus nidulans, Aspergillus niger, Aspergillus oryzae, Chrysosporium keratinophilum, Chrysosporium lucknowense, Chrysosporium tropicum, Chrysosporium merdarium, Chrysosporium inops, Chrysosporium pannicola, Chrysosporium queenslandicum, Chrysosporium zonatum, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulfureum, Fusarium torulosum, Fusarium trichothecioides, Fusarium venenatum, Humicola grisea, Humicola insolens, Humicola lanuginosa, Irpex lacteus, Mucor miehei, Myceliophthora thermophila, Neurospora crassa, Penicillium funiculosum, Penicillium purpurogenum, Phanerochaete chrysosporium, Thielavia achromatica, Thielavia albomyces, Thielavia albopilosa, Thielavia australeinsis, Thielavia fimeti, Thielavia microspora, Thielavia ovispora, Thielavia peruviana, Thielavia spededonium, Thielavia setosa, Thielavia subthermophila, Thielavia terrestris, Trichoderma harzianum, Trichoderma koningii, Trichoderma longibrachiatum, Trichoderma reesei, Trichoderma viride,* or *Trichophaea saccata* polypeptide having enzyme activity.

Chemically modified or protein engineered mutants of the polypeptides having enzyme activity may also be used.

One or more (several) components of the enzyme composition may be a recombinant component, *i.e.,* produced by cloning of a DNA sequence encoding the single component and subsequent cell transformed with the DNA sequence and expressed in a host (see, for example, WO 91/17243 and WO 91/17244). The host is preferably a heterologous host (enzyme is foreign to host), but the host may under certain conditions also be a homologous host (enzyme is native to host). Monocomponent cellulolytic enzymes may also be prepared by purifying such a protein from a fermentation broth.

In one aspect, the one or more (*e.g.*, several) cellulolytic enzymes comprise a commercial cellulolytic enzyme preparation. Examples of commercial cellulolytic enzyme preparations suitable for use in the present invention include, for example, CELLIC™ CTec (Novozymes A/S), CELLIC™ CTec2 (Novozymes A/S), CELLIC™ CTec3 (Novozymes A/S), CELLUCLAST™ (Novozymes A/S), NOVOZYM™ 188 (Novozymes A/S), CELLUZYME™ (Novozymes A/S), CEREFLO™ (Novozymes A/S), and ULTRAFLO™ (Novozymes A/S), ACCELERASE™ (Genencor Int.), LAMINEX™ (Genencor Int.), SPEZYME™ CP (Genencor Int.), FILTRASE® NL (DSM); METHAPLUS® S/L 100 (DSM). ROHAMENT™ 7069 W (Rohm GmbH), FIBREZYME® LDI (Dyadic International, Inc.), FIBREZYME® LBR (Dyadic International, Inc.), or VISCOSTAR® 150L (Dyadic International, Inc.). The cellulase enzymes are added in amounts effective from about 0.001 to about 5.0 wt % of solids, e.g., about 0.025 to about 4.0 wt % of solids or about 0.005 to about 2.0 wt % of solids.

In the processes of the present invention, any GH61 polypeptide having cellulolytic enhancing activity can be used, such as those polypeptides described *supra.*

Examples of bacterial endoglucanases that can be used in the processes of the present invention, include, but are not limited to, an *Acidothermus cellulolyticus* endoglucanase (WO 91/05039; WO 93/15186; U.S. Patent No. 5,275,944; WO 96/02551; U.S. Patent No. 5,536,655, WO 00/70031, WO 05/093050); *Thermobifida fusca* endoglucanase III (WO 05/093050); and *Thermobifida fusca* endoglucanase V (WO 05/093050).

Examples of fungal endoglucanases that can be used in the present invention, include, but are not limited to, a *Trichoderma reesei* endoglucanase I (Penttila et al., 1986, Gene 45: 253-263, *Trichoderma reesei* Cel7B endoglucanase I (GENBANK™ accession no. M15665), *Trichoderma reesei* endoglucanase II (Saloheimo et al., 1988, Gene 63:11-22), *Trichoderma reesei* Cel5A endoglucanase II (GENBANK™ accession no. M19373), *Trichoderma reesei* endoglucanase III (Okada et al., 1988, Appl. Environ. Microbiol. 64: 555-563, GENBANK™ accession no. AB003694), *Trichoderma reesei* endoglucanase V (Saloheimo et al., 1994, Molecular Microbiology 13: 219-228, GENBANK™ accession no. Z33381), *Aspergillus aculeatus* endoglucanase (Ooi et al., 1990, Nucleic Acids Research 18: 5884), *Aspergillus kawachii* endoglucanase (Sakamoto et al., 1995, Current Genetics 27: 435-439), *Erwinia carotovara* endoglucanase (Saarilahti et al., 1990, Gene 90: 9-14), *Fusarium oxysporum* endoglucanase (GENBANK™ accession no. L29381), *Humicola grisea* var. *thermoidea* endoglucanase (GENBANK™ accession no. AB003107), *Melanocarpus albomyces* endoglucanase (GENBANK™ accession no. MAL515703), *Neurospora crassa* endoglucanase (GENBANK™ accession no. XM_324477), *Humicola insolens* endoglucanase V, *Myceliophthora thermophila* CBS 117.65 endoglucanase, basidiomycete CBS 495.95 endoglucanase, basidiomycete CBS 494.95 endoglucanase, *Thielavia terrestris* NRRL 8126 CEL6B endoglucanase, *Thielavia terrestris* NRRL 8126 CEL6C endoglucanase, *Thielavia terrestris* NRRL 8126 CEL7C endoglucanase, *Thielavia terrestris* NRRL 8126 CEL7E endoglucanase, *Thielavia terrestris* NRRL 8126 CEL7F endoglucanase, *Cladorrhinum foecundissimum* ATCC 62373 CEL7A endoglucanase, and *Trichoderma reesei* strain No. VTT-D-80133 endoglucanase (GENBANK™ accession no. M15665).

Examples of cellobiohydrolases useful in the present invention include, but are not limited to, *Aspergillus aculeatus* cellobiohydrolase II (WO 2011/059740), *Chaetomium thermophilum* cellobiohydrolase I, *Chaetomium thermophilum* cellobiohydrolase II, *Humicola insolens* cellobiohydrolase I, *Myceliophthora thermophila* cellobiohydrolase II (WO 2009/042871), *Thielavia hyrcanie* cellobiohydrolase II (WO 2010/141325), *Thielavia terrestris* cellobiohydrolase II (CEL6A, WO 2006/074435), *Trichoderma reesei* cellobiohydrolase I, *Trichoderma reesei* cellobiohydrolase II, and *Trichophaea saccata* cellobiohydralase II (WO 2010/057086).

Examples of beta-glucosidases useful in the present invention include, but are not limited to, beta-glucosidases from *Aspergillus aculeatus* (Kawaguchi et al., 1996, Gene 173: 287-288), *Aspergillus fumigatus* (WO 2005/047499), *Aspergillus niger* (Dan et al., 2000, J. Biol. Chem. 275: 4973-4980), *Aspergillus oryzae* (WO 2002/095014), *Penicillium brasilianum* IBT 20888 (WO 2007/019442 and WO 2010/088387), *Thielavia terrestris* (WO 2011/035029), and *Trichophaea saccata* (WO 2007/019442).

The beta-glucosidase may be a fusion protein. In one aspect, the beta-glucosidase is an *Aspergillus oryzae* beta-glucosidase variant BG fusion protein (WO 2008/057637) or an *Aspergillus oryzae* beta-glucosidase fusion protein (WO 2008/057637). In one aspect the beta-glucosidase is an *Aspergillus fumigatus* beta-glucosidase (SEQ ID NO: 2 in WO2005/047499) or a variant thereof disclosed in WO 2012/044915, such as one with one or more of the following substitutions" F100D, S283G, N456E, F512Y.

Other useful endoglucanases, cellobiohydrolases, and beta-glucosidases are disclosed in numerous Glycosyl Hydrolase families using the classification according to Henrissat B., 1991, A classification of glycosyl hydrolases based on amino-acid sequence similarities, Biochem. J. 280: 309-316, and Henrissat and Bairoch, 1996, Updating the sequence-based classification of glycosyl hydrolases, Biochem. J. 316: 695-696.

Other cellulolytic enzymes that may be used in the present invention are described in WO 98/13465, WO 98/015619, WO 98/015633, WO 99/06574, WO 99/10481, WO 99/025847, WO 99/031255, WO 2002/101078, WO 2003/027306, WO 2003/052054, WO 2003/052055, WO 2003/052056, WO 2003/052057, WO 2003/052118, WO 2004/016760, WO 2004/043980, WO 2004/048592, WO 2005/001065, WO 2005/028636, WO 2005/093050, WO 2005/093073, WO 2006/074005, WO 2006/117432, WO 2007/071818, WO 2007/071820, WO 2008/008070, WO 2008/008793, U.S. Patent No. 5,457,046, U.S. Patent No. 5,648,263, and U.S. Patent No. 5,686,593.

In the processes of the present invention, any GH61 polypeptide having cellulolytic enhancing activity can be used.

Examples of GH61 polypeptides having cellulolytic enhancing activity useful in the processes of the present invention include, but are not limited to, GH61 polypeptides from *Thielavia terrestris* (WO 2005/074647, WO 2008/148131, and WO 2011/035027), *Thermoascus aurantiacus* (WO 2005/074656 and WO 2010/065830), *Trichoderma reesei* (WO 2007/089290), *Myceliophthora thermophila* (WO 2009/085935, WO 2009/085859, WO 2009/085864, WO 2009/085868), *Aspergillus fumigatus* (WO 2010/138754), GH61 polypeptides from *Penicillium pinophilum* (WO 2011/005867), *Thermoascus* sp. (WO 2011/039319), *Penicillium* sp. (WO 2011/041397), and *Thermoascus crustaceous* (WO 2011/041504).

In one aspect, the GH61 polypeptide having cellulolytic enhancing activity is used in the presence of a soluble activating divalent metal cation according to WO 2008/151043, *e.g.*, manganese sulfate.

In one aspect, the GH61 polypeptide having cellulolytic enhancing activity is used in the presence of a dioxy compound, a bicylic compound, a heterocyclic compound, a nitrogen-containing compound, a quinone compound, a sulfur-containing compound, or a liquor obtained from a pretreated cellulosic material such as pretreated corn stover (PCS).

The dioxy compound may include any suitable compound containing two or more oxygen atoms. In some aspects, the dioxy compounds contain a substituted aryl moiety as described herein. The dioxy compounds may comprise one or more (*e.g.*, several) hydroxyl and/or hydroxyl derivatives, but also include substituted aryl moieties lacking hydroxyl and hydroxyl derivatives. Non-limiting examples of the dioxy compounds include pyrocatechol or catechol; caffeic acid; 3,4-dihydroxybenzoic acid; 4-tert-butyl-5-methoxy-1,2-benzenediol; pyrogallol; gallic acid; methyl-3,4,5-trihydroxybenzoate; 2,3,4-trihydroxybenzophenone; 2,6-dimethoxyphenol; sinapinic acid; 3,5-dihydroxybenzoic acid; 4-chloro-1,2-benzenediol; 4-nitro-1,2-benzenediol; tannic acid; ethyl gallate; methyl glycolate; dihydroxyfumaric acid; 2-butyne-1,4-diol; (croconic acid; 1,3-propanediol; tartaric acid; 2,4-pentanediol; 3-ethyoxy-1,2-propanediol; 2,4,4'-trihydroxybenzophenone; cis-2-butene-1,4-diol; 3,4-dihydroxy-3-cyclobutene-1,2-dione; dihydroxyacetone; acrolein acetal; methyl-4-hydroxybenzoate; 4-hydroxybenzoic acid; and methyl-3,5-dimethoxy-4-hydroxybenzoate; or a salt or solvate thereof.

The bicyclic compound may include any suitable substituted fused ring system as described herein. The compounds may comprise one or more (e.g., several) additional rings, and are not limited to a specific number of rings unless otherwise stated. In one aspect, the bicyclic compound is a flavonoid. In another aspect, the bicyclic compound is an optionally subsituted isoflavonoid. In another aspect, the bicyclic compound is an optionally substituted flavylium ion, such as an optionally substituted anthocyanidin or optionally substituted anthocyanin, or derivative thereof. Non-limiting examples of the bicyclic compounds include epicatechin; quercetin; myricetin; taxifolin; kaempferol; morin; acacetin; naringenin; isorhamnetin; apigenin; cyanidin; cyanin; kuromanin; keracyanin; or a salt or solvate thereof.

The heterocyclic compound may be any suitable compound, such as an optionally substituted aromatic or non-aromatic ring comprising a heteroatom, as described herein. In one aspect, the heterocyclic is a compound comprising an optionally substituted heterocycloalkyl moiety or an optionally substituted heteroaryl moiety. In another aspect, the optionally substituted heterocycloalkyl moiety or optionally substituted heteroaryl moiety is an optionally substituted 5-membered heterocycloalkyl or an optionally substituted 5-membered heteroaryl moiety. In another aspect, the optionally substituted heterocycloalkyl or optionally substituted heteroaryl moiety is an optionally substituted moiety selected from pyrazolyl, furanyl, imidazolyl, isoxazolyl, oxadiazolyl, oxazolyl, pyrrolyl, pyridyl, pyrimidyl, pyridazinyl, thiazolyl, triazolyl, thienyl, dihydrothieno-pyrazolyl, thianaphthenyl, carbazolyl, benzimidazolyl, benzothienyl, benzofuranyl, indolyl, quinolinyl, benzotriazolyl, benzothiazolyl, benzooxazolyl, benzimidazolyl, isoquinolinyl, isoindolyl, acridinyl, benzoisazolyl, dimethylhydantoin, pyrazinyl, tetrahydrofuranyl, pyrrolinyl, pyrrolidinyl, morpholinyl, indolyl, diazepinyl, azepinyl, thiepinyl, piperidinyl, and oxepinyl. In another aspect, the optionally substituted heterocycloalkyl moiety or optionally substituted heteroaryl moiety is an optionally substituted furanyl. Non-limiting examples of the heterocyclic compounds include (1,2-dihydroxyethyl)-3,4-dihydroxyfuran-2(5H)-one; 4-hydroxy-5-methyl-3-furanone; 5-hydroxy-2(5H)-furanone; [1,2-dihydroxyethyl]furan-2,3,4(5H)-trione; $\alpha$-hydroxy-$\gamma$-butyrolactone; ribonic $\gamma$-lactone; aldohexuronicaldohexuronic acid $\gamma$-lactone; gluconic acid $\delta$-lactone; 4-hydroxycoumarin; dihydrobenzofuran; 5-(hydroxymethyl)furfural; furoin; 2(5H)-furanone; 5,6-dihydro-2H-pyran-2-one; and 5,6-dihydro-4-hydroxy-6-methyl-2H-pyran-2-one; or a salt or solvate thereof.

The nitrogen-containing compound may be any suitable compound with one or more nitrogen atoms. In one aspect, the nitrogen-containing compound comprises an amine, imine, hydroxylamine, or nitroxide moiety. Non-limiting examples of thenitrogen-containing compounds include acetone oxime; violuric acid; pyridine-2-aldoxime; 2-aminophenol; 1,2-benzenediamine; 2,2,6,6-tetramethyl-1-piperidinyloxy; 5,6,7,8-tetrahydrobiopterin; 6,7-dimethyl-5,6,7,8-tetrahydropterine; and maleamic acid; or a salt or solvate thereof.

The quinone compound may be any suitable compound comprising a quinone moiety as described herein. Non-limiting examples of the quinone compounds include 1,4-benzoquinone; 1,4-naphthoquinone; 2-hydroxy-1,4-naphthoquinone; 2,3-dimethoxy-5-methyl-1,4-benzoquinone or coenzyme $Q_0$; 2,3,5,6-tetramethyl-1,4-benzoquinone or duroquinone; 1,4-dihydroxyanthraquinone; 3-hydroxy-1-methyl-5,6-indolinedione or adrenochrome; 4-tert-butyl-5-methoxy-1,2-benzoquinone; pyrroloquinoline quinone; or a salt or solvate thereof.

The sulfur-containing compound may be any suitable compound comprising one or more sulfur atoms. In one aspect, the sulfur-containing comprises a moiety selected from thionyl, thioether, sulfinyl, sulfonyl, sulfamide, sulfonamide, sulfonic acid, and sulfonic ester. Non-limiting examples of the sulfur-containing compounds include ethanethiol; 2-propanethiol; 2-propene-1-thiol; 2-mercaptoethanesulfonic acid; benzenethiol; benzene-1,2-dithiol; cysteine; methionine; glutathione; cystine; or a salt or solvate thereof.

In one aspect, an effective amount of such a compound described above to cellulosic material as a molar ratio to glucosyl units of cellulose is about $10^{-6}$ to about 10, e.g., about $10^{-6}$ to about 7.5, about $10^{-6}$ to about 5, about $10^{-6}$ to about 2.5, about $10^{-6}$ to about 1, about $10^{-5}$ to about 1, about $10^{-5}$ to about $10^{-1}$, about $10^{-4}$ to about $10^{-1}$, about $10^{-3}$ to about $10^{-1}$, or about $10^{-3}$ to about $10^{-2}$. In another aspect, an effective amount of such a compound described above is about 0.1 $\mu$M to about 1 M, e.g., about 0.5 $\mu$M to about 0.75 M, about 0.75 $\mu$M to about 0.5 M, about 1 $\mu$M to about 0.25 M, about 1 $\mu$M to about 0.1 M, about 5 $\mu$M to about 50 mM, about 10 $\mu$M to about 25 mM, about 50 $\mu$M to about 25 mM, about 10 $\mu$M to about 10 mM, about 5 $\mu$M to about 5 mM, ord about 0.1 mM to about 1 mM.

The term "liquor" means the solution phase, either aqueous, organic, or a combination thereof, arising from treatment of a lignocellulose and/or hemicellulose material in a slurry, or monosaccharides thereof, e.g., xylose, arabinose, mannose, etc., under conditions as described herein, and the soluble contents thereof. A liquor for cellulolytic enhancement of a GH61 polypeptide can be produced by treating a lignocellulose or hemicellulose material (or feedstock) by applying heat and/or pressure, optionally in the presence of a catalyst, e.g., acid, optionally in the presence of an organic solvent, and optionally in combination with physical disruption of the material, and then separating the solution from the residual solids. Such conditions determine the degree of cellulolytic enhancement obtainable through the combination of liquor and a GH61 polypeptide during hydrolysis of a cellulosic substrate by a cellulase preparation. The liquor can be separated from the treated material using a method standard in the art, such as filtration, sedimentation, or centrifugation.

In one aspect, an effective amount of the liquor to cellulose is about $10^{-6}$ to about 10 g per g of cellulose, *e.g.*, about $10^{-6}$ to about 7.5 g, about $10^{-6}$ to about 5, about $10^{-6}$ to about 2.5 g, about $10^{-6}$ to about 1 g, about $10^{-5}$ to about 1 g, about $10^{-5}$ to about $10^{-1}$ g, about $10^{-4}$ to about $10^{-1}$ g, about $10^{-3}$ to about $10^{-1}$ g, or about $10^{-3}$ to about $10^{-2}$ g per g of cellulose.

In one aspect, the one or more (*e.g.*, several) hemicellulolytic enzymes comprise a commercial hemicellulolytic enzyme preparation. Examples of commercial hemicellulolytic enzyme preparations suitable for use in the present invention include, for example, SHEARZYME™ (Novozymes A/S), CELLIC™ HTec (Novozymes A/S), CELLIC™ HTec2 (Novozymes A/S), VISCOZYME® (Novozymes A/S), ULTRAFLO® (Novozymes A/S), PULPZYME® HC (Novozymes A/S), MULTIFECT® Xylanase (Genencor), ACCELLERASE® XY (Genencor), ACCELLERASE® XC (Genencor), ECOPULP® TX-200A (AB Enzymes), HSP 6000 Xylanase (DSM), DEPOL™ 333P (Biocatalysts Limit, Wales, UK), DEPOL™ 740L. (Biocatalysts Limit, Wales, UK), and DEPOL™ 762P (Biocatalysts Limit, Wales, UK).

Examples of xylanases useful in the processes of the present invention include, but are not limited to, xylanases from *Aspergillus aculeatus* (GeneSeqP:AAR63790; WO 94/21785), *Aspergillus fumigatus* (WO 2006/078256), *Penicillium pinophilum* (WO 2011/041405), *Penicillium* sp. (WO 2010/126772), *Thielavia terrestris* NRRL 8126 (WO 2009/079210), and *Trichophaea saccata* GH10 (WO 2011/057083).

Examples of beta-xylosidases useful in the processes of the present invention include, but are not limited to, beta-xylosidases from *Neurospora crassa* (SwissProt accession number Q7SOW4), *Trichoderma reesei* (UniProtKB/TrEMBL accession number Q92458), and *Talaromyces emersonii* (SwissProt accession number Q8X212).

Examples of acetylxylan esterases useful in the processes of the present invention include, but are not limited to, acetylxylan esterases from *Aspergillus aculeatus* (WO 2010/108918), *Chaetomium globosum* (Uniprot accession number Q2GWX4), *Chaetomium gracile* (GeneSeqP accession number AAB82124), *Humicola insolens* DSM 1800 (WO 2009/073709), *Hypocrea jecorina* (WO 2005/001036), *Myceliophtera thermophila* (WO 2010/014880), *Neurospora crassa* (UniProt accession number q7s259), *Phaeosphaeria nodorum* (Uniprot accession number Q0UHJ1), and *Thielavia terrestris* NRRL 8126 (WO 2009/042846).

Examples of feruloyl esterases (ferulic acid esterases) useful in the processes of the present invention include, but are not limited to, feruloyl esterases form *Humicola insolens* DSM 1800 (WO 2009/076122), *Neosartorya fischeri* (UniProt Accession number A1D9T4), *Neurospora crassa* (UniProt accession number Q9HGR3), *Penicillium aurantiogriseum* (WO 2009/127729), and *Thielavia terrestris* (WO 2010/053838 and WO 2010/065448).

Examples of arabinofuranosidases useful in the processes of the present invention include, but are not limited to, arabinofuranosidases from *Aspergillus niger* (GeneSeqP accession number AAR94170), *Humicola insolens* DSM 1800 (WO 2006/114094 and WO 2009/073383), and *M. giganteus* (WO 2006/114094).

Examples of alpha-glucuronidases useful in the processes of the present invention include, but are not limited to, alpha-glucuronidases from *Aspergillus clavatus* (UniProt accession number alcc12), *Aspergillus fumigatus* (SwissProt accession number Q4WW45), *Aspergillus niger* (Uniprot accession number Q96WX9), *Aspergillus terreus* (SwissProt accession number Q0CJP9), *Humicola insolens* (WO 2010/014706), *Penicillium aurantiogriseum* (WO 2009/068565), *Talaromyces emersonii* (UniProt accession number Q8X211), and *Trichoderma reesei* (Uniprot accession number Q99024).The polypeptides having enzyme activity used in the processes of the present invention may be produced by fermentation of the above-noted microbial strains on a nutrient medium containing suitable carbon and nitrogen sources and inorganic salts, using procedures known in the art (see, *e.g.*, Bennett, J.W. and LaSure, L. (eds.), More Gene Manipulations in Fungi, Academic Press, CA, 1991). Suitable media are available from commercial suppliers or may be prepared according to published compositions (*e.g.*, in catalogues of the American Type Culture Collection). Temperature ranges and other conditions suitable for growth and enzyme production are known in the art (see, *e.g.*, Bailey, J.E., and Ollis, D.F., Biochemical Engineering Fundamentals, McGraw-Hill Book Company, NY, 1986).

The fermentation can be any method of cultivation of a cell resulting in the expression or isolation of an enzyme or protein. Fermentation may, therefore, be understood as comprising shake flask cultivation, or small- or large-scale fermentation (including continuous, batch, fed-batch, or solid state fermentations) in laboratory or industrial fermentors performed in a suitable medium and under conditions allowing the enzyme to be expressed or isolated. The resulting enzymes produced by the methods described above may be recovered from the fermentation medium and purified by conventional procedures.

**Nucleic Acid Constructs**

An isolated polynucleotide encoding a polypeptide, *e.g.*, a lipase, protease, pectinase, a GH61 polypeptide having cellulolytic enhancing activity, a cellulolytic enzyme, a hemicellulolytic enzyme, etc., may be manipulated in a variety of ways to provide for expression of the polypeptide by constructing a nucleic acid construct comprising an isolated polynucleotide encoding the polypeptide operably linked to one or more (several) control sequences that direct the expression of the coding sequence in a suitable host cell under conditions compatible with the control sequences.

A polynucleotide may be manipulated in a variety of ways to provide for expression of the polypeptide. Manipulation of the polynucleotide prior to its insertion into a vector may be desirable or necessary depending on the expression vector. The techniques for modifying polynucleotides utilizing recombinant DNA methods are well known in the art.

The control sequence may be a promoter, a polynucleotide that is recognized by a host cell for expression of a polynucleotide encoding a polypeptide of the present invention. The promoter contains transcriptional control sequences that mediate the expression of the polypeptide. The promoter may be any polynucleotide that shows transcriptional activity in the host cell including mutant, truncated, and hybrid promoters, and may be obtained from genes encoding extracellular or intracellular polypeptides either homologous or heterologous to the host cell.

Examples of suitable promoters for directing transcription of the nucleic acid constructs of the present invention in a bacterial host cell are the promoters obtained from the *Bacillus amyloliquefaciens* alpha-amylase gene (*amyQ*), *Bacillus licheniformis* alpha-amylase gene *(amyL)*, *Bacillus licheniformis* penicillinase gene (*penP*), *Bacillus stearothermophilus* maltogenic amylase gene (*amyM*), *Bacillus subtilis* levansucrase gene (*sacB*), *Bacillus subtilis xylA* and *xylB* genes, *Bacillus thuringiensis cryIIIA* gene (Agaisse and Lereclus, 1994, Molecular Microbiology 13: 97-107), *E. coli lac* operon, *E. coli trc* promoter (Egon et al., 1988, Gene 69: 301-315), *Streptomyces coelicolor* agarase gene (*dagA*), and prokaryotic beta-lactamase gene (Villa-Kamaroff et al., 1978, Proc. Natl. Acad. Sci. USA 75: 3727-3731), as well as the *tac* promoter (DeBoer et al., 1983, Proc. Natl. Acad. Sci. USA 80: 21-25). Further promoters are described in "Useful proteins from recombinant bacteria" in Gilbert et al., 1980, Scientific American 242: 74-94; and in Sambrook *et al.,* 1989, *supra.* Examples of tandem promoters are disclosed in WO 99/43835.

Examples of suitable promoters for directing transcription of the nucleic acid constructs of the present invention in a filamentous fungal host cell are promoters obtained from the genes for *Aspergillus nidulans* acetamidase, *Aspergillus niger* neutral alpha-amylase, *Aspergillus niger* acid stable alpha-amylase, *Aspergillus niger* or *Aspergillus awamori* glucoamylase (*glaA*), *Aspergillus oryzae* TAKA amylase, *Aspergillus oryzae* alkaline protease, *Aspergillus oryzae* triose phosphate isomerase, *Fusarium oxysporum* trypsin-like protease (WO 96/00787), *Fusarium venenatum* amyloglucosidase (WO 00/56900), *Fusarium venenatum* Daria (WO 00/56900), *Fusarium venenatum* Quinn (WO 00/56900), *Rhizomucor miehei* lipase, *Rhizomucor miehei* aspartic proteinase, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase IV, *Trichoderma reesei* endoglucanase V, *Trichoderma reesei* xylanase I, *Trichoderma reesei* xylanase II, *Trichoderma reesei* beta-xylosidase, as well as the NA2-tpi promoter (a modified promoter from an *Aspergillus* neutral alpha-amylase gene in which the untranslated leader has been replaced by an untranslated leader from an *Aspergillus* triose phosphate isomerase gene; non-limiting examples include modified promoters from an *Aspergillus niger* neutral alpha-amylase gene in which the untranslated leader has been replaced by an untranslated leader from an *Aspergillus nidulans* or *Aspergillus oryzae* triose phosphate isomerase gene); and mutant, truncated, and hybrid promoters thereof.

In a yeast host, useful promoters are obtained from the genes for *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH1, ADH2/GAP), *Saccharomyces cerevisiae* triose phosphate isomerase (TPI), *Saccharomyces cerevisiae* metallothionein (CUP1), and *Saccharomyces cerevisiae* 3-phosphoglycerate kinase. Other useful promoters for yeast host cells are described by Romanos et al., 1992, Yeast 8: 423-488.

The control sequence may also be a transcription terminator, which is recognized by a host cell to terminate transcription. The terminator is operably linked to the 3'-terminus of the polynucleotide encoding the polypeptide. Any terminator that is functional in the host cell may be used in the present invention.

Preferred terminators for bacterial host cells are obtained from the genes for *Bacillus clausii* alkaline protease (*aprH*)*, Bacillus licheniformis* alpha-amylase (*amyL*)*,* and *Escherichia coli* ribosomal RNA (*rrnB*).

Preferred terminators for filamentous fungal host cells are obtained from the genes for *Aspergillus nidulans* anthranilate synthase, *Aspergillus niger* glucoamylase, *Aspergillus niger* alpha-glucosidase, *Aspergillus oryzae* TAKA amylase, and *Fusarium oxysporum* trypsin-like protease.

Preferred terminators for yeast host cells are obtained from the genes for *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos *et al.,* 1992, *supra.*

The control sequence may also be an mRNA stabilizer region downstream of a promoter and upstream of the coding sequence of a gene which increases expression of the gene.

Examples of suitable mRNA stabilizer regions are obtained from a *Bacillus thuringiensis cryIIIA* gene (WO 94/25612) and a *Bacillus subtilis* SP82 gene (Hue et al., 1995, Journal of Bacteriology 177: 3465-3471).

The control sequence may also be an mRNA stabilizer region downstream of a promoter and upstream of the coding sequence of a gene which increases expression of the gene.

Examples of suitable mRNA stabilizer regions are obtained from a *Bacillus thuringiensis cryIIIA* gene (WO 94/25612) and a *Bacillus subtilis* SP82 gene (Hue et al., 1995, Journal of Bacteriology 177: 3465-3471).

The control sequence may also be a leader, a nontranslated region of an mRNA that is important for translation by the host cell. The leader is operably linked to the 5'-terminus of the polynucleotide encoding the polypeptide. Any leader that is functional in the host cell may be used.

Preferred leaders for filamentous fungal host cells are obtained from the genes for *Aspergillus oryzae* TAKA amylase and *Aspergillus nidulans* triose phosphate isomerase.

Suitable leaders for yeast host cells are obtained from the genes for *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

The control sequence may also be a polyadenylation sequence, a sequence operably linked to the 3'-terminus of the polynucleotide and, when transcribed, is recognized by the host cell as a signal to add polyadenosine residues to transcribed mRNA. Any polyadenylation sequence that is functional in the host cell may be used.

Preferred polyadenylation sequences for filamentous fungal host cells are obtained from the genes for *Aspergillus nidulans* anthranilate synthase, *Aspergillus niger* glucoamylase, *Aspergillus niger* alpha-glucosidase *Aspergillus oryzae* TAKA amylase, and *Fusarium oxysporum* trypsin-like protease.

Useful polyadenylation sequences for yeast host cells are described by Guo and Sherman, 1995, Mol. Cellular Biol. 15: 5983-5990.

The control sequence may also be a signal peptide coding region that encodes a signal peptide linked to the N-terminus of a polypeptide and directs the polypeptide into the cell's secretory pathway. The 5'-end of the coding sequence of the polynucleotide may inherently contain a signal peptide coding sequence naturally linked in translation reading frame with the segment of the coding sequence that encodes the polypeptide. Alternatively, the 5'-end of the coding sequence may contain a signal peptide coding sequence that is foreign to the coding sequence. A foreign signal peptide coding sequence may be required where the coding sequence does not naturally contain a signal peptide coding sequence. Alternatively, a foreign signal peptide coding sequence may simply replace the natural signal peptide coding sequence in order to enhance secretion of the polypeptide. However, any signal peptide coding sequence that directs the expressed polypeptide into the secretory pathway of a host cell may be used.

Effective signal peptide coding sequences for bacterial host cells are the signal peptide coding sequences obtained from the genes for *Bacillus* NCIB 11837 maltogenic amylase, *Bacillus licheniformis* subtilisin, *Bacillus licheniformis* beta-lactamase, *Bacillus stearothermophilus* alpha-amylase, *Bacillus stearothermophilus* neutral proteases (*nprT, nprS, nprM*), and *Bacillus subtilis prsA*. Further signal peptides are described by Simonen and Palva, 1993, Microbiological Reviews 57: 109-137.

Effective signal peptide coding sequences for filamentous fungal host cells are the signal peptide coding sequences obtained from the genes for *Aspergillus niger* neutral amylase, *Aspergillus niger* glucoamylase, *Aspergillus oryzae* TAKA amylase, *Humicola insolens* cellulase, *Humicola insolens* endoglucanase V, *Humicola lanuginosa* lipase, and *Rhizomucor miehei* aspartic proteinase.

Useful signal peptides for yeast host cells are obtained from the genes for *Saccharomyces cerevisiae* alpha-factor and *Saccharomyces cerevisiae* invertase. Other useful signal peptide coding sequences are described by Romanos *et al.,* 1992, *supra.*

The control sequence may also be a propeptide coding sequence that encodes a propeptide positioned at the N-terminus of a polypeptide. The resultant polypeptide is known as a proenzyme or propolypeptide (or a zymogen in some cases). A propolypeptide is generally inactive and can be converted to an active polypeptide by catalytic or autocatalytic cleavage of the propeptide from the propolypeptide. The propeptide coding sequence may be obtained from the genes for *Bacillus subtilis* alkaline protease (*aprE*), *Bacillus subtilis* neutral protease (*nprT*), *Myceliophthora thermophila* laccase (WO 95/33836), *Rhizomucor miehei* aspartic proteinase, and *Saccharomyces cerevisiae* alpha-factor.

Where both signal peptide and propeptide sequences are present, the propeptide sequence is positioned next to the N-terminus of a polypeptide and the signal peptide sequence is positioned next to the N-terminus of the propeptide sequence.

It may also be desirable to add regulatory sequences that regulate expression of the polypeptide relative to the growth of the host cell. Examples of regulatory systems are those that cause expression of the gene to be turned on or off in response to a chemical or physical stimulus, including the presence of a regulatory compound. Regulatory systems in prokaryotic systems include the *lac, tac,* and *trp* operator systems. In yeast, the ADH2 system or GAL1 system may be used. In filamentous fungi, the *Aspergillus niger* glucoamylase promoter, *Aspergillus oryzae* TAKA alpha-amylase promoter, and *Aspergillus oryzae* glucoamylase promoter may be used. Other examples of regulatory sequences are those that allow for gene amplification. In eukaryotic systems, these regulatory sequences include the dihydrofolate reductase gene that is amplified in the presence of methotrexate, and the metallothionein genes that are amplified with heavy metals. In these cases, the polynucleotide encoding the polypeptide would be operably linked with the regulatory sequence.

## Expression Vectors

The present invention also relates to recombinant expression vectors comprising a polynucleotide of the present invention, a promoter, and transcriptional and translational stop signals. The various nucleotide and control sequences may be joined together to produce a recombinant expression vector that may include one or more convenient restriction sites to allow for insertion or substitution of the polynucleotide encoding the polypeptide at such sites. Alternatively, the polynucleotide may be expressed by inserting the polynucleotide or a nucleic acid construct comprising the polynucleotide into an appropriate vector for expression. In creating the expression vector, the coding sequence is located in the vector so that the coding sequence is operably linked with the appropriate control sequences for expression.

The recombinant expression vector may be any vector (*e.g.*, a plasmid or virus) that can be conveniently subjected to recombinant DNA procedures and can bring about expression of the polynucleotide. The choice of the vector will typically depend on the compatibility of the vector with the host cell into which the vector is to be introduced. The vector may be a linear or closed circular plasmid.

The vector may be an autonomously replicating vector, *i.e.,* a vector that exists as an extrachromosomal entity, the replication of which is independent of chromosomal replication, *e.g.*, a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vector preferably contains one or more selectable markers that permit easy selection of transformed, transfected, transduced, or the like cells. A selectable marker is a gene the product of which provides for biocide or viral resistance, resistance to heavy metals, prototrophy to auxotrophs, and the like.

Examples of bacterial selectable markers are *Bacillus licheniformis* or *Bacillus subtilis dal* genes, or markers that confer antibiotic resistance such as ampicillin, chloramphenicol, kanamycin, neomycin, spectinomycin, or tetracycline resistance. Suitable markers for yeast host cells include, but are not limited to, ADE2, HIS3, LEU2, LYS2, MET3, TRP1, and URA3. Selectable markers for use in a filamentous fungal host cell include, but are not limited to, *amdS* (acetamidase), *argB* (ornithine carbamoyltransferase), bar (phosphinothricin acetyltransferase), *hph* (hygromycin phosphotransferase), *niaD* (nitrate reductase), *pyrG* (orotidine-5'-phosphate decarboxylase), sC (sulfate adenyltransferase), and *trpC* (anthranilate synthase), as well as equivalents thereof. Preferred for use in an *Aspergillus* cell are *Aspergillus nidulans* or *Aspergillus oryzae amdS* and *pyrG* genes and a *Streptomyces hygroscopicus bar gene.*

The vector preferably contains an element(s) that permits integration of the vector into the host cell's genome or autonomous replication of the vector in the cell independent of the genome.

For integration into the host cell genome, the vector may rely on the polynucleotide's sequence encoding the polypeptide or any other element of the vector for integration into the genome by homologous or non-homologous recombination. Alternatively, the vector may contain additional polynucleotides for directing integration by homologous recombination into the genome of the host cell at a precise location(s) in the chromosome(s). To increase the likelihood of integration at a precise location, the integrational elements should contain a sufficient number of nucleic acids, such as 100 to 10,000 base pairs, 400 to 10,000 base pairs, and 800 to 10,000 base pairs, which have a high degree of sequence identity to the corresponding target sequence to enhance the probability of homologous recombination. The integrational elements may be any sequence that is homologous with the target sequence in the genome of the host cell. Furthermore, the integrational elements may be non-encoding or encoding polynucleotides. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate *in vivo.*

Examples of bacterial origins of replication are the origins of replication of plasmids pBR322, pUC19, pACYC177, and pACYC184 permitting replication in *E. coli,* and pUB110, pE194, pTA1060, and pAMβ1 permitting replication in *Bacillus.*

Examples of origins of replication for use in a yeast host cell are the 2 micron origin of replication, ARS1, ARS4, the combination of ARS1 and CEN3, and the combination of ARS4 and CEN6.

Examples of origins of replication useful in a filamentous fungal cell are AMA1 and ANS1 (Gems et al., 1991, Gene 98: 61-67; Cullen et al., 1987, Nucleic Acids Res. 15: 9163-9175; WO 00/24883). Isolation of the AMA1 gene and construction of plasmids or vectors comprising the gene can be accomplished according to the methods disclosed in WO 00/24883.

More than one copy of a polynucleotide of the present invention may be inserted into a host cell to increase production of a polypeptide. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the elements described above to construct the recombinant expression vectors of the present invention are well known to one skilled in the art (see, *e.g.,* Sambrook *et al.,* 1989, *supra).*

**Host Cells**

The present disclosure also relates to recombinant host cells, comprising a polynucleotide operably linked to one or more control sequences that direct the production of a polypeptide. A construct or vector comprising a polynucleotide is introduced into a host cell so that the construct or vector is maintained as a chromosomal integrant or as a self-replicating extra-chromosomal vector as described earlier. The term "host cell" encompasses any progeny of a parent cell that is not identical to the parent cell due to mutations that occur during replication. The choice of a host cell will to a large extent depend upon the gene encoding the polypeptide and its source.

The host cell may be any cell useful in the recombinant production of a polypeptide of the present invention, *e.g.*, a prokaryote or a eukaryote.

The prokaryotic host cell may be any Gram-positive or Gram-negative bacterium. Gram-positive bacteria include, but are not limited to, *Bacillus, Clostridium, Enterococcus, Geobacillus, Lactobacillus, Lactococcus, Oceanobacillus, Staphylococcus, Streptococcus,* and *Streptomyces.* Gram-negative bacteria include, but are not limited to, *Campylobacter, E. coli, Flavobacterium, Fusobacterium, Helicobacter, Ilyobacter, Neisseria, Pseudomonas, Salmonella,* and *Ureaplasma.*

The bacterial host cell may be any *Bacillus* cell including, but not limited to, *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis* cells.

The bacterial host cell may also be any *Streptococcus* cell including, but not limited to, *Streptococcus equisimilis, Streptococcus pyogenes, Streptococcus uberis,* and *Streptococcus equi* subsp. *Zooepidemicus* cells.

The bacterial host cell may also be any *Streptomyces* cell including, but not limited to, *Streptomyces achromogenes, Streptomyces avermitilis, Streptomyces coelicolor, Streptomyces griseus,* and *Streptomyces lividans* cells.

The introduction of DNA into a *Bacillus* cell may be effected by protoplast transformation (see, *e.g.,* Chang and Cohen, 1979, Mol. Gen. Genet. 168: 111-115), competent cell transformation (see, *e.g.,* Young and Spizizen, 1961, J. Bacteriol. 81: 823-829, or Dubnau and Davidoff-Abelson, 1971, J. Mol. Biol. 56: 209-221), electroporation (see, *e.g.,* Shigekawa and Dower, 1988, Biotechniques 6: 742-751), or conjugation (see, *e.g.,* Koehler and Thorne, 1987, J. Bacteriol. 169: 5271-5278). The introduction of DNA into an *E. coli* cell may be effected by protoplast transformation (see, *e.g.,* Hanahan, 1983, J. Mol. Biol. 166: 557-580) or electroporation (see, *e.g.,* Dower et al., 1988, Nucleic Acids Res. 16: 6127-6145). The introduction of DNA into a *Streptomyces* cell may be effected by protoplast transformation, electroporation (see, *e.g.,* Gong et al., 2004, Folia Microbiol. (Praha) 49: 399-405), conjugation (see, *e.g.,* Mazodier et al., 1989, J. Bacteriol. 171: 3583-3585), or transduction (see, *e.g.,* Burke et al., 2001, Proc. Natl. Acad. Sci. USA 98: 6289-6294). The introduction of DNA into a *Pseudomonas* cell may be effected by electroporation (see, *e.g.,* Choi et al., 2006, J. Microbiol. Methods 64: 391-397) or conjugation (see, *e.g.,* Pinedo and Smets, 2005, Appl. Environ. Microbiol. 71: 51-57). The introduction of DNA into a *Streptococcus* cell may be effected by natural competence (see, *e.g.,* Perry and Kuramitsu, 1981, Infect. Immun. 32: 1295-1297), protoplast transformation (see, *e.g.,* Catt and Jollick, 1991, Microbios 68: 189-207), electroporation (see, *e.g.,* Buckley et al., 1999, Appl. Environ. Microbiol. 65: 3800-3804), or conjugation (see, *e.g.,* Clewell, 1981, Microbiol. Rev. 45: 409-436). However, any method known in the art for introducing DNA into a host cell can be used.

The host cell may also be a eukaryote, such as a mammalian, insect, plant, or fungal cell.

The host cell may be a fungal cell. "Fungi" as used herein includes the phyla Ascomycota, Basidiomycota, Chytridiomycota, and Zygomycota as well as the Oomycota and all mitosporic fungi (as defined by Hawksworth et al., In, Ainsworth and Bisby's Dictionary of The Fungi, 8th edition, 1995, CAB International, University Press, Cambridge, UK).

The fungal host cell may be a yeast cell. "Yeast" as used herein includes ascosporogenous yeast (Endomycetales), basidiosporogenous yeast, and yeast belonging to the Fungi Imperfecti (Blastomycetes). Since the classification of yeast may change in the future, for the purposes of this invention, yeast shall be defined as described in Biology and Activities of Yeast (Skinner, Passmore, and Davenport, editors, Soc. App. Bacteriol. Symposium Series No. 9, 1980).

The yeast host cell may be a *Candida, Hansenula, Kluyveromyces, Pichia, Saccharomyces, Schizosaccharomyces,* or *Yarrowia* cell, such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccha-*

*romyces oviformis,* or *Yarrowia lipolytica* cell.

The fungal host cell may be a filamentous fungal cell. "Filamentous fungi" include all filamentous forms of the subdivision Eumycota and Oomycota (as defined by Hawksworth *et al.,* 1995, *supra*). The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

The filamentous fungal host cell may be an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell.

For example, the filamentous fungal host cell may be an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

Fungal cells may be transformed by a process involving protoplast formation, transformation of the protoplasts, and regeneration of the cell wall in a manner known *per se.* Suitable procedures for transformation of *Aspergillus* and *Trichoderma* host cells are described in EP 238023, Yelton et al., 1984, Proc. Natl. Acad. Sci. USA 81: 1470-1474, and Christensen et al., 1988, Bio/Technology 6: 1419-1422. Suitable methods for transforming *Fusarium* species are described by Malardier et al., 1989, Gene 78: 147-156, and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, J. Bacteriol. 153: 163; and Hinnen et al., 1978, Proc. Natl. Acad. Sci. USA 75: 1920.

**Examples**

**Example 1: Lipase, protease and pectinase as re-pulping aid enzymes to boost biomass hydrolysis.**

Old newspaper plus magazines in the ratio of 80:20 ("ONP") was used as the biomass substrate. ONP was shred and milled into small particles. Slurries were prepared with the milled ONP at 6% total solids (TS). The ONP slurries were treated in the Lab-O-Mat (LABOMAT <<BFA-24>>, Werner Mathis U.S.A. Inc., Concord, N.C., USA), with and without enzymes, at the following conditions:

- 6% TS, pH 7, 50°C and overnight (16 hours) (ONP control);
- 6%TS, pH 7, 50°C and overnight (16 hours) with lipase, protease and pectinase enzymes (10:1:10) (500 ppm lipase (SEQ ID NO:1) + 50 ppm protease (SEQ ID NO:2) + 500 ppm pectinase (SEQ ID NO:3) (ONP-Enz).

After treatment, hydrolysis was performed at 5.3% TS, pH 5.0, 50°C for 3 days, with cellulolytic enzyme solution (a *Trichoderma reesei* cellulase preparation containing *Aspergillus oryzae* beta-glucosidase fusion protein (WO 2008/057637) and *Thermoascus aurantiacus* GH61A polypeptide (WO 2005/074656) and an *Aspergillus aculeatus* GH10 xylanase (WO 94/021785) preparation at a ratio of 4:1), at total 3 mg-protein/g-substrate.

The collected samples were filtered using 0.20 $\mu$m syringe filters (Millipore, Bedford, MA, USA) and the filtrates were analyzed for sugar content as described below. When not used immediately, filtered aliquots were frozen at -20°C. The sugar concentrations of samples diluted in 0.005 M $H_2SO_4$ were measured using a 4.6 x 250 mm AMINEX® HPX-87H column (Bio-Rad Laboratories, Inc., Hercules, CA, USA) by elution with 0.05% w/w benzoic acid-0.005 M $H_2SO_4$ at 65°C at a flow rate of 0.6 ml per minute, and quantitation by integration of the glucose, cellobiose, and xylose signals from refractive index detection (CHEMSTATION®, AGILENT® 1100 HPLC, Agilent Technologies, Santa Clara, CA, USA) calibrated by pure sugar samples. The resultant glucose and cellobiose equivalents were used to calculate the percentage of cellulose conversion for each reaction.

Glucose, cellobiose, and xylose were measured individually. Measured sugar concentrations were adjusted for the appropriate dilution factor. The net concentrations of enzymatically-produced sugars were determined by adjusting the measured sugar concentrations for corresponding background sugar concentrations in unwashed biomass at zero time point. All HPLC data processing was performed using MICROSOFT EXCEL™ software (Microsoft, Richland, WA, USA).

The glucose released per substrate was calculated according to the following equation:

$$\text{Glucose released (g)} = \text{glucose concentration} / \text{\% total solids in hydrolysis.}$$

Results are presented in Table 1 showing hydrolysis performance of ONP and OPN enzymatic treated in accordance with the present disclosure at 5.3% TS, pH 5.0, 50°C for 3 days, with cellulolytic enzymes at 3 mg-protein/g-substrate.

Table 1.

|  | 3 days hydrolysis (g-Glucose/ kg-feedstock) |
|---|---|
| ONP - Control | 92 |
| ONP - Enzymatically treated in accordance with present disclosure | 104 |

**Example 2: Amylase enzymes as boosters for biomass hydrolysis**

Old corrugated cardboard (OCC) was used as the biomass substrate. OCC was shred and milled into small particles. Slurries were prepared with the milled OCC at 6% total solids (TS) and were re-pulped at pH 7, 50°C and overnight (16 hours) in the Lab-O-Mat (LABOMAT <<BFA-24>>, Werner Mathis U.S.A. Inc., Concord, N.C., USA).

After re-pulping, hydrolysis was performed at 5.3% TS, pH 5.0, 50°C for 3 days, with total 3 mg-protein/g-substrate with 2 different enzymes combos:

- (C+H) cellulolytic enzyme solution (a *Trichoderma reesei* cellulase preparation containing *Aspergillus oryzae* beta-glucosidase fusion protein (WO 2008/057637) and *Thermoascus aurantiacus* GH61A polypeptide (WO 2005/074656) and an *Aspergillus aculeatus* GH10 xylanase (WO 94/21785) preparation at a ratio of 4:1);
- (C+H+S) cellulolytic enzyme solution (a *Trichoderma reesei* cellulase preparation containing *Aspergillus oryzae* beta-glucosidase fusion protein (WO 2008/057637) and *Thermoascus aurantiacus* GH61A polypeptide (WO 2005/074656) and an *Aspergillus aculeatus* GH10 xylanase (WO 94/21785) preparation at a ratio of 4:1), plus a blend of *Rhizomucer pusillus* amylase (SEQ ID NO:4), *Talaromices emersoni* glucoamylase (SEQ ID NO:6) and *Trametes cingulata* glucoamylase (SEQ ID NO:7) at a ratio of 8:2:1.

The collection of samples, sugars measurements and glucose released calculations were followed as described in Example 1.

Results are presented in Table 2 showing hydrolysis performance of OCC with (C+H+S) and without (C+H) amylase, besides the cellulolytic enzymes, at total 3 mg-protein/g-substrate, at 5.3%TS, pH 5.0, 50°C for 3 days.

Table 2:

|  | 3 days hydrolysis (g-Glucose/ kg-feedstock) |
|---|---|
| OCC - Control | 232 |
| OCC - With amylase | 245 |

**Example 3: Amylase enzymes as boosters for biomass hydrolysis Experimental**

Mixed office waste (MOW) was used as the biomass substrate, which was shred, milled and re-pulped as in example 2.

After re-pulping, hydrolysis was performed at 5.3% TS, pH 5.0, 50°C for 3 days, with total 3 mg-protein/g-substrate with 2 different enzymes combos:

- (C+H) cellulolytic enzyme solution (a *Trichoderma reesei* cellulase preparation containing *Aspergillus oryzae* beta-glucosidase fusion protein (WO 2008/057637) and *Thermoascus aurantiacus* GH61A polypeptide (WO 2005/074656) and an *Aspergillus aculeatus* GH10 xylanase (WO 94/21785) preparation at a ratio of 4:1);
- (C+H+S) cellulolytic enzyme solution(a *Trichoderma reesei* cellulase preparation containing *Aspergillus oryzae* beta-glucosidase fusion protein (WO 2008/057637) and *Thermoascus aurantiacus* GH61A polypeptide (WO 2005/074656) and an *Aspergillus aculeatus* GH10 xylanase (WO 94/21785) preparation at a ratio of 4:1), plus a blend of *Rhizomucer pusillus* amylase (SEQ ID NO:4), *Talaromyces emersoni* glucoamylase (SEQ ID NO:6) and *Trametes cingulata* glucoamylase SEQ ID NO:7) at a ratio of 8:2:1 respectively.

The collection of samples, sugars measurements and glucose released calculations were followed as described in Example 1.

Results are presented in Table 3 showing hydrolysis performance of MOW with (C+H+S) and without (C+H) amylase, besides the cellulolytic enzymes, at 3 mg-protein/g-substrate, at 5.3% TS, pH 5.0, 50°C for 3 days.

Table 3:

|  | **3 days hydrolysis (g-Glucose/ kg-feedstock)** |
| --- | --- |
| MOW - Control | 316 |
| MOW - With amylase | 347 |

**Example 4: Amylase enzymes as boosters for biomass hydrolysis Experimental**

TetraPack packaging material (TetraPack) was used as the biomass substrate, which was shred, milled and re-pulped as in example 1.

After re-pulping, hydrolysis was performed at 5.3% TS, pH 5.0, 50°C for 3 days, with total 3 mg-protein/g-substrate with 2 different enzymes combos:

- (C+H) cellulolytic enzyme solution (a *Trichoderma reesei* cellulase preparation containing *Aspergillus oryzae* beta-glucosidase fusion protein (WO 2008/057637) and *Thermoascus aurantiacus* GH61A polypeptide (WO 2005/074656) and an *Aspergillus aculeatus* GH10 xylanase (WO 94/21785) preparation at a ratio of 4:1);
- (C+H+S) cellulolytic enzyme solution(a *Trichoderma reesei* cellulase preparation containing *Aspergillus oryzae* beta-glucosidase fusion protein (WO 2008/057637) and *Thermoascus aurantiacus* GH61A polypeptide (WO 2005/074656) and an *Aspergillus aculeatus* GH10 xylanase (WO 94/21785) preparation at a ratio of 4:1), plus a blend of *Rhizomucer pusillus* amylase (SEQ ID NO:4), *Talaromices emersoni* glucoamylase (SEQ ID NO:6) and *Trametes cingulata* glucoamylase (SEQ ID NO:7) at a ratio of 8:2:1.

The collection of samples, sugars measurements and glucose released calculations were followed as describes in Example 2.

Results are presented in table 4 showing hydrolysis performance of TetraPack with (C+H+S) and without (C+H) amylase, besides the cellulolytic enzymes, at 3 mg-protein/g-substrate, at 5.3% TS, pH 5.0, 50°C for 3 days.

Table 4:

|  | **3 days hydrolysis (g-Glucose/ kg-feedstock)** |
| --- | --- |
| TetraPack - Control | 345 |
| TetraPack - With amylase | 361 |

**Example 5: Mannanase enzyme as boosters for biomass hydrolysis Experimental**

Recycled fiber material (RecycFiber), with some content of mannans, was used as the biomass substrate.

Slurries were prepared with the RecycFiber at 6% total solids (TS) and were re-pulped at pH 7, 50°C and overnight (16 hours) in the Lab-O-Mat (LABOMAT <<BFA-24>>, Werner Mathis U.S.A. Inc., Concord, N.C., USA).

After re-pulping, hydrolysis was performed at 5.3% TS, pH 5.0, 50°C for 3 days, with total 3 mg-protein/g-substrate with 2 different enzymes combinations:

- (Recycled Fibers - Control) cellulolytic enzyme solution (A blend of an *Aspergillus aculeatus* GH10 xylanase (WO 94/21785) and a *Trichoderma reesei* cellulase preparation containing *Aspergillus fumigatus* beta-glucosidase (WO 2005/047499) and *Thermoascus aurantiacus* GH61A polypeptide (WO 2005/074656));

- (Recycled Fibers - With mannanase) cellulolytic enzyme solution (A blend of an *Aspergillus aculeatus* GH10 xylanase (WO 94/21785) and a *Trichoderma reesei* cellulase preparation containing *Aspergillus fumigatus* beta-glucosidase (WO 2005/047499) and *Thermoascus aurantiacus* GH61A polypeptide (WO 2005/074656)), plus *Aspergillus niger* mannanase.

The collection of samples, sugars measurements and glucose released calculations were followed as describes in Example 1.

Results are presented in Table 5 showing hydrolysis performance of recycled fibers with (C+H+M) and without (C+H) mannanase, besides the cellulytic enzymes, at 3 mg-protein/g-substrate, at 5.3% TS, pH 5.0, 50°C for 3 days.

Table 5:

|  | 3 days hydrolysis (g-Glucose/ kg-feedstock) |
| --- | --- |
| Recycled Fibers - Control | 176 |
| Recycled Fibers - With mannanase | 196 |

**Example 6: Mannanase enzyme as boosters for biomass hydrolysis Experimental**

Recycled fiber material (RecycFiber), with some content of mannans, was used as the biomass substrate, which was re-pulped as in example 5.

After re-pulping, hydrolysis was performed at 5.3% TS, pH 5.0, 50°C for 3 days, with total 3 mg-protein/g-substrate with 2 different enzymes combos:

- (Recycled Fibers - Control) cellulolytic enzyme solution (a *Trichoderma reesei* cellulase preparation containing *Aspergillus oryzae* beta-glucosidase fusion protein (WO 2008/057637) and *Thermoascus aurantiacus* GH61A polypeptide (WO 2005/074656) and an *Aspergillus aculeatus* GH10 xylanase (WO 94/21785) preparation at a ratio of 90:10);
- (Recycled Fibers - With mannanase) cellulolytic enzyme solution (a *Trichoderma reesei* cellulase preparation containing *Aspergillus oryzae* beta-glucosidase fusion protein (WO 2008/057637) and *Thermoascus aurantiacus* GH61A polypeptide (WO 2005/074656); an *Aspergillus aculeatus* GH10 xylanase (WO 94/21785) preparation; and mannanase enzyme of SEQ ID NO: 5).

The collection of samples, sugars measurements and glucose released calculations were followed as describes in Example 1.

Results are presented in Table 6 showing hydrolysis performance of recycled fibers with (C+H'+M) and without (C+H') mannanase, besides the cellulytic enzymes, at 3 mg-protein/g-substrate, at 5.3% TS, pH 5.0, 50°C for 3 days.

Table 6:

|  | 3 days hydrolysis (g-Glucose/ kg-feedstock) |
| --- | --- |
| Recycled Fibers - Control | 173 |
| Recycled Fibers - With mannanase | 194 |

**Claims**

1. A method for increasing cellulolytic enzyme activity during the hydrolysis of cellulosic material comprising:

   (a) contacting the cellulosic material with cutinase, peptidase and pectate lyase enzymes to form pretreated cellulosic material; and
   (b) hydrolyzing the pretreated cellulosic material with one or more enzyme compositions comprising one or more cellulolytic enzymes and wherein hydrolyzing step (b) further comprises contacting the pretreated cellulosic material from step (a) with one or more amylases and glucoamylases and/or mannanase enzymes.

2. The method in accordance with claim 1 comprising post-treating the pretreated cellulosic material with an enzymatic pre-treatment, chemical pre-treatment, mechanical pre-treatment and/or a physical pretreatment.

3. A method for hydrolyzing a pretreated cellulosic material comprising saccharifying a cellulosic material with an enzyme composition comprising one or more cellulolytic enzymes and further one or more amylases and glucoamylases and/or mannanase enzymes, wherein the cellulosic material was pretreated by contacting the cellulosic material with cutinase, peptidase and pectate lyase enzymes to form pretreated cellulosic material.

4. The method of claim 3, wherein the enzyme composition comprises one or more (several) enzymes selected from the group consisting of a cellulase, a GH61 polypeptide having cellulolytic enhancing activity, a hemicellulase, an expansin, an esterase, a laccase, a ligninolytic enzyme, a pectinase, a peroxidase, a protease, a swollenin, an amylase and a mannanase.

5. A method for producing a fermentation product, comprising:

   (a) saccharifying a pretreated cellulosic material with an enzyme composition comprising one or more cellulolytic enzymes, and at least one or more amylases and glucoamylases and/or mannanase enzymes;
   (b) fermenting the saccharified pretreated cellulosic material with one or more (several) fermenting microorganisms to produce the fermentation product; and
   (c) recovering the fermentation product from the fermentation, wherein the pretreated cellulosic material was pretreated by contacting cellulosic material with cutinase, peptidase and pectate lyase enzymes.

6. The method of claim 5, wherein the enzyme composition comprises one or more (several) enzymes selected from the group consisting of a cellulase, a GH61 polypeptide having cellulolytic enhancing activity, a hemicellulase, an esterase, an expansin, a laccase, a ligninolytic enzyme, a pectinase, a peroxidase, a protease, and a swollenin.

7. The method of claim 6, wherein the cellulase is one or more (several) enzymes selected from the group consisting of an endoglucanase, a cellobiohydrolase, and a beta-glucosidase.

8. The method of claim 7, wherein the hemicellulase is one or more (several) enzymes selected from the group consisting of a xylanase, an acetyxylan esterase, a feruloyl esterase, an arabinofuranosidase, a xylosidase, and a glucuronidase.

9.  The method of any of claims 5-8, wherein steps (a) and (b) are performed simultaneously in a simultaneous saccharification and fermentation.

10. The method of any of claims 5-9, wherein the fermentation product is an alcohol, an alkane, a cycloalkane, an alkene, an amino acid, a gas, isoprene, a ketone, an organic acid, or polyketide

**Patentansprüche**

1.  Verfahren zum Erhöhen von cellulolytischer Enzymaktivität während der Hydrolyse von cellulosischem Material, umfassend:

    (a) Inkontaktbringen des cellulosischen Materials mit Cutinase, Peptidase und Pectatlyase-Enzymen, um vorbehandeltes cellulosisches Material zu bilden; und
    (b) Hydrolysieren des vorbehandelten cellulosischen Materials mit einer oder mehreren Enzymzusammensetzungen, die ein oder mehrere cellulolytische Enzyme umfassen, und wobei der Hydrolyseschritt (b) des Weiteren Inkontaktbringen des vorbehandelten cellulosischen Materials aus Schritt (a) mit einer oder mehreren Amylasen und Glucoamylasen und/oder Mannanase-Enzymen umfasst.

2.  Verfahren nach Anspruch 1, das Nachbehandeln des vorbehandelten cellulosischen Materials mit einer enzymatischen Vorbehandlung, einer chemischen Vorbehandlung, einer mechanischen Vorbehandlung und/oder einer physikalischen Vorbehandlung umfasst.

3.  Verfahren zum Hydrolysieren eines vorbehandelten cellulosischen Materials, das Verzuckern eines cellulosischen Materials mit einer Enzymzusammensetzung umfasst, die ein oder mehrere cellulolytische Enzyme und des Weiteren eine oder mehrere Amylasen und Glucoamylasen und/oder Mannanase-Enzyme umfasst, wobei das cellulosische Material durch Inkontaktbrigen des cellulosischen Materials mit Cutinase, Peptidase und Pectatlyase-Enzymen vorbehandelt wurde, um vorbehandeltes cellulosisches Material zu bilden.

4.  Verfahren nach Anspruch 3, wobei die Enzymzusammensetzung eines oder mehrere (einige) Enzyme umfasst, die aus der Gruppe bestehend aus einer Cellulase, einem GH61-Polypeptid mit cellulolytisch verstärkender Aktivität, einer Hemicellulase, einem Expansin, einer Esterase, einer Laccase, eienm ligninolytischen Enzym, einer Pectinase, einer Peroxidase, einer Protease, einem Swollenin, einer Amylase und einer Mannanase ausgewählt sind.

5.  Verfahren zum Herstellen eines Fermentationsprodukts, umfassend:

    (a) Verzuckern eines vorbehandelten cellulosischen Materials mit einer Enzymzusammensetzung, die ein oder mehrere cellulolytische Enzyme und mindestens eine oder mehrere Amylasen und Glucoamylasen und/oder Mannanase-Enzyme umfasst;
    (b) Fermentieren des verzuckerten vorbehandelten cellulosischen Materials mit einem oder mehreren (einigen) fermentierenden Mikroorganismen, um das Fermentationsprodukt herzustellen; und
    (c) Gewinnen des Fermentationsprodukts aus der Fermentation, wobei das vorbehandelte cellulosische Material durch Inkontaktbringen von cellulosischem Material mit Cutinase, Peptidase und Pectatlyase-Enzymen vorbehandelt wurde.

6.  Verfahren nach Anspruch 5, wobei die Enzymzusammensetzung ein oder mehrere (einige) Enzyme ausgewählt aus der Gruppe bestehend aus einer Cellulase, einem GH61-Polypeptid mit cellulolytisch verstärkender Aktivität, einer Hemicellulase, einer Esterase, einem Expansin, einer Laccase, einem ligninolytischen Enzym, einer Pectinase, einer Peroxidase, einer Protease und einem Swollenin umfasst.

7.  Verfahren nach Anspruch 6, wobei die Cellulase ein oder mehrere (einige) Enzyme ausgewählt aus der Gruppe bestehend aus einer Endoglucanase, einer Cellobiohydrolase und einer beta-Glucosidase ist.

8.  Verfahren nach Anspruch 7, wobei die Hemicellulase ein oder mehrere (einige) Enzyme ausgewählt aus der Gruppe bestehend aus einer Xylanase, einer Acetylxylanesterase, einer Feruloylesterase, einer Arabinofuranosidase, einer Xylosidase und einer Glucuronidase ist.

9.  Verfahren nach einem beliebigen der Ansprüche 5-8, wobei die Schritte (a) und (b) gleichzeitig in einer simultanen Verzuckerung und Fermentation durchgeführt werden.

**10.** Verfahren nach einem beliebigen der Ansprüche 5-9, wobei das Fermentationsprodukt ein Alkohol, ein Alkan, ein Cycloalkan, ein Alken, eine Aminosäure, ein Gas, Isopren, ein Keton, eine organische Säure oder ein Polyketid ist.

**Revendications**

**1.** Méthode pour accroître l'activité enzymatique cellulolytique pendant l'hydrolyse d'une matière cellulosique comprenant :

(a) la mise en contact de la matière cellulosique avec des enzymes cutinase, peptidase et pectate lyase pour former une matière cellulosique prétraitée ; et
(b) l'hydrolyse de la matière cellulosique prétraitée avec une ou plusieurs compositions enzymatiques comprenant une ou plusieurs enzymes cellulolytiques et dans laquelle l'étape d'hydrolyse (b) comprend en outre la mise en contact de la matière cellulosique prétraitée de l'étape (a) avec une ou plusieurs enzymes amylases et glucoamylases et/ou mannanase.

**2.** Méthode selon la revendication 1 comprenant le post-traitement de la matière cellulosique prétraitée par un prétraitement enzymatique, un prétraitement chimique, un prétraitement mécanique et/ou un prétraitement physique.

**3.** Méthode pour hydrolyser une matière cellulosique prétraitée comprenant la saccharification d'une matière cellulosique avec une composition enzymatique comprenant une ou plusieurs enzymes cellulolytiques et en outre une ou plusieurs enzymes amylases et glucoamylases et/ou mannanase, dans laquelle la matière cellulosique a été prétraitée par mise en contact de la matière cellulosique avec des enzymes cutinase, peptidase et pectate lyase pour former une matière cellulosique prétraitée.

**4.** Méthode selon la revendication 3, dans laquelle la composition enzymatique comprend une enzyme ou plus (plusieurs) choisie(s) dans le groupe constitué par une cellulase, un polypeptide GH61 ayant une activité d'amélioration cellulolytique, une hémicellulase, une expansine, une estérase, une laccase, une enzyme ligninolytique, une pectinase, une peroxydase, une protéase, une swollénine, une amylase et une mannanase.

**5.** Méthode pour produire un produit de fermentation, comprenant :

(a) la saccharification d'une matière cellulosique prétraitée avec une composition enzymatique comprenant une ou plusieurs enzymes cellulolytiques, et au moins une ou plusieurs enzymes amylases et glucoamylases et/ou mannanase ;
(b) la fermentation de la matière cellulosique prétraitée saccharifiée avec un micro-organisme de fermentation ou plus (plusieurs) pour obtenir le produit de fermentation ; et
(c) la récupération du produit de fermentation à partir de la fermentation, dans laquelle la matière cellulosique prétraitée a été prétraitée par mise en contact de la matière cellulosique avec des enzymes cutinase, peptidase et pectate lyase.

**6.** Méthode selon la revendication 5, dans laquelle la composition enzymatique comprend une enzyme ou plus (plusieurs) choisie(s) dans le groupe constitué par une cellulase, un polypeptide GH61 ayant une activité d'amélioration cellulolytique, une hémicellulase, une estérase, une expansine, une laccase, une enzyme ligninolytique, une pectinase, une peroxydase, une protéase, et une swollénine.

**7.** Méthode selon la revendication 6, dans laquelle la cellulase est une enzyme ou plus (plusieurs) choisie(s) dans le groupe constitué par une endoglucanase, une cellobiohydrolase, et une bêta-glucosidase.

**8.** Méthode selon la revendication 7, dans laquelle l'hémicellulase est une enzyme ou plus (plusieurs) choisie(s) dans le groupe constitué par une xylanase, une acétyxylane estérase, une féruloyle estérase, une arabinofuranosidase, une xylosidase, ou une glucuronidase.

**9.** Méthode selon l'une quelconque des revendications 5 à 8, dans laquelle les étapes (a) et (b) sont effectuées simultanément dans une saccharification et fermentation simultanées.

**10.** Méthode selon l'une quelconque des revendications 5 à 9, dans laquelle le produit de fermentation est un alcool, un alcane, un cycloalcane, un alcène, un acide aminé, un gaz, l'isoprène, une cétone, un acide organique, ou un polycétide.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009042622 A
- WO 02095014 A
- WO 2006110891 A
- WO 2006110899 A
- WO 2006110900 A
- WO 2006110901 A
- US 20020164730 A
- US 5827719 A
- WO 9414963 A
- WO 9414964 A
- WO 0005389 A
- US 6960459 B
- US 7833771 B
- EP 238023 A
- EP 305216 A
- WO 9205249 A
- WO 9311254 A
- US 20100034797 A
- US 20090029440 A
- US 20110053822 A
- US 20100279915 A
- WO 9502044 A
- WO 2003048353 A
- WO 8906279 A
- WO 8906270 A
- WO 1998020115 A
- WO 0144452 A
- WO 0158275 A
- WO 0158276 A
- WO 2003006602 A
- WO 2004099401 A
- US 20110028378 A
- US 20100322915 A
- US 20100304433 A
- US 20110158976 A
- US 5972873 A
- US 20070060493 A
- EP 897985 A
- WO 9320193 A
- WO 02092741 A
- WO 03095638 A
- WO 2004092479 A
- JP 11682877 B
- WO 9517413 A
- WO 9522625 A
- US 5223409 A
- WO 9206204 A
- WO 9919467 A
- WO 9623873 A
- WO 9623874 A
- WO 9741213 A
- WO 0060059 A
- WO 0210355 A
- US 6093562 A
- US 6297038 B
- US 6187576 B
- WO 8901969 A
- WO 2004055178 A
- WO 2005003311 A
- US 20050054071 A
- US 60638614 B
- US 11316535 B
- WO 2006069290 A
- EP 140410 B1
- WO 9200381 A
- WO 0004136 A
- WO 0104273 A
- WO 8402921 A
- US 4727026 A
- WO 9928448 A
- US RE32153 E
- US 4587215 A
- EP 135138 A
- WO 8601831 A
- WO 2006069289 A
- US 2007066618 W
- WO 2005045018 A
- US 4598048 A
- US 4604355 A
- US 6162628 A
- US 20110076741 A
- WO 9425576 A
- WO 9324622 A
- JP 03047076 A
- JP 63056289 A
- JP 63036775 A
- JP 08051975 A
- WO 9711164 A
- WO 9118974 A
- WO 99064573 A
- US 7183093 B
- US 6060299 A
- US 6376445 B
- US 5795764 A
- WO 2003062430 A
- WO 9117243 A
- WO 9117244 A
- WO 9105039 A

- WO 9315186 A
- US 5275944 A
- WO 9602551 A
- US 5536655 A
- WO 0070031 A
- WO 05093050 A
- WO 2011059740 A
- WO 2009042871 A
- WO 2010141325 A
- WO 2006074435 A
- WO 2010057086 A
- WO 2005047499 A
- WO 2002095014 A
- WO 2007019442 A
- WO 2010088387 A
- WO 2011035029 A
- WO 2008057637 A
- WO 2012044915 A
- WO 9813465 A
- WO 98015619 A
- WO 98015633 A
- WO 9906574 A
- WO 9910481 A
- WO 99025847 A
- WO 99031255 A
- WO 2002101078 A
- WO 2003027306 A
- WO 2003052054 A
- WO 2003052055 A
- WO 2003052056 A
- WO 2003052057 A
- WO 2003052118 A
- WO 2004016760 A
- WO 2004043980 A
- WO 2004048592 A
- WO 2005001065 A
- WO 2005028636 A
- WO 2005093050 A
- WO 2005093073 A
- WO 2006074005 A
- WO 2006117432 A
- WO 2007071818 A
- WO 2007071820 A
- WO 2008008070 A
- WO 2008008793 A
- US 5457046 A

- US 5648263 A
- US 5686593 A
- WO 2005074647 A
- WO 2008148131 A
- WO 2011035027 A
- WO 2005074656 A
- WO 2010065830 A
- WO 2007089290 A
- WO 2009085935 A
- WO 2009085859 A
- WO 2009085864 A
- WO 2009085868 A
- WO 2010138754 A
- WO 2011005867 A
- WO 2011039319 A
- WO 2011041397 A
- WO 2011041504 A
- WO 2008151043 A
- WO 9421785 A
- WO 2006078256 A
- WO 2011041405 A
- WO 2010126772 A
- WO 2009079210 A
- WO 2011057083 A
- WO 2010108918 A
- WO 2009073709 A
- WO 2005001036 A
- WO 2010014880 A
- WO 2009042846 A
- WO 2009076122 A
- WO 2009127729 A
- WO 2010053838 A
- WO 2010065448 A
- WO 2006114094 A
- WO 2009073383 A
- WO 2010014706 A
- WO 2009068565 A
- WO 9943835 A
- WO 9600787 A
- WO 0056900 A
- WO 9425612 A
- WO 9533836 A
- WO 0024883 A
- WO 94021785 A

**Non-patent literature cited in the description**

- **ZHANG et al.** Outlook for cellulase improvement: Screening and selection strategies. *Biotechnology Advances,* 2006, vol. 24, 452-481
- **GHOSE.** Measurement of cellulase activities. *Pure Appl. Chem.,* 1987, vol. 59, 257-68
- **ZHANG et al.** *Biotechnology Advances,* 2006, vol. 24, 452-481

- **GHOSE.** *Pure and Appl. Chem.,* 1987, vol. 59, 257-268
- **TEERI.** Crystalline cellulose degradation: New insight into the function of cellobiohydrolases. *Trends in Biotechnology,* 1997, vol. 15, 160-167

- **TEERI et al.** Trichoderma reesei cellobiohydrolases: why so efficient on crystalline cellulose?. *Biochem. Soc. Trans.,* 1998, vol. 26, 173-178
- **LEVER et al.** *Anal. Biochem.,* 1972, vol. 47, 273-279
- **VAN TILBEURGH et al.** *FEBS Letters,* 1982, vol. 149, 152-156
- **VAN TILBEURGH ; CLAEYSSENS.** *FEBS Letters,* 1985, vol. 187, 283-288
- **TOMME et al.** *Eur. J. Biochem.,* 1988, vol. 170, 575-581
- **VENTURI et al.** Extracellular beta-D-glucosidase from Chaetomium thermophilum var. coprophilum: production, purification and some biochemical properties. *J. Basic Microbiol.,* 2002, vol. 42, 55-66
- **HENRISSAT.** A classification of glycosyl hydrolases based on amino-acid sequence similarities. *Biochem. J.,* 1991, vol. 280, 309-316
- **HENRISSAT B. ; BAIROCH A.** Updating the sequence-based classification of glycosyl hydrolases. *Biochem. J.,* 1996, vol. 316, 695-696
- **SHALLOM ; SHOHAM.** Microbial hemicellulases. *Current Opinion In Microbiology,* 2003, vol. 6 (3), 219-228
- **GHOSE ; BISARIA.** *Pure & Appl. Chem.,* 1987, vol. 59, 1739-1752
- **BIELY ; PUCHARD.** Recent progress in the assays of xylanolytic enzymes. *Journal of the Science of Food and Agriculture,* 2006, vol. 86 (11), 1636-1647
- **SPANIKOVA ; BIELY.** Glucuronoyl esterase - Novel carbohydrate esterase produced by Schizophyllum commune. *FEBS Letters,* 2006, vol. 580 (19), 4597-4601
- **HERRMANN et al.** The beta-D-xylosidase of Trichoderma reesei is a multifunctional beta-D-xylan xylohydrolase. *Biochemical Journal,* 1997, vol. 321, 375-381
- **BAILEY ; BIELY ; POUTANEN.** Interlaboratory testing of methods for assay of xylanase activity. *Journal of Biotechnology,* 1992, vol. 23 (3), 257-270
- **LEVER.** A new reaction for colorimetric determination of carbohydrates. *Anal. Biochem,* 1972, vol. 47, 273-279
- Enzyme Nomenclature. Academic Press, 1992
- ENZYME NOMENCLATURE. 1993
- ENZYME NOMENCLATURE. 1994
- ENZYME NOMENCLATURE. 1995
- ENZYME NOMENCLATURE. 1997
- *Eur. J. Biochem.,* 1994, vol. 223 (5), 1-5
- *Eur. J. Biochem.,* 1995, vol. 232, 1-6
- *Eur. J. Biochem.,* 1996, vol. 237, 1-5
- *Eur. J. Biochem.,* 1997, vol. 250, 1-6
- *Eur. J. Biochem.,* 1999, vol. 264, 610-650
- **VRIES.** *J. Bacteriol.,* 1998, vol. 180, 243-249
- **WISELOGEL et al.** Handbook on Bioethanol. Taylor & Francis, 1995, 105-118
- **WYMAN.** *Bioresource Technology,* 1994, vol. 50, 3-16
- **LYND.** *Applied Biochemistry and Biotechnology,* 1990, vol. 24/25, 695-719
- Recent Progress in Bioconversion of Lignocellulosics. **MOSIER et al.** Advances in Biochemical Engineering/Biotechnology. Springer-Verlag, 1999, vol. 65, 23-40
- **NIELSEN et al.** *Protein Engineering,* 1997, vol. 10, 1-6
- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **RICE et al.** *EMBOSS: The European Molecular Biology Open Software Suite,* 2000
- Enzyme Nomenclature 1992 from NC-IUBMB. Academic Press, 1992
- *Eur. J. Biochem.,* 1994, vol. 223 (1-5), 1-5
- *Eur. J. Biochem.,* 1997, vol. 250, 1-6
- **JAYANI et al.** Microbial pectinolytic enzymes:A review. *Process Biochemistry,* 2005, vol. 40, 2931-2944
- **MOSIER et al.** *Bioresource Technology,* 2005, vol. 96, 673-686
- **SCHELL et al.** *Appl. Biochem and Biotechn.,* 2003, vol. 105-108, 69-85
- Pretreatment of biomass. **HSU, T.-A.** Handbook on Bioethanol: Production and Utilization. Taylor & Francis, 1996, 179-212
- **GHOSH ; SINGH.** Physicochemical and biological treatments for enzymatic/microbial conversion of lignocellulosic biomass. *Adv. Appl. Microbiol.,* 1993, vol. 39, 295-333
- Pretreating lignocellulosic biomass: a review, in Enzymatic Conversion of Biomass for Fuels Production. **MCMILLAN, J. D.** ACS Symposium Series 566. American Chemical Society, 1994
- Ethanol production from renewable resources. **GONG, C. S. ; CAO, N. J. ; DU, J. ; TSAO, G. T.** Advances in Biochemical Engineering/Biotechnology. Springer-Verlag Berlin Heidelberg, 1999, vol. 65, 207-241
- **OLSSON ; HAHN-HAGERDAL.** Fermentation of lignocellulosic hydrolysates for ethanol production. *Enz. Microb. Tech.,* 1996, vol. 18, 312-331
- **VALLANDER ; ERIKSSON.** Production of ethanol from lignocellulosic materials: State of the art. *Adv. Biochem. Eng./Biotechnol.,* 1990, vol. 42, 63-95
- *Eur. J. Biochem.,* 1994, vol. 223, 1-5
- **P.E. KOLATTUKUDY.** Lipases. Elsevier, 1984, 471-504

- **LONGHI et al.** *Journal of Molecular Biology,* 1997, vol. 268 (4), 779-799
- *Appl. Environm. Microbiol.,* 1998, vol. 64, 2794-2799
- *Proteins: Structure, Function and Genetics,* 1996, vol. 26, 442-458
- *J. of Computational Chemistry,* 1996, vol. 17, 1783-1803
- *Protein Engineering,* 1993, vol. 6, 157-165
- *Proteins: Structure, Function, and Genetics,* 1998, vol. 33, 253-264
- *J. of Biotechnology,* 1998, vol. 66, 11-26
- *Biochemistry,* 1996, vol. 35, 398-410
- *Chemistry and Physics of Lipids,* 1999, vol. 97, 181-191
- *Proteins: Structure, Function, and Genetics,* 1998, vol. 31, 320-333
- *Biochimica et Biophysica Acta,* 1999, vol. 1441, 185-196
- *Appl. Environm. Microbiol.,* 1998, vol. 64, 316-324
- *BioTechniques,* 1999, vol. 27, 1102-1108
- **KOAZE et al.** *Agr. Biol. Chem. Japan,* 1964, vol. 28, 216
- **YOSHIDA.** *J. Agr. Chem. Soc. Japan,* 1954, vol. 28, 66
- **HAYASHIDA et al.** *Agric. Biol. Chem.,* 1977, vol. 42 (5), 927-933
- Hand-book of Proteolytic Enzymes. Aca-demic Press, 1998
- **BERKA et al.** *Gene,* 1990, vol. 96, 313
- **BERKA et al.** *Gene,* 1993, vol. 125, 195-198
- **GOMI et al.** *Biosci. Biotech. Biochem.,* 1993, vol. 57, 1095-1100
- **SAWADA et al.** *Experientia,* 1983, vol. 39, 377
- **H. NEURATH ; R.L. HILL.** In, The Proteins. Academic Press, 1979
- **CUNNINGHAM ; WELLS.** *Science,* 1989, vol. 244, 1081-1085
- **HILTON et al.** *J. Biol. Chem.,* 1996, vol. 271, 4699-4708
- **VOS et al.** *Science,* 1992, vol. 255, 306-312
- **SMITH.** *J. Mol. Biol.,* 1992, vol. 224, 899-904
- **WLODAVER et al.** *FEBS Lett.,* 1992, vol. 309, 59-64
- **REIDHAAR-OLSON ; SAUER.** *Science,* 1988, vol. 241, 53-57
- **BOWIE ; SAUER.** *Proc. Natl. Acad. Sci. USA,* 1989, vol. 86, 2152-2156
- **LOWMAN et al.** *Biochemistry,* 1991, vol. 30, 10832-10837
- **DERBYSHIRE et al.** *Gene,* 1986, vol. 46, 145
- **NER et al.** *DNA,* 1988, vol. 7, 127
- **NESS et al.** *Nature Biotechnology,* 1999, vol. 17, 893-896
- **KANEKO et al.** *J. Ferment. Bioeng.,* 1996, vol. 81, 292-298
- **BOEL.** *EMBO J.,* 1984, vol. 3 (5), 1097-1102
- *Agric. Biol. Chem.,* 1991, vol. 55 (4), 941-949
- **CHEN.** *Prot. Eng.,* 1996, vol. 9, 499-505
- **CHEN et al.** *Prot. Eng.,* 1995, vol. 8, 575-582
- **CHEN et al.** *Biochem. J.,* 1994, vol. 301, 275-281
- **FIEROBE et al.** *Biochemistry,* 1996, vol. 35, 8698-8704
- **LI et al.** *Protein Eng.,* 1997, vol. 10, 1199-1204
- **NAGASAKA,Y. et al.** Purification and properties of the raw-starch-degrading glucoamylases from Corticium rolfsii. *Appl. Microbiol. Biotechnol.,* 1998, vol. 50, 323-330
- **FOGARTY ; KELLY.** *Progress in Industrial Microbiology,* 1979, vol. 15, 112-115
- **TALBOT et al.** *Appl. Environ. Microbiol.,* 1990, vol. 56 (11), 3505-3510
- **MENDOZA et al.** *World J. Microbiol. Biotech.,* 1994, vol. 10 (5), 551-555
- **LIN et al.** *Appl. Microbiol. Biotechnol.,* 2006, vol. 69, 627-642
- **CHEN ; HO.** Cloning and improving the expression of Pichia stipitis xylose reductase gene in Saccharomyces cerevisiae. *Appl. Biochem. Biotechnol.,* 1993, vol. 39-40, 135-147
- **HO et al.** Genetically engineered Saccharomyces yeast capable of effectively cofermenting glucose and xylose. *Appl. Environ. Microbiol.,* 1998, vol. 64, 1852-1859
- **KOTTER ; CIRIACY.** Xylose fermentation by Saccharomyces cerevisiae. *Appl. Microbiol. Biotechnol.,* 1993, vol. 38, 776-783
- **WALFRIDSSON et al.** Xylose-metabolizing Saccharomyces cerevisiae strains overexpressing the TKL1 and TAL1 genes encoding the pentose phosphate pathway enzymes transketolase and transaldolase. *Appl. Environ. Microbiol.,* 1995, vol. 61, 4184-4190
- **KUYPER et al.** Minimal metabolic engineering of Saccharomyces cerevisiae for efficient anaerobic xylose fermentation: a proof of principle. *FEMS Yeast Research,* 2004, vol. 4, 655-664
- **BEALL et al.** Parametric studies of ethanol production from xylose and other sugars by recombinant Escherichia coli. *Biotech. Bioeng.,* 1991, vol. 38, 296-303
- **INGRAM et al.** Metabolic engineering of bacteria for ethanol production. *Biotechnol. Bioeng.,* 1998, vol. 58, 204-214
- **ZHANG et al.** Metabolic engineering of a pentose metabolism pathway in ethanologenic Zymomonas mobilis. *Science,* 1995, vol. 267, 240-243
- **DEANDA et al.** Development of an arabinose-fermenting Zymomonas mobilis strain by metabolic pathway engineering. *Appl. Environ. Microbiol.,* 1996, vol. 62, 4465-4470
- The Alcohol Textbook. Nottingham University Press, 1999

- **ALFENORE et al.** Improving ethanol production and viability of Saccharomyces cerevisiae by a vitamin feeding strategy during fed-batch process. Springer-Verlag, 2002
- Ethanol production from renewable resources. **GONG, C. S. ; CAO, N. J. ; DU, J. ; TSAO, G. T.** Advances in Biochemical Engineering/Biotechnology. Springer-Verlag, 1999, vol. 65, 207-241
- **SILVEIRA ; JONAS.** The biotechnological production of sorbitol. *Appl. Microbiol. Biotechnol.,* 2002, vol. 59, 400-408
- **NIGAM, P. ; SINGH, D.** Processes for fermentative production of xylitol - a sugar substitute. *Process Biochemistry,* 1995, vol. 30 (2), 117-124
- **EZEJI et al.** Production of acetone, butanol and ethanol by Clostridium beijerinckii BA101 and in situ recovery by gas stripping. *World Journal of Microbiology and Biotechnology,* 2003, vol. 19 (6), 595-603
- **RICHARD ; MARGARITIS.** Empirical modeling of batch fermentation kinetics for poly(glutamic acid) production and other microbial biopolymers. *Biotechnology and Bioengineering,* 2004, vol. 87 (4), 501-515
- **KATAOKA et al.** Studies on hydrogen production by continuous culture system of hydrogen-producing anaerobic bacteria. *Water Science and Technology,* 1997, vol. 36 (6-7), 41-47
- **GUNASEELAN.** *Biomass and Bioenergy,* 1997, vol. 3 (1-2), 83-114
- **CHEN, R. ; LEE, Y. Y.** Membrane-mediated extractive fermentation for lactic acid production from cellulosic biomass. *Appl. Biochem. Biotechnol.,* 1997, vol. 63-65, 435-448
- **PENTTILA et al.** *Gene,* 1986, vol. 45, 253-263
- **SALOHEIMO et al.** *Gene,* 1988, vol. 63, 11-22
- **OKADA et al.** *Appl. Environ. Microbiol.,* 1988, vol. 64, 555-563
- **SALOHEIMO et al.** *Molecular Microbiology,* 1994, vol. 13, 219-228
- **OOI et al.** *Nucleic Acids Research,* 1990, vol. 18, 5884
- **SAKAMOTO et al.** *Current Genetics,* 1995, vol. 27, 435-439
- **SAARILAHTI et al.** *Gene,* 1990, vol. 90, 9-14
- **KAWAGUCHI et al.** *Gene,* 1996, vol. 173, 287-288
- **DAN et al.** *J. Biol. Chem.,* 2000, vol. 275, 4973-4980
- **HENRISSAT B.** A classification of glycosyl hydrolases based on amino-acid sequence similarities. *Biochem. J.,* 1991, vol. 280, 309-316
- **HENRISSAT ; BAIROCH.** Updating the sequence-based classification of glycosyl hydrolases. *Biochem. J.,* 1996, vol. 316, 695-696
- More Gene Manipulations in Fungi. Academic Press, 1991
- **BAILEY, J.E. ; OLLIS, D.F.** Biochemical Engineering Fundamentals. McGraw-Hill Book Company, 1986
- **AGAISSE ; LERECLUS.** *Molecular Microbiology,* 1994, vol. 13, 97-107
- **EGON et al.** *Gene,* 1988, vol. 69, 301-315
- **VILLA-KAMAROFF et al.** *Proc. Natl. Acad. Sci. USA,* 1978, vol. 75, 3727-3731
- **DEBOER et al.** *Proc. Natl. Acad. Sci. USA,* 1983, vol. 80, 21-25
- **GILBERT et al.** Useful proteins from recombinant bacteria. *Scientific American,* 1980, vol. 242, 74-94
- **ROMANOS et al.** *Yeast,* 1992, vol. 8, 423-488
- **HUE et al.** *Journal of Bacteriology,* 1995, vol. 177, 3465-3471
- **GUO ; SHERMAN.** *Mol. Cellular Biol.,* 1995, vol. 15, 5983-5990
- **SIMONEN ; PALVA.** *Microbiological Reviews,* 1993, vol. 57, 109-137
- **GEMS et al.** *Gene,* 1991, vol. 98, 61-67
- **CULLEN et al.** *Nucleic Acids Res.,* 1987, vol. 15, 9163-9175
- **CHANG ; COHEN.** *Mol. Gen. Genet.,* 1979, vol. 168, 111-115
- **YOUNG ; SPIZIZEN.** *J. Bacteriol.,* 1961, vol. 81, 823-829
- **DUBNAU ; DAVIDOFF-ABELSON.** *J. Mol. Biol.,* 1971, vol. 56, 209-221
- **SHIGEKAWA ; DOWER.** *Biotechniques,* 1988, vol. 6, 742-751
- **KOEHLER ; THORNE.** *J. Bacteriol.,* 1987, vol. 169, 5271-5278
- **HANAHAN.** *J. Mol. Biol.,* 1983, vol. 166, 557-580
- **DOWER et al.** *Nucleic Acids Res.,* 1988, vol. 16, 6127-6145
- **GONG et al.** *Folia Microbiol. (Praha),* 2004, vol. 49, 399-405
- **MAZODIER et al.** *J. Bacteriol.,* 1989, vol. 171, 3583-3585
- **BURKE et al.** *Proc. Natl. Acad. Sci. USA,* 2001, vol. 98, 6289-6294
- **CHOI et al.** *J. Microbiol. Methods,* 2006, vol. 64, 391-397
- **PINEDO ; SMETS.** *Appl. Environ. Microbiol.,* 2005, vol. 71, 51-57
- **PERRY ; KURAMITSU.** *Infect. Immun.,* 1981, vol. 32, 1295-1297
- **CATT ; JOLLICK.** *Microbios,* 1991, vol. 68, 189-207
- **BUCKLEY et al.** *Appl. Environ. Microbiol.,* 1999, vol. 65, 3800-3804
- **CLEWELL.** *Microbiol. Rev.,* 1981, vol. 45, 409-436
- **HAWKSWORTH et al.** Ainsworth and Bisby's Dictionary of The Fungi. CAB International, University Press, 1995

- Biology and Activities of Yeast. Soc. App. Bacteriol. Symposium Series No. 9. 1980
- **YELTON et al.** *Proc. Natl. Acad. Sci. USA,* 1984, vol. 81, 1470-1474
- **CHRISTENSEN et al.** *Bio/Technology,* 1988, vol. 6, 1419-1422
- **MALARDIER et al.** *Gene,* 1989, vol. 78, 147-156
- Guide to Yeast Genetics and Molecular Biology. **BECKER ; GUARENTE.** Methods in Enzymology. Academic Press, Inc, vol. 194, 182-187
- **ITO et al.** *J. Bacteriol.,* 1983, vol. 153, 163
- **HINNEN et al.** *Proc. Natl. Acad. Sci. USA,* 1978, vol. 75, 1920

SEQUENCE LISTING

<110> Novozymes North America, Inc. Gaspar, Armindo Ribiero Xu, Hui Croonenberghs, James Luo, James Rane, Kishore

<120> Processes For Pretreating Cellulosic Material And Improving Hydrolysis Thereof

<130> 12045-WO-PCT

<160> 7

<170> PatentIn version 3.5

<210> 1
<211> 194
<212> PRT
<213> Humicola insolens cutinase

<400> 1

```
Gln Leu Gly Ala Ile Gln Asn Asp Leu Glu Ser Gly Ser Pro Asp Ala
1               5                   10                  15

Cys Pro Asp Ala Ile Leu Ile Phe Ala Arg Gly Ser Thr Glu Pro Gly
                20                  25                  30

Asn Met Gly Ile Thr Val Gly Pro Ala Leu Ala Asn Gly Leu Lys Glu
            35                  40                  45

His Ile Pro Asn Ile Trp Ile Gln Gly Val Gly Gly Pro Tyr Asp Ala
            50                  55                  60

Ala Leu Ala Thr Asn Phe Leu Pro Arg Gly Thr Ser Gln Ala Asn Ile
65                  70                  75                  80

Asp Glu Gly Lys Arg Leu Phe His Leu Ala His Gln Lys Cys Pro Asn
                85                  90                  95

Thr Pro Val Val Ala Gly Gly Tyr Ser Gln Gly Ala Ala Leu Ile Ala
                100                 105                 110

Ala Ala Val Ser Glu Leu Ser Gly Ala Val Lys Glu Gln Val Lys Gly
            115                 120                 125

Val Val Leu Phe Gly Tyr Thr Gln Asn Leu Gln Asn Arg Gly Gly Ile
    130                 135                 140

Pro Asn Tyr Pro Arg Glu Arg Thr Lys Val Phe Cys Asn Val Gly Asp
145                 150                 155                 160

Ala Val Cys Thr Gly Thr Leu Ile Ile Thr Pro Ala His Leu Ser Tyr
                165                 170                 175

Thr Ile Gln Ala Arg Gly Glu Ala Ala Arg Phe Leu Val Asp Arg Ile
            180                 185                 190

Arg Ala
```

<210> 2
<211> 270
<212> PRT
<213> Mature savinase bacillus lentus

<400> 2

```
Ala Gln Ser Val Pro Trp Gly Ile Ser Arg Val Gln Ala Pro Ala Ala
1               5                   10                  15

His Asn Arg Gly Leu Thr Gly Ser Gly Val Lys Val Ala Val Leu Asp
            20                  25                  30

Thr Gly Ile Ser Thr His Pro Asp Leu Asn Ile Arg Gly Gly Ala Ser
            35                  40                  45

Phe Val Pro Gly Glu Pro Ser Thr Gln Asp Gly Asn Gly His Gly Thr
        50                  55                  60

His Val Ala Gly Thr Ile Ala Ala Leu Asn Asn Ser Ile Gly Val Leu
65              70                  75                  80

Gly Val Ala Pro Ser Ala Glu Leu Tyr Ala Val Lys Val Leu Gly Ala
                85                  90                  95

Ser Glu Gly Ser Gly Ser Val Ser Ser Ile Ala Gln Gly Leu Glu Trp
            100                 105                 110

Ala Gly Asn Asn Gly Met His Val Ala Asn Leu Ser Leu Gly Ser Pro
            115                 120                 125

Ser Pro Ser Ala Thr Leu Glu Gln Ala Val Asn Ser Ala Thr Ser Arg
    130                 135                 140

Gly Val Leu Val Val Ala Ala Ser Gly Asn Ser Gly Ala Gly Ser Ile
145                 150                 155                 160

Ser Tyr Pro Ala Arg Tyr Ala Asn Ala Met Ala Val Gly Ala Thr Asp
                165                 170                 175

Gln Asn Asn Asn Arg Ala Ser Phe Ser Gln Tyr Gly Ala Gly Leu Asp
                180                 185                 190
```

```
            Ile Val Ala Pro Gly Val Asn Val Gln Ser Thr Tyr Pro Gly Ser Thr
                    195             200             205

            Tyr Ala Ser Leu Asn Gly Thr Ser Met Ala Thr Pro His Val Ala Gly
                    210             215             220

            Ala Ala Ala Leu Val Lys Gln Lys Asn Pro Ser Trp Ser Asn Val Gln
            225             230             235             240

            Ile Arg Asn His Leu Lys Asn Thr Ala Thr Ser Leu Gly Ser Thr Asn
                    245             250             255

            Leu Tyr Gly Ser Gly Leu Val Asn Ala Glu Ala Ala Thr Arg
                    260             265             270
```

<210> 3
<211> 385
<212> PRT
<213> Aspergillus aculeatus

<400> 3

```
            Lys Thr Ser Met His Leu Asn Thr Thr Leu Leu Val Ser Leu Ala Leu
            1               5               10              15

            Gly Ala Ala Ser Val Leu Ala Ser Pro Ala Pro Pro Ala Ile Thr Ala
                    20              25              30

            Pro Pro Thr Ala Glu Glu Ile Ala Lys Arg Ala Thr Thr Cys Thr Phe
                    35              40              45

            Ser Gly Ser Asn Gly Ala Ser Ser Ala Ser Lys Ser Lys Thr Ser Cys
                    50              55              60

            Ser Thr Ile Val Leu Ser Asn Val Ala Val Pro Ser Gly Thr Thr Leu
            65              70              75              80

            Asp Leu Thr Lys Leu Asn Asp Gly Thr His Val Ile Phe Ser Gly Glu
                    85              90              95

            Thr Thr Phe Gly Tyr Lys Glu Trp Ser Gly Pro Leu Ile Ser Val Ser
                    100             105             110

            Gly Ser Asp Leu Thr Ile Thr Gly Ala Ser Gly His Ser Ile Asn Gly
                    115             120             125

            Asp Gly Ser Arg Trp Trp Asp Gly Glu Gly Gly Asn Gly Gly Lys Thr
                    130             135             140
```

```
Lys Pro Lys Phe Phe Ala Ala His Ser Leu Thr Asn Ser Val Ile Ser
145             150             155             160

Gly Leu Lys Ile Val Asn Ser Pro Val Gln Val Phe Ser Val Ala Gly
                165             170             175

Ser Asp Tyr Leu Thr Leu Lys Asp Ile Thr Ile Asp Asn Ser Asp Gly
            180             185             190

Asp Asp Asn Gly Gly His Asn Thr Asp Ala Phe Asp Ile Gly Thr Ser
            195             200             205

Thr Tyr Val Thr Ile Ser Gly Ala Thr Val Tyr Asn Gln Asp Asp Cys
    210             215             220

Val Ala Val Asn Ser Gly Glu Asn Ile Tyr Phe Ser Gly Gly Tyr Cys
225             230             235             240

Ser Gly Gly His Gly Leu Ser Ile Gly Ser Val Gly Gly Arg Ser Asp
            245             250             255

Asn Thr Val Lys Asn Val Thr Phe Val Asp Ser Thr Ile Ile Asn Ser
            260             265             270

Asp Asn Gly Val Arg Ile Lys Thr Asn Ile Asp Thr Thr Gly Ser Val
        275             280             285

Ser Asp Val Thr Tyr Lys Asp Ile Thr Leu Thr Ser Ile Ala Lys Tyr
    290             295             300

Gly Ile Val Val Gln Gln Asn Tyr Gly Asp Thr Ser Ser Thr Pro Thr
305             310             315             320

Thr Gly Val Pro Ile Thr Asp Phe Val Leu Asp Asn Val His Gly Ser
            325             330             335

Val Val Ser Ser Gly Thr Asn Ile Leu Ile Ser Cys Gly Ser Gly Ser
        340             345             350

Cys Ser Asp Trp Thr Trp Thr Asp Val Ser Val Ser Gly Gly Lys Thr
    355             360             365

Ser Ser Lys Cys Thr Asn Val Pro Ser Gly Ala Ser Cys Asn Gly Ala
    370             375             380

Ser
385
```

<210> 4
<211> 471
<212> PRT
<213> Rhizomucor pusillus amylase

<400> 4

```
Met Lys Phe Ser Ile Ser Leu Ser Ala Ala Ile Val Leu Phe Ala Ala
1               5               10              15

Ala Thr Ser Leu Ala Ser Pro Leu Pro Gln Gln Gln Arg Tyr Gly Lys
            20              25              30

Arg Ala Thr Ser Asp Asp Trp Lys Ser Lys Ala Ile Tyr Gln Leu Leu
        35              40              45

Thr Asp Arg Phe Gly Arg Ala Asp Asp Ser Thr Ser Asn Cys Ser Asn
    50              55              60

Leu Ser Asn Tyr Cys Gly Gly Thr Tyr Glu Gly Ile Thr Lys His Leu
65              70              75              80

Asp Tyr Ile Ser Gly Met Gly Phe Asp Ala Ile Trp Ile Ser Pro Ile
            85              90              95

Pro Lys Asn Ser Asp Gly Gly Tyr His Gly Tyr Trp Ala Thr Asp Phe
            100             105             110

Tyr Gln Leu Asn Ser Asn Phe Gly Asp Glu Ser Gln Leu Lys Ala Leu
        115             120             125

Ile Gln Ala Ala His Glu Arg Asp Met Tyr Val Met Leu Asp Val Val
    130             135             140

Ala Asn His Ala Gly Pro Thr Ser Asn Gly Tyr Ser Gly Tyr Thr Phe
145             150             155             160

Gly Asp Ala Ser Leu Tyr His Pro Lys Cys Thr Ile Asp Tyr Asn Asp
            165             170             175

Gln Thr Ser Ile Glu Gln Cys Trp Val Ala Asp Glu Leu Pro Asp Ile
        180             185             190

Asp Thr Glu Asn Ser Asp Asn Val Ala Ile Leu Asn Asp Ile Val Ser
        195             200             205

Gly Trp Val Gly Asn Tyr Ser Phe Asp Gly Ile Arg Ile Asp Thr Val
        210             215             220

Lys His Ile Arg Lys Asp Phe Trp Thr Gly Tyr Ala Glu Ala Ala Gly
225             230             235             240
```

```
Val Phe Ala Thr Gly Glu Val Phe Asn Gly Asp Pro Ala Tyr Val Gly
            245                 250                 255

Pro Tyr Gln Lys Tyr Leu Pro Ser Leu Ile Asn Tyr Pro Met Tyr Tyr
            260                 265                 270

Ala Leu Asn Asp Val Phe Val Ser Lys Ser Lys Gly Phe Ser Arg Ile
            275                 280                 285

Ser Glu Met Leu Gly Ser Asn Arg Asn Ala Phe Glu Asp Thr Ser Val
    290                 295                 300

Leu Thr Thr Phe Val Asp Asn His Asp Asn Pro Arg Phe Leu Asn Ser
305                 310                 315                 320

Gln Ser Asp Lys Ala Leu Phe Lys Asn Ala Leu Thr Tyr Val Leu Leu
            325                 330                 335

Gly Glu Gly Ile Pro Ile Val Tyr Tyr Gly Ser Glu Gln Gly Phe Ser
            340                 345                 350

Gly Gly Ala Asp Pro Ala Asn Arg Glu Val Leu Trp Thr Thr Asn Tyr
            355                 360                 365

Asp Thr Ser Ser Asp Leu Tyr Gln Phe Ile Lys Thr Val Asn Ser Val
    370                 375                 380

Arg Met Lys Ser Asn Lys Ala Val Tyr Met Asp Ile Tyr Val Gly Asp
385                 390                 395                 400

Asn Ala Tyr Ala Phe Lys His Gly Asp Ala Leu Val Val Leu Asn Asn
            405                 410                 415

Tyr Gly Ser Gly Ser Thr Asn Gln Val Ser Phe Ser Val Ser Gly Lys
            420                 425                 430

Phe Asp Ser Gly Ala Ser Leu Met Asp Ile Val Ser Asn Ile Thr Thr
            435                 440                 445

Thr Val Ser Ser Asp Gly Thr Val Thr Phe Asn Leu Lys Asp Gly Leu
    450                 455                 460

Pro Ala Ile Phe Thr Ser Ala
465                 470
```

<210> 5
<211> 383

<212> PRT
<213> Aspergillus niger mannanase

<400> 5

```
Met Lys Leu Ser Asn Ala Leu Leu Thr Leu Ala Ser Leu Ala Leu Ala
1               5                   10                  15

Asn Val Ser Thr Ala Leu Pro Lys Ala Ser Pro Ala Pro Ser Thr Ser
        20                  25                  30

Ser Ser Ala Ala Ser Thr Ser Phe Ala Ser Thr Ser Gly Leu Gln Phe
        35                  40                  45

Thr Ile Asp Gly Glu Thr Gly Tyr Phe Ala Gly Thr Asn Ser Tyr Trp
        50                  55                  60

Ile Gly Phe Leu Thr Asp Asn Ser Asp Val Asp Leu Val Met Ser His
65                  70                  75                  80

Leu Lys Ser Ser Gly Leu Lys Ile Leu Arg Val Trp Gly Phe Asn Asp
                85                  90                  95

Val Thr Ser Gln Pro Ser Ser Gly Thr Val Trp Tyr Gln Leu His Gln
            100                 105                 110

Asp Gly Lys Ser Thr Ile Asn Thr Gly Ala Asp Gly Leu Gln Arg Leu
            115                 120                 125

Asp Tyr Val Val Ser Ser Ala Glu Gln His Asp Ile Lys Leu Ile Ile
        130                 135                 140

Asn Phe Val Asn Tyr Trp Thr Asp Tyr Gly Gly Met Ser Ala Tyr Val
145                 150                 155                 160

Ser Ala Tyr Gly Gly Ser Asp Glu Thr Asp Phe Tyr Thr Ser Asp Thr
                165                 170                 175

Ile Gln Ser Ala Tyr Gln Thr Tyr Ile Lys Thr Val Val Glu Arg Tyr
            180                 185                 190

Ser Asn Ser Ser Ala Val Phe Ala Trp Glu Leu Ala Asn Glu Pro Arg
            195                 200                 205

Cys Pro Ser Cys Asp Thr Ser Val Leu Tyr Asn Trp Ile Glu Lys Thr
    210                 215                 220

Ser Lys Phe Ile Lys Gly Leu Asp Ala Asp His Met Val Cys Ile Gly
225                 230                 235                 240

Asp Glu Gly Phe Gly Leu Asn Ile Asp Ser Asp Gly Ser Tyr Pro Tyr
                245                 250                 255
```

50

EP 2 734 633 B1

```
            Gln Phe Ser Glu Gly Leu Asn Phe Thr Met Asn Leu Gly Ile Asp Thr
                        260             265             270

            Ile Asp Phe Gly Thr Leu His Leu Tyr Pro Asp Ser Trp Gly Thr Ser
                        275             280             285

            Asp Asp Trp Gly Asn Gly Trp Ile Thr Ala His Gly Ala Ala Cys Lys
                        290             295             300

            Ala Ala Gly Lys Pro Cys Leu Leu Glu Glu Tyr Gly Val Thr Ser Asn
            305             310             315             320

            His Cys Ser Val Glu Ser Pro Trp Gln Lys Thr Ala Leu Asn Thr Thr
                        325             330             335

            Gly Val Gly Ala Asp Leu Phe Trp Gln Tyr Gly Asp Asp Leu Ser Thr
                        340             345             350

            Gly Lys Ser Pro Asp Asp Gly Asn Thr Ile Tyr Tyr Gly Thr Ser Asp
                        355             360             365

            Tyr Glu Cys Leu Val Thr Asp His Val Ala Ala Ile Gly Ser Ala
                370             375             380
```

<210> 6
<211> 591
<212> PRT
<213> Talaromices emersoni glucoamylase

<400> 6

```
            Ala Thr Gly Ser Leu Asp Ser Phe Leu Ala Thr Glu Thr Pro Ile Ala
            1               5               10              15

            Leu Gln Gly Val Leu Asn Asn Ile Gly Pro Asn Gly Ala Asp Val Ala
                        20              25              30

            Gly Ala Ser Ala Gly Ile Val Val Ala Ser Pro Ser Arg Ser Asp Pro
                        35              40              45

            Asn Tyr Phe Tyr Ser Trp Thr Arg Asp Ala Ala Leu Thr Ala Lys Tyr
                50              55              60

            Leu Val Asp Ala Phe Asn Arg Gly Asn Lys Asp Leu Glu Gln Thr Ile
            65              70              75              80

            Gln Gln Tyr Ile Ser Ala Gln Ala Lys Val Gln Thr Ile Ser Asn Pro
                        85              90              95
```

51

```
Ser Gly Asp Leu Ser Thr Gly Gly Leu Gly Glu Pro Lys Phe Asn Val
            100                 105                 110

Asn Glu Thr Ala Phe Thr Gly Pro Trp Gly Arg Pro Gln Arg Asp Gly
            115                 120                 125

Pro Ala Leu Arg Ala Thr Ala Leu Ile Ala Tyr Ala Asn Tyr Leu Ile
    130                 135                 140

Asp Asn Gly Glu Ala Ser Thr Ala Asp Glu Ile Ile Trp Pro Ile Val
145                 150                 155                 160

Gln Asn Asp Leu Ser Tyr Ile Thr Gln Tyr Trp Asn Ser Ser Thr Phe
                165                 170                 175

Asp Leu Trp Glu Glu Val Glu Gly Ser Ser Phe Phe Thr Thr Ala Val
            180                 185                 190

Gln His Arg Ala Leu Val Glu Gly Asn Ala Leu Ala Thr Arg Leu Asn
            195                 200                 205

His Thr Cys Ser Asn Cys Val Ser Gln Ala Pro Gln Val Leu Cys Phe
    210                 215                 220

Leu Gln Ser Tyr Trp Thr Gly Ser Tyr Val Leu Ala Asn Phe Gly Gly
225                 230                 235                 240

Ser Gly Arg Ser Gly Lys Asp Val Asn Ser Ile Leu Gly Ser Ile His
            245                 250                 255

Thr Phe Asp Pro Ala Gly Gly Cys Asp Asp Ser Thr Phe Gln Pro Cys
            260                 265                 270

Ser Ala Arg Ala Leu Ala Asn His Lys Val Val Thr Asp Ser Phe Arg
            275                 280                 285

Ser Ile Tyr Ala Ile Asn Ser Gly Ile Ala Glu Gly Ser Ala Val Ala
    290                 295                 300

Val Gly Arg Tyr Pro Glu Asp Val Tyr Gln Gly Gly Asn Pro Trp Tyr
305                 310                 315                 320

Leu Ala Thr Ala Ala Ala Ala Glu Gln Leu Tyr Asp Ala Ile Tyr Gln
            325                 330                 335

Trp Lys Lys Ile Gly Ser Ile Ser Ile Thr Asp Val Ser Leu Pro Phe
            340                 345                 350

Phe Gln Asp Ile Tyr Pro Ser Ala Ala Val Gly Thr Tyr Asn Ser Gly
```

52

355          360          365

Ser Thr Thr Phe Asn Asp Ile Ile Ser Ala Val Gln Thr Tyr Gly Asp
   370             375            380

Gly Tyr Leu Ser Ile Val Glu Lys Tyr Thr Pro Ser Asp Gly Ser Leu
   385             390            395            400

Thr Glu Gln Phe Ser Arg Thr Asp Gly Thr Pro Leu Ser Ala Ser Ala
            405            410            415

Leu Thr Trp Ser Tyr Ala Ser Leu Leu Thr Ala Ser Ala Arg Arg Gln
            420            425            430

Ser Val Val Pro Ala Ser Trp Gly Glu Ser Ser Ala Ser Ser Val Leu
            435            440            445

Ala Val Cys Ser Ala Thr Ser Ala Thr Gly Pro Tyr Ser Thr Ala Thr
   450             455            460

Asn Thr Val Trp Pro Ser Ser Gly Ser Gly Ser Ser Thr Thr Thr Ser
465             470            475            480

Ser Ala Pro Cys Thr Thr Pro Thr Ser Val Ala Val Thr Phe Asp Glu
            485            490            495

Ile Val Ser Thr Ser Tyr Gly Glu Thr Ile Tyr Leu Ala Gly Ser Ile
            500            505            510

Pro Glu Leu Gly Asn Trp Ser Thr Ala Ser Ala Ile Pro Leu Arg Ala
            515            520            525

Asp Ala Tyr Thr Asn Ser Asn Pro Leu Trp Tyr Val Thr Val Asn Leu
   530             535            540

Pro Pro Gly Thr Ser Phe Glu Tyr Lys Phe Phe Lys Asn Gln Thr Asp
545             550            555            560

Gly Thr Ile Val Trp Glu Asp Asp Pro Asn Arg Ser Tyr Thr Val Pro
            565            570            575

Ala Tyr Cys Gly Gln Thr Thr Ala Ile Leu Asp Asp Ser Trp Gln
            580            585            590

<210> 7
<211> 574
<212> PRT

<213> Trametes cingulata glucoamylase

<400> 7

```
Met Arg Phe Thr Leu Leu Thr Ser Leu Leu Gly Leu Ala Leu Gly Ala
1           5               10              15

Phe Ala Gln Ser Ser Ala Ala Asp Ala Tyr Val Ala Ser Glu Ser Pro
        20              25              30

Ile Ala Lys Ala Gly Val Leu Ala Asn Ile Gly Pro Ser Gly Ser Lys
        35              40              45

Ser Asn Gly Ala Lys Ala Gly Ile Val Ile Ala Ser Pro Ser Thr Ser
    50              55              60

Asn Pro Asn Tyr Leu Tyr Thr Trp Thr Arg Asp Ser Ser Leu Val Phe
65              70              75              80

Lys Ala Leu Ile Asp Gln Phe Thr Thr Gly Glu Asp Thr Ser Leu Arg
            85              90              95

Thr Leu Ile Asp Glu Phe Thr Ser Ala Glu Ala Ile Leu Gln Gln Val
            100             105             110

Pro Asn Pro Ser Gly Thr Val Ser Thr Gly Gly Leu Gly Glu Pro Lys
        115             120             125

Phe Asn Ile Asp Glu Thr Ala Phe Thr Asp Ala Trp Gly Arg Pro Gln
        130             135             140

Arg Asp Gly Pro Ala Leu Arg Ala Thr Ala Ile Ile Thr Tyr Ala Asn
145             150             155             160

Trp Leu Leu Asp Asn Lys Asn Thr Thr Tyr Val Thr Asn Thr Leu Trp
            165             170             175

Pro Ile Ile Lys Leu Asp Leu Asp Tyr Val Ala Ser Asn Trp Asn Gln
        180             185             190

Ser Thr Phe Asp Leu Trp Glu Glu Ile Asn Ser Ser Ser Phe Phe Thr
        195             200             205

Thr Ala Val Gln His Arg Ala Leu Arg Glu Gly Ala Thr Phe Ala Asn
    210             215             220

Arg Ile Gly Gln Thr Ser Val Val Ser Gly Tyr Thr Thr Gln Ala Asn
225             230             235             240

Asn Leu Leu Cys Phe Leu Gln Ser Tyr Trp Asn Pro Thr Gly Gly Tyr
            245             250             255
```

```
Ile Thr Ala Asn Thr Gly Gly Gly Arg Ser Gly Lys Asp Ala Asn Thr
        260             265             270

Val Leu Thr Ser Ile His Thr Phe Asp Pro Ala Ala Gly Cys Asp Ala
        275             280             285

Val Thr Phe Gln Pro Cys Ser Asp Lys Ala Leu Ser Asn Leu Lys Val
    290             295             300

Tyr Val Asp Ala Phe Arg Ser Ile Tyr Ser Ile Asn Ser Gly Ile Ala
305             310             315             320

Ser Asn Ala Ala Val Ala Thr Gly Arg Tyr Pro Glu Asp Ser Tyr Met
            325             330             335

Gly Gly Asn Pro Trp Tyr Leu Thr Thr Ser Ala Val Ala Glu Gln Leu
            340             345             350

Tyr Asp Ala Leu Ile Val Trp Asn Lys Leu Gly Ala Leu Asn Val Thr
            355             360             365

Ser Thr Ser Leu Pro Phe Phe Gln Gln Phe Ser Ser Gly Val Thr Val
    370             375             380

Gly Thr Tyr Ala Ser Ser Ser Thr Phe Lys Thr Leu Thr Ser Ala
385             390             395             400

Ile Lys Thr Phe Ala Asp Gly Phe Leu Ala Val Asn Ala Lys Tyr Thr
            405             410             415

Pro Ser Asn Gly Gly Leu Ala Glu Gln Tyr Ser Arg Ser Asn Gly Ser
            420             425             430

Pro Val Ser Ala Val Asp Leu Thr Trp Ser Tyr Ala Ala Ala Leu Thr
            435             440             445

Ser Phe Ala Ala Arg Ser Gly Lys Thr Tyr Ala Ser Trp Gly Ala Ala
    450             455             460

Gly Leu Thr Val Pro Thr Thr Cys Ser Gly Ser Gly Gly Ala Gly Thr
465             470             475             480

Val Ala Val Thr Phe Asn Val Gln Ala Thr Thr Val Phe Gly Glu Asn
            485             490             495

Ile Tyr Ile Thr Gly Ser Val Pro Ala Leu Gln Asn Trp Ser Pro Asp
            500             505             510

Asn Ala Leu Ile Leu Ser Ala Ala Asn Tyr Pro Thr Trp Ser Ser Thr
```

```
                    515                     520                        525


         Val Asn Leu Pro Ala Ser Thr Thr Ile Glu Tyr Lys Tyr Ile Arg Lys
             530                     535                  540


         Phe Asn Gly Ala Val Thr Trp Glu Ser Asp Pro Asn Asn Ser Ile Thr
         545                     550                  555                  560


         Thr Pro Ala Ser Gly Thr Phe Thr Gln Asn Asp Thr Trp Arg
                         565                     570
```